# EUROPEAN PATENT SPECIFICATION

(11) **EP 4 114 865 B1**
(45) Date of publication and mention of the grant of the patent: **28.01.2026**
(21) Application number: 21709038.0
(22) Date of filing: 05.03.2021
(51) Int. Cl.: C07K 19/00, C07K 16/46, C12N 15/62, A61K 38/02, A61K 39/395

(54) **PROTEASE-PROCESSED MOLECULES**
PROTEASEVERARBEITETE MOLEKÜLE
MOLÉCULES TRAITÉES PAR LA PROTÉASE

(30) Priority: 05.03.2020 EP 20305233
(43) Date of publication of application: 11.01.2023
(73) Proprietor: SANOFI, 75017 Paris (FR)
(72) Inventor: LANGER, Thomas, 65926 Frankfurt am Main (DE); RAO, Ercole, 65926 Frankfurt am Main (DE); WEIL, Sandra, 65926 Frankfurt am Main (DE)
(74) Representative: Zwicker, Jörk
(86) International application number: PCT/EP2021/055673
(87) International publication number: WO 2021/176090

(56) References cited:
- WO-A1-2012/025530
- WO-A1-2012/158948
- WO-A1-2013/006544
- WO-A1-2013/100534
- WO-A2-2016/046778
- IZIDORO M A ET AL: "A study of human furin specificity using synthetic peptides derived from natural substrates, and effects of potassium ions", ARCHIVES OF BIOCHEMISTRY AND BIOPHYSICS, ACADEMIC PRESS, US, vol. 487, no. 2, 15 July 2009 (2009-07-15), pages 105 - 114, XP026283796, ISSN: 0003-9861, [retrieved on 20090527], DOI: 10.1016/J.ABB.2009.05.013
- P. DUCKERT ET AL: "Prediction of proprotein convertase cleavage sites", PROTEIN ENGINEERING, DESIGN AND SELECTION, vol. 17, no. 1, 1 January 2004 (2004-01-01), GB, pages 107 - 112, XP055386397, ISSN: 1741-0126, DOI: 10.1093/protein/gzh013

## Description

### Technical Field of the Invention

The present invention relates to protein molecules comprising at least one protease-cleavable linker. It also relates to protein molecules obtainable by protease-processing of such protein molecules as well as their use in therapy.

### Background of the Invention

Naturally occurring IgG antibodies are bivalent and monospecific. Multispecific, e.g., bispecific antibodies having binding specificities for multiple different antigens can be produced using recombinant technologies and are projected to have broad clinical applications. It is well known that complete IgG antibody molecules are Y-shaped molecules comprising four polypeptide chains: two heavy chains and two light chains. Each light chain consists of two domains, the N-terminal domain being known as the variable or VL domain (or region) and the C-terminal domain being known as the constant or CL domain/region (constant kappa (Cr) or constant lambda (Cλ) domain). Each heavy chain consists of four or five domains, depending on the class of the antibody. The N-terminal domain is known as the variable (or VH) domain (or region), which is followed by the first constant (or CH1) domain, the hinge region, and then the second and third constant (or CH2 and CH3) domains. In an assembled antibody, the VL and VH domains associate together to form an antigen binding site. Also, the CL and CH1 domains associate together to keep one heavy chain associated with one light chain. The two heavy-light chain heterodimers associate together by interaction of the CH2 and CH3 domains and interaction between the hinge regions on the two heavy chains.

One of the main problems coming along with the generation of multispecific antibodies is the light chain mispairing. While the pairing of two different heavy chains can be addressed by technologies like knobs-into-holes, it is also essential to impose correct light chain association. This is probably a bigger challenge from a structural point of view, since the modifications should be applied within the Fab fragment interface, which requires more modifications. In any case, the heterodimerization of heavy chains and their association with the cognate light chain require a quite intensive number of different designs to achieve proper chain association, avoiding the formation of unwanted species. Furthermore, expression of multispecific molecules from different vectors/plasmids, encoding light chains and heavy chains, requires translation of all plasmids to the same extent to provide optimal ratios for correct chain pairing.

WO 2012/158948 pertains to HIV neutralizing antibodies and discloses an immunoglobulin heavy chain having the sequence SVDE at its N-terminus. Izidoro et al. (Archives of Biochemistry and Biophysics 487 (2009) 105-114) discloses Dengue virus envelope glycoproteins comprising the cleavage site HRRXKR. WO 2013/006544 discloses different furin cleavage sites such as RX[R/K]R↓ or TRHRQPR↓GWEQL derived from domain II of *P. aeruginosa* toxin A, or RKKR. WO 2012/025530 pertains to bispecific antibodies comprising cleavable peptide linkers between VH-VH domains, within the constant domain, and between the variable and the constant domains, respectively. A furin cleavage site is identified as having the sequence RHRR↓AL, specific furin cleavage sites can have the sequence of QSSRHRRAL or LSHRSKRSL. WO 2016/046778 discloses protease-activatable proteins. Furin cleavage sites for these proteins are identified as RRRRR, RRRRRR and GQSSRHRRAL.

The present invention addresses the light chain pairing problem by a change of paradigm, from design-derived multispecific formats to process-derived multispecific formats. Here, no modifications of antibody sequences are needed.

### Summary of the Invention

The invention is defined by the appended claims. Any part of the description which does not fall under the scope of the appended claims is for illustrative purposes only.

In one aspect, the present invention relates to a protein comprising at least one polypeptide chain having the formula

PP₁-PCL-PP₂,

wherein
PP₁ is a first polypeptide,
PP₂ is a second polypeptide, and
PCL is a protease-cleavable linker comprising at least one protease-cleavage site comprising the amino acid sequence HRRX₁X₂RSVDE (SEQ ID NO: 42), wherein X₁ and X₂ are independently selected from the group consisting of KK, KQ, KR, QK, QQ, QR, RK, RQ and RR.

In one embodiment, the at least one protease-cleavage site comprises the amino acid sequence HRRRKRSVDE (SEQ ID NO: 43) or the amino acid sequence HRRQQRSVDE (SEQ ID NO: 44).

In one embodiment, protease-cleavage of the linker results in a change in activity of the protein.

In one embodiment, the change in activity is binding or increased binding to at least one antigen.

In one embodiment, protease-cleavage occurs intracellularly.

In one embodiment, the protease-cleavable linker comprises two protease-cleavage sites, wherein the two protease-cleavage sites are located at the N-terminus and at the C-terminus of the protease-cleavable linker, respectively, wherein the two protease-cleavage sites may be the same or different.

In one embodiment, PP₁ comprises at least one immunoglobulin constant region and/or at least one immunoglobulin variable region, and/or PP₂ comprises at least one immunoglobulin constant region and/or at least one immunoglobulin variable region.

In one embodiment,
PP₁ comprises an immunoglobulin constant region and PP₂ comprises an immunoglobulin variable region,
PP₁ comprises an immunoglobulin variable region and PP₂ comprises an immunoglobulin constant region,
PP₁ comprises an immunoglobulin constant region and PP₂ comprises an immunoglobulin constant region, or
PP₁ comprises an immunoglobulin variable region and PP₂ comprises an immunoglobulin variable region.

In one embodiment, the protein is an antibody or antibody derivative.

In one embodiment, the protein is a single chain antibody, preferably a multispecific single chain antibody.

In one embodiment, the protein comprises a polypeptide chain having a formula selected from the group consisting of:
(a)

   VL-CL-PCL-VH-CH1-hinge-CH2-CH3,

   wherein
   VL is an immunoglobulin light chain variable region;
   CL is an immunoglobulin light chain constant region;
   VH is an immunoglobulin heavy chain variable region;
   CH1 is an immunoglobulin CH1 heavy chain constant region;
   hinge is an immunoglobulin hinge region;
   CH2 is an immunoglobulin CH2 heavy chain constant region;
   CH3 is an immunoglobulin CH3 heavy chain constant region; and
   PCL is the protease-cleavable linker;
(b)

   VLₐ-CLₐ-PCL-VL_{b}-CL_{b},

   wherein
   VLₐ is a first immunoglobulin light chain variable region;
   VL_{b} is a second immunoglobulin light chain variable region;
   CLₐ is a first immunoglobulin light chain constant region;
   CL_{b} is a second immunoglobulin light chain constant region; and
   PCL is the protease-cleavable linker; and
(c)

   VLₐ-CLₐ-PCL-VL_{b}-CL_{b}-L-hinge-CH2-CH3

   wherein
   VLₐ is a first immunoglobulin light chain variable region;
   VL_{b} is a second immunoglobulin light chain variable region;
   CLₐ is a first immunoglobulin light chain constant region;
   CL_{b} is a second immunoglobulin light chain constant region;
   L is an optional linker;
   hinge is an immunoglobulin hinge region;
   CH2 is an immunoglobulin CH2 heavy chain constant region;
   CH3 is an immunoglobulin CH3 heavy chain constant region; and
   PCL is the protease-cleavable linker.

In one embodiment, the protein comprises a polypeptide chain having the formula VL-CL-PCL-VH-CH1-hinge-CH2-CH3, wherein VL pairs with VH to form an antigen binding site.

In one embodiment, the antigen binding site becomes active or exhibits increased activity upon protease-cleavage of the protease-cleavable linker.

In one embodiment, the protein comprises two copies of the polypeptide chain having the formula VL-CL-PCL-VH-CH1-hinge-CH2-CH3, wherein the two copies are associated with each other via at least two disulfide bonds.

In one embodiment, the protein comprises a first polypeptide chain having the formula VLₐ-CLₐ-PCL-VHₐ-CH1ₐ-hinge-CH2-CH3, and a second polypeptide chain having the formula VL_{b}-CL_{b}-PCL-VH_{B}-CH1_{b}-hinge-CH2-CH3,
wherein
VLₐ is a first immunoglobulin light chain variable region;
VL_{b} is a second immunoglobulin light chain variable region;
CLₐ is a first immunoglobulin light chain constant region;
CL_{b} is a second immunoglobulin light chain constant region;
VHₐ is a first immunoglobulin heavy chain variable region;
VH_{b} is a second immunoglobulin heavy chain variable region;
CH1ₐ is a first immunoglobulin CH1 heavy chain constant region; and
CH1_{b} is a second immunoglobulin CH1 heavy chain constant region;
wherein VLₐ pairs with VHₐ to form an antigen binding site binding to antigen A, and wherein VL_{b} pairs with VH_{b} to form an antigen binding site binding to antigen B; and
wherein the first polypeptide chain is associated with the second polypeptide chain via at least two disulfide bonds and, optionally, via at least one additional Fc interaction.

In one embodiment, the antigen binding site A and/or the antigen binding site B become active or exhibit increased activity upon protease-cleavage of the protease-cleavable linkers.

In one embodiment, the at least one additional Fc interaction is at least one knob-into-hole interaction.

In one embodiment, the protein comprises a first polypeptide chain having the formula VL_{b}-L1-VLₐ-L2-CL, a second polypeptide chain having the formula VHₐ-L3-VH_{b}-L4-CH1-hinge-CH2-CH3 and a third polypeptide chain having the formula VL_{c}-CL_{c}-PCL-VH_{c}-CH1_{c}-hinge-CH2-CH3, wherein
VLₐ is a first immunoglobulin light chain variable region;
VL_{b} is a second immunoglobulin light chain variable region;
VL_{c} is a third immunoglobulin light chain variable region;
CL is an immunoglobulin light chain constant region;
CL_{c} is an immunoglobulin light chain constant region;
VHₐ is a first immunoglobulin heavy chain variable region;
VH_{b} is a second immunoglobulin heavy chain variable region;
VH_{c} is a third immunoglobulin heavy chain variable region;
CH1 is an immunoglobulin CH1 heavy chain constant region;
CH1_{c} is an immunoglobulin CH1 heavy chain constant region;
hinge is an immunoglobulin hinge region;
CH2 is an immunoglobulin CH2 heavy chain constant region; and
CH3 is an immunoglobulin CH3 heavy chain constant region;
L1, L2, L3 and L4 are linkers;
wherein VLₐ pairs with VHₐ to form an antigen binding site binding to antigen A, wherein VL_{b} pairs with VH_{b} to form an antigen binding site binding to antigen B, and wherein VL_{c} pairs with VH_{c} to form an antigen binding site binding to antigen C; and
wherein the second polypeptide chain is associated with the third polypeptide chain via at least two disulfide bonds and, optionally, via at least one additional Fc interaction.

In one embodiment, the antigen binding site C becomes active or exhibits increased activity upon protease-cleavage of the protease-cleavable linker.

In one embodiment, the at least one additional Fc interaction is at least one knob-into-hole interaction.

In one embodiment, the protein comprises a first polypeptide chain having the formula VLₐ-CLₐ-PCL-VL_{b}-CL_{b}, and a second polypeptide chain having the formula VHₐ-CH1ₐ-L-VH_{b}-CH1_{b}-hinge-CH2-CH3,
wherein
VHₐ is a first immunoglobulin heavy chain variable region;
VH_{b} is a second immunoglobulin heavy chain variable region;
CH1ₐ is a first immunoglobulin CH1 heavy chain constant region;
CH1_{b} is a second immunoglobulin CH1 heavy chain constant region;
L is an optional linker;
hinge is an immunoglobulin hinge region;
CH2 is an immunoglobulin CH2 heavy chain constant region; and
CH3 is an immunoglobulin CH3 heavy chain constant region;
wherein VLₐ pairs with VHₐ to form an antigen binding site binding to antigen A, and wherein VL_{b} pairs with VH_{b} to form an antigen binding site binding to antigen B.

In one embodiment, the antigen binding site B becomes active or exhibits increased activity upon protease-cleavage of the protease-cleavable linker.

In one embodiment, the protein comprises two copies of the first polypeptide chain and two copies of the second polypeptide chain, wherein the two copies of the second polypeptide chain are associated with each other via at least two disulfide bonds.

In one embodiment, the protein comprises a first polypeptide chain having the formula VL_{b}-CL_{b}-PCL-VLₐ-CLₐ, a second polypeptide chain having the formula VL_{c}-CL_{c}-PCL-VL_{d}-CL_{d}, a third polypeptide chain having the formula VHₐ-CH1ₐ-L-VH_{b}-CH1_{b}-hinge-CH2-CH3, and a fourth polypeptide chain having the formula VH_{c}-CH1_{c}-L-VH_{d}-CH1_{d}-hinge-CH2-CH3,
wherein
VLₐ is a first immunoglobulin light chain variable region;
VL_{b} is a second immunoglobulin light chain variable region;
VL_{c} is a third immunoglobulin light chain variable region;
VL_{d} is a fourth immunoglobulin light chain variable region;
CLₐ is a first immunoglobulin light chain constant region;
CL_{b} is a second immunoglobulin light chain constant region;
CL_{c} is a third immunoglobulin light chain constant region;
CL_{d} is a fourth immunoglobulin light chain constant region;
VHₐ is a first immunoglobulin heavy chain variable region;
VH_{b} is a second immunoglobulin heavy chain variable region;
VH_{c} is a third immunoglobulin heavy chain variable region;
VH_{d} is a fourth immunoglobulin heavy chain variable region;
CH1ₐ is a first immunoglobulin CH1 heavy chain constant region;
CH1_{b} is a second immunoglobulin CH1 heavy chain constant region;
CH1_{c} is a third immunoglobulin CH1 heavy chain constant region;
CH1_{d} is a fourth immunoglobulin CH1 heavy chain constant region;
L is an optional linker;
hinge is an immunoglobulin hinge region;
CH2 is an immunoglobulin CH2 heavy chain constant region; and
CH3 is an immunoglobulin CH3 heavy chain constant region;
wherein VLₐ pairs with VHₐ to form an antigen binding site binding to antigen A, wherein VL_{b} pairs with VH_{b} to form an antigen binding site binding to antigen B, wherein VL_{c} pairs with VH_{c} to form an antigen binding site binding to antigen C, and wherein VL_{d} pairs with VH_{d} to form an antigen binding site binding to antigen D; and
wherein the third polypeptide chain is associated with the fourth polypeptide chain via at least two disulfide bonds and, optionally, via at least one additional Fc interaction.

In one embodiment, the antigen binding site B and/or the antigen binding site D become active or exhibit increased activity upon protease-cleavage of the protease-cleavable linkers.

In one embodiment, the at least one additional Fc interaction is at least one knob-into-hole interaction.

In one embodiment, the protein comprises a first polypeptide chain having the formula VLₐ-CLₐ-PCL-VL_{b}-CL_{b}-L-hinge-CH2-CH3, and a second polypeptide chain having the formula VHₐ-CH1ₐ-L-VH_{b}-CH1_{b}-hinge-CH2-CH3,
wherein
VHₐ is a first immunoglobulin heavy chain variable region;
VH_{b} is a second immunoglobulin heavy chain variable region;
CH1ₐ is a first immunoglobulin CH1 heavy chain constant region;
CH1_{b} is a second immunoglobulin CH1 heavy chain constant region;
L is an optional linker;
hinge is an immunoglobulin hinge region;
CH2 is an immunoglobulin CH2 heavy chain constant region; and
CH3 is an immunoglobulin CH3 heavy chain constant region;
wherein VLₐ pairs with VHₐ to form an antigen binding site binding to antigen A, and wherein VL_{b} pairs with VH_{b} to form an antigen binding site binding to antigen B; and
wherein the first polypeptide chain is associated with the second polypeptide chain via at least two disulfide bonds and, optionally, via at least one additional Fc interaction.

In one embodiment, the antigen binding site B becomes active or exhibits increased activity upon protease-cleavage of the protease-cleavable linker.

In one embodiment, the at least one additional Fc interaction is at least one knob-into-hole interaction.

In another aspect, the present invention relates to a nucleic acid or set of nucleic acids encoding the protein as defined above.

In another aspect, the present invention relates to a vector or set of vectors comprising the nucleic acid or set of nucleic acids as defined above.

In another aspect, the present invention relates to a host cell comprising a protein as defined above, a nucleic acid or set of nucleic acids as defined above, or a vector or set of vectors as defined above.

In one embodiment, the host cell expresses an endogenous or exogenous furin and/or furin-like protease.

In one embodiment, the host cell is a mammalian cell.

In one aspect, the present invention relates to a method of producing a protein comprising the steps:
i) culturing a host cell as defined above; and
ii) isolating the protein from the host cell.

In another aspect, the present invention relates to a protein obtainable by the method as defined above.

In yet another aspect, the present invention relates to a protein obtainable by furin and/or furin-like protease-cleavage of a protein as defined above.

In one embodiment, the protein as defined above is a therapeutically active protein.

In one aspect, the present invention relates to a protein as defined above for use in therapy.

In another aspect, the present invention relates to the use of a protein as defined above in the manufacture of a medicament.

In yet another aspect, the present invention relates to a pharmaceutical composition or kit comprising a protein as defined above.

In another aspect, the present invention relates to a peptide comprising the amino acid sequence of any one of SEQ ID NO: 42, wherein X₁ and X₂ are independently selected from the group consisting of KK, KQ, KR, QK, QQ, QR, RK, RQ and RR, or of SEQ ID NOs: 43 or 44, to a protein comprising said peptide, to a nucleic acid encoding said peptide or protein, to a vector comprising said nucleic acid, or to a host cell comprising said peptide, protein, nucleic acid or vector.

### Description of the Figures

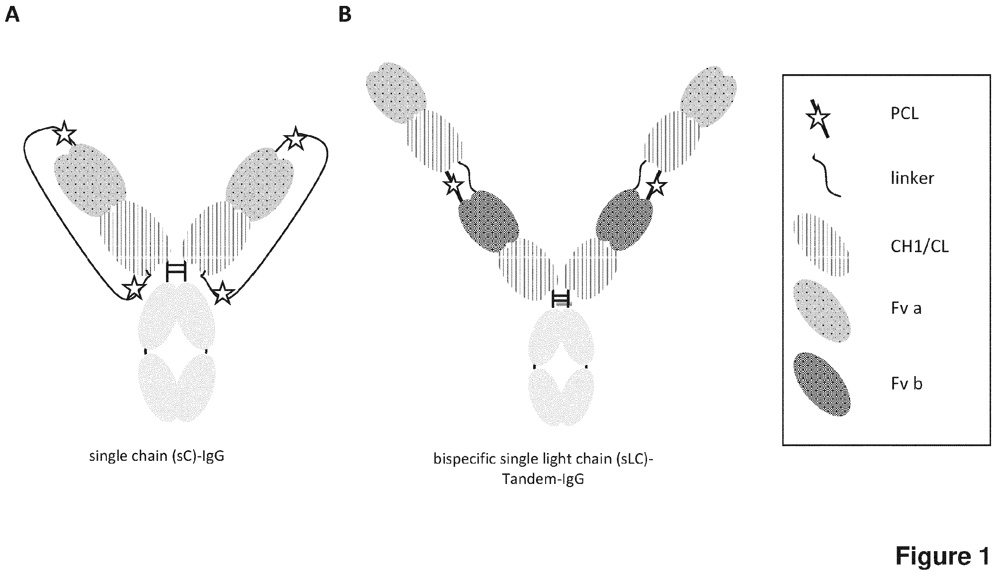

**Figure 1****: Protease-cleavable homodimeric antibody formats.** (A) Single chain (sC) monoclonal IgG. The CL (e.g., Cκ) domain is elongated at its C-terminus via a protease-cleavable linker (PCL) sequence with the N-terminus of the VH domain of the heavy chain. (B) Single light chain (sLC) bivalent, bispecific Tandem-lgG. The VH-CH1 (VHb) domain of the heavy chain is elongated at its N-terminus with a second VH-CH1 domain (VHa), e.g., via a (G₄S)₃ linker (SEQ ID NO: 55). The VL-CL domain (VLb) of the light chain is elongated at its N-terminus with a second VL-CL domain (VLa) using a PCL sequence.

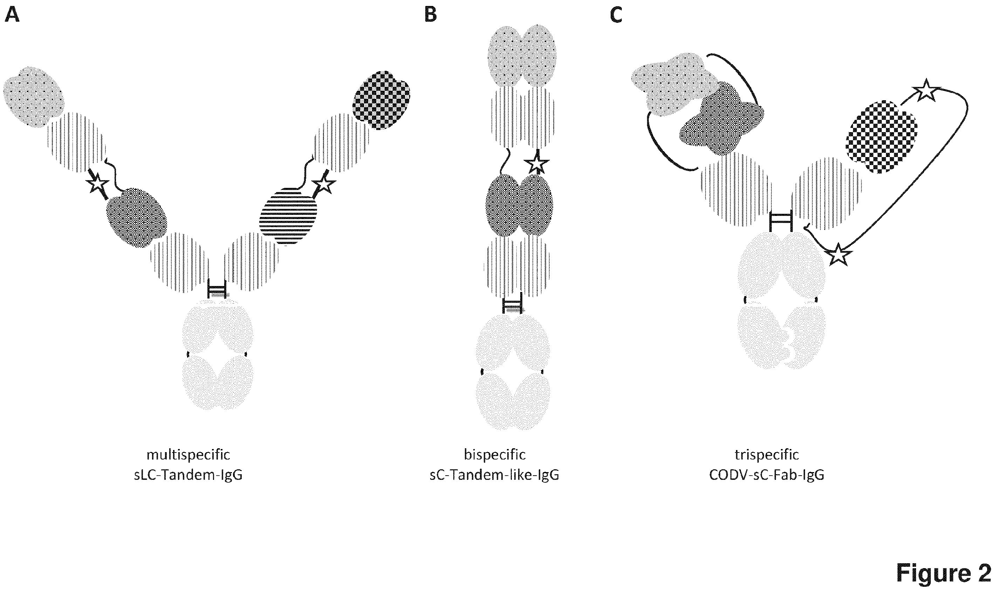

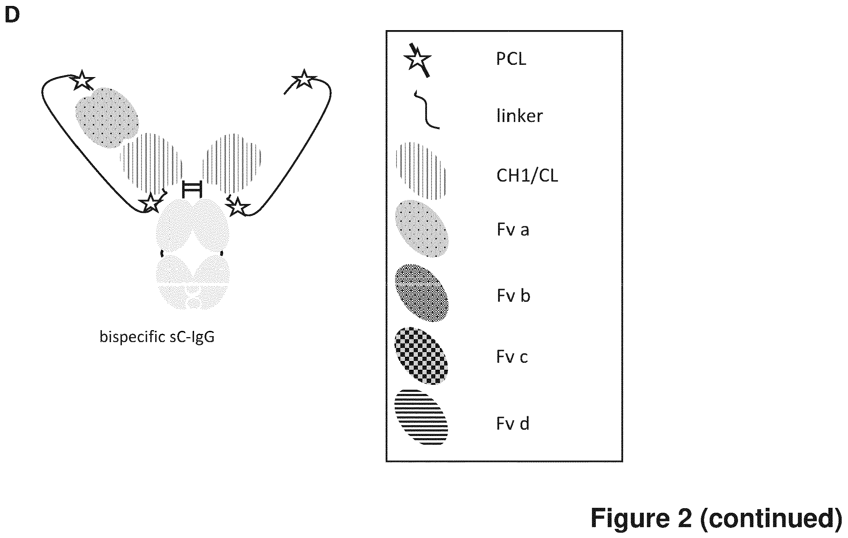

**Figure 2****: Protease-cleavable homodimeric antibody formats.** (A) Single light chain (sLC) multispecific Tandem-lgG. The VH-CH1 (VHb and VHd) domains of the heavy chains are elongated at the N-termini with a second VH-CH1 domain (VHa and VHc), e.g., via a (G₄S)₃ linker (SEQ ID NO: 55). The VL-CL domains (VLb and VLd) of the light chains are elongated at the N-termini with a second VL-CL domain (VLa and VLc) using a PCL sequence. (B) Single chain (sC) monovalent, bispecific Tandem-like-lgG. The VH-CH1 domain (VHb) of the first heavy chain is elongated at its N-terminus with a second VH-CH1 domain (VHa), e.g., via a (G₄S)₃ linker (SEQ ID NO: 55); the hinge region of the second heavy chain is elongated with two VL-CL-domains, wherein VLb and Fv a are connected via a PCL sequence. (C) Single chain (sC) Fab containing trispecific CODV-IgG. One arm of the antibody comprises a CODV-LC (VLb-linker-VLa-linker-CL) associated with a CODV-HC (VHa-linker-VHb-linker-CH1-hinge-CH2-CH3) the other arm comprises a single chain (sC) Fab arm, where the VHc domain is elongated at its N-terminus with a VLc-CL domain using a PCL sequence. (D) Single chain (sC) bispecific IgG. The VHa and VHb domains of each HC are elongated at the N-termini with a VLa-CL and VLb-CL domain, respectively, using a PCL sequence. The heavy chains of all heterodimeric molecules preferably comprise one or more knops-into-holes (KIH) mutations forming an asymmetric antibody.

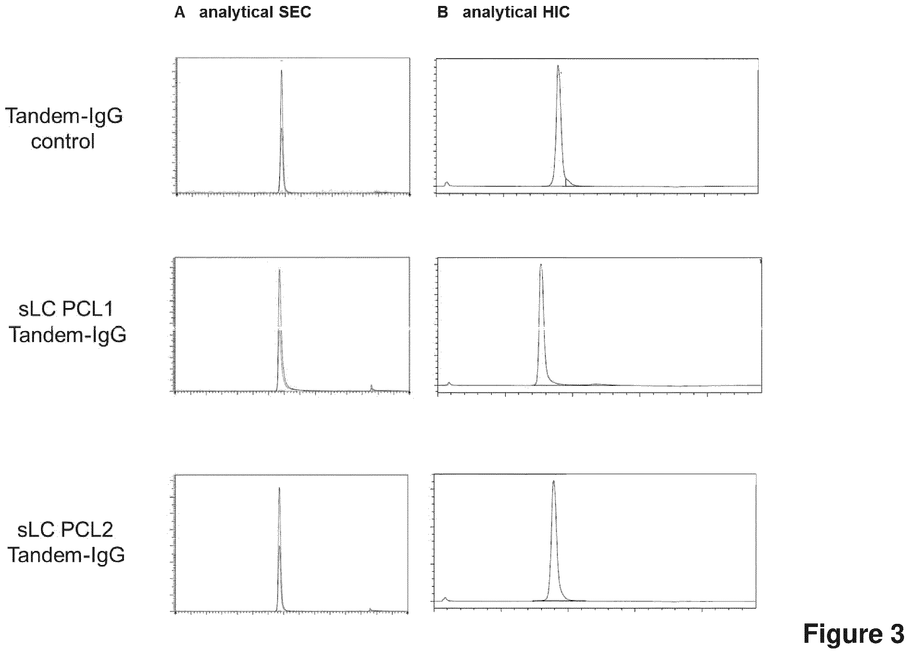

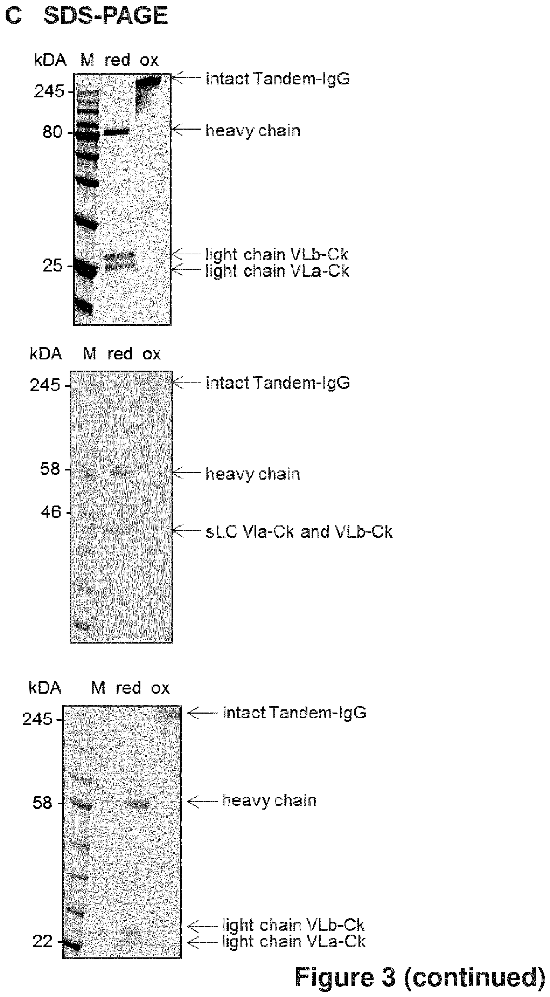

Figure 3: Analysis of Tandem-anti-IL4 x anti-IL13-hulgG1 constructs from HEK293-FS cells. Panel (A) shows the purity of the Tandem-IgG products (Tandem-lgG control; sLC-PCL1-Tandem-IgG and sLC-PCL2-Tandem-lgG) after affinity and preparative SEC using analytical size exclusion chromatography. Panel (B) shows the homogeneity of the antibody products using analytical hydrophobic-interaction chromatography. Panel (C) shows the reduced (one heavy chain and two light chains/ one single LC, respectively) and oxidized form (intact Tandem-IgG) of the antibody using 4-12% Bis/Tris MOPS sodium dodecyl sulfate polyacrylamide gel electrophoresis (SDS-PAGE).

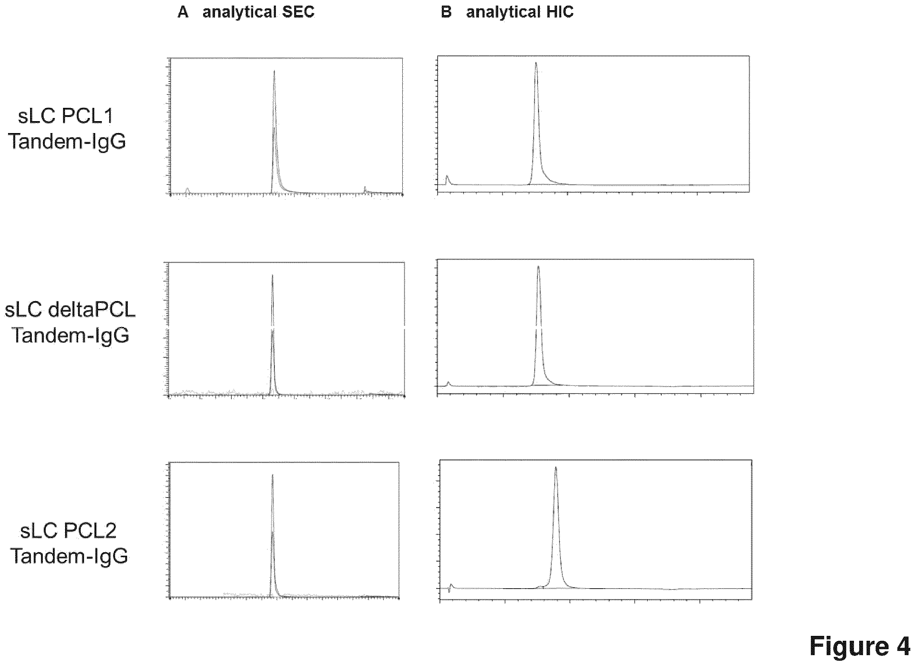

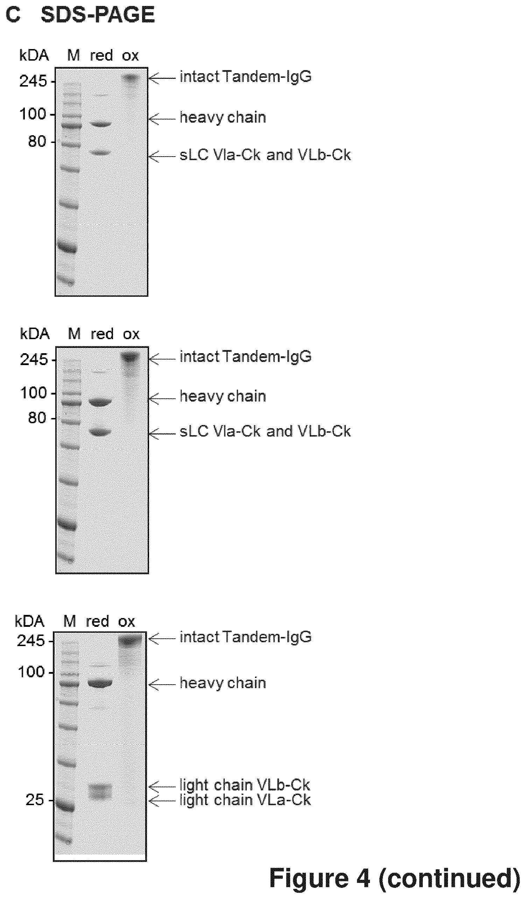

Figure 4: Analysis of Tandem-anti-IL4 x anti-IL13-hulgG1 constructs from HEK293-FS cells. Panel **(A)** shows the purity of the Tandem-lgG products (sLC-PCL1-Tandem-lgG, sLC deltaPCL Tandem-lgG and sLC-PCL2-Tandem-lgG) after affinity and preparative SEC using analytical size exclusion chromatography. Panel **(B)** shows the homogeneity of the antibody products using analytical hydrophobic-interaction chromatography. Panel **(C)** shows the reduced (one heavy chain and two light chains/ one single LC, respectively) and oxidized form (intact Tandem-IgG) of the antibody using 4-12% Bis/Tris MOPS sodium dodecyl sulfate polyacrylamide gel electrophoresis (SDS-PAGE).

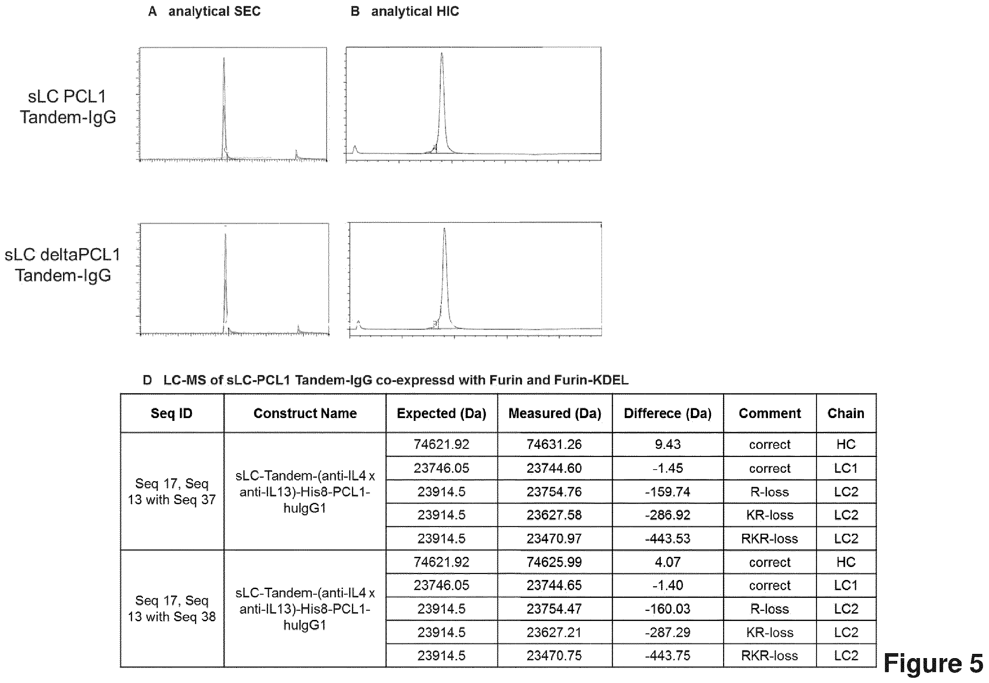

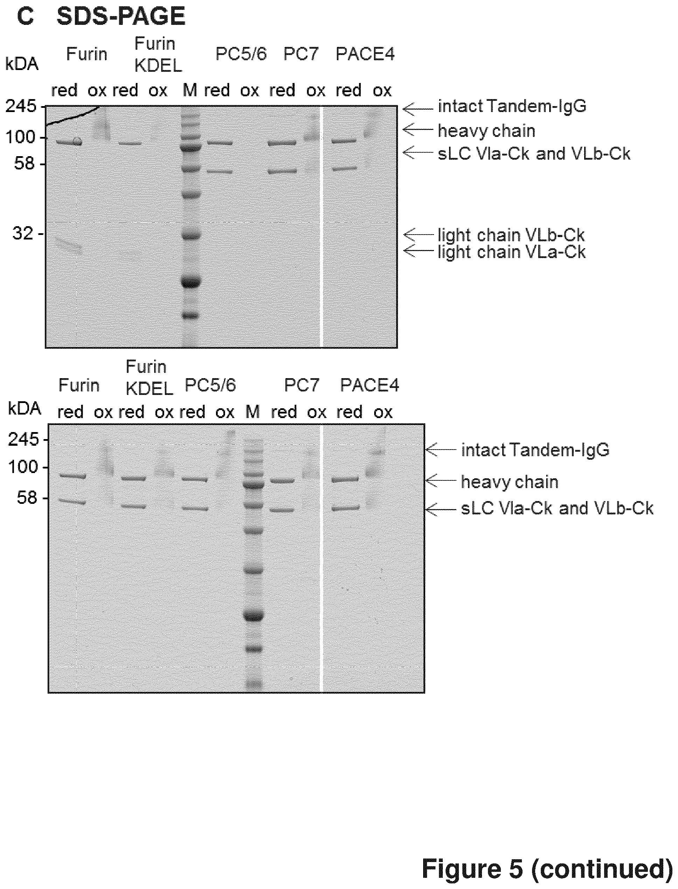

**Figure 5****: Analysis of sLC Tandem-IgG co-expressed with proteases in HEK293-FS cells.** Panel **(A)** shows the purity of the Tandem-lgG products (sLC-PCL1-Tandem-lgG, sLC deltaPCL Tandem-IgG) after affinity and preparative SEC using analytical size exclusion chromatography. Panel **(B)** shows the homogeneity of the antibody products using analytical hydrophobic-interaction chromatography. Panel **(C)** shows the reduced (one heavy chain and two light chains/ one single LC, respectively) and oxidized form (intact Tandem-lgG) of the antibody using 4-12% Bis/Tris MOPS sodium dodecyl sulfate polyacrylamide gel electrophoresis (SDS-PAGE). Panel **(D)** shows LC-MS analysis after deglycosylation and under reducing conditions of sLC Tandem-IgG-PCL1 after co-expression with furin and furin-KDEL.

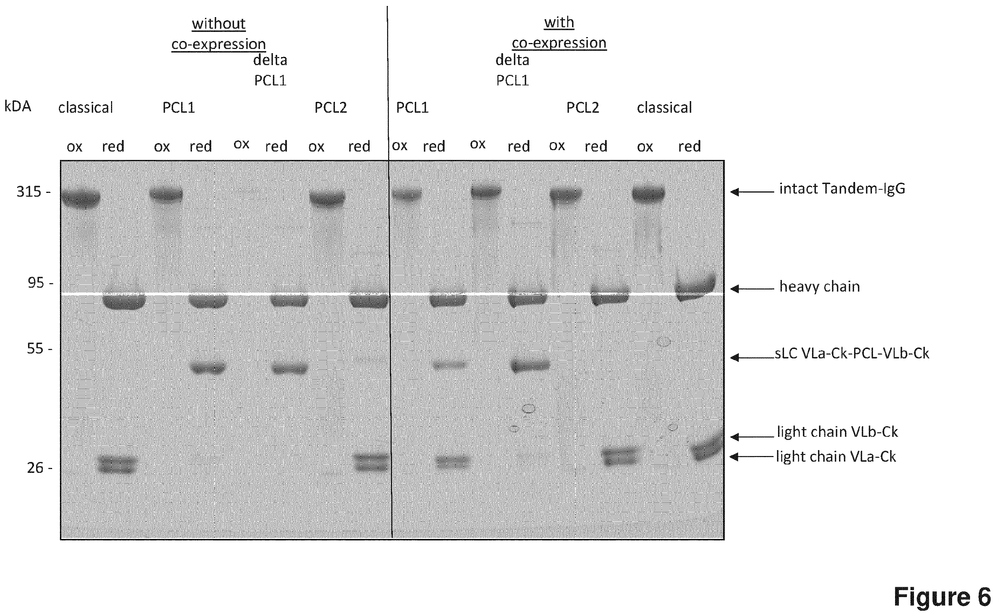

**Figure 6****: Analysis of sLC Tandem-IgGs with and without co-expression of protease in stable CHO 9E4 cell pools.** The oxidized (intact Tandem-IgG) and reduced forms (one heavy chain and two light chains / one single LC, respectively) of purified antibodies expressed in stable cell pools with (left panel) and without (right panel) protease co-expression are shown using 4-12% Bis/Tris MOPS sodium dodecyl sulfate polyacrylamide gel electrophoresis (SDS-PAGE).

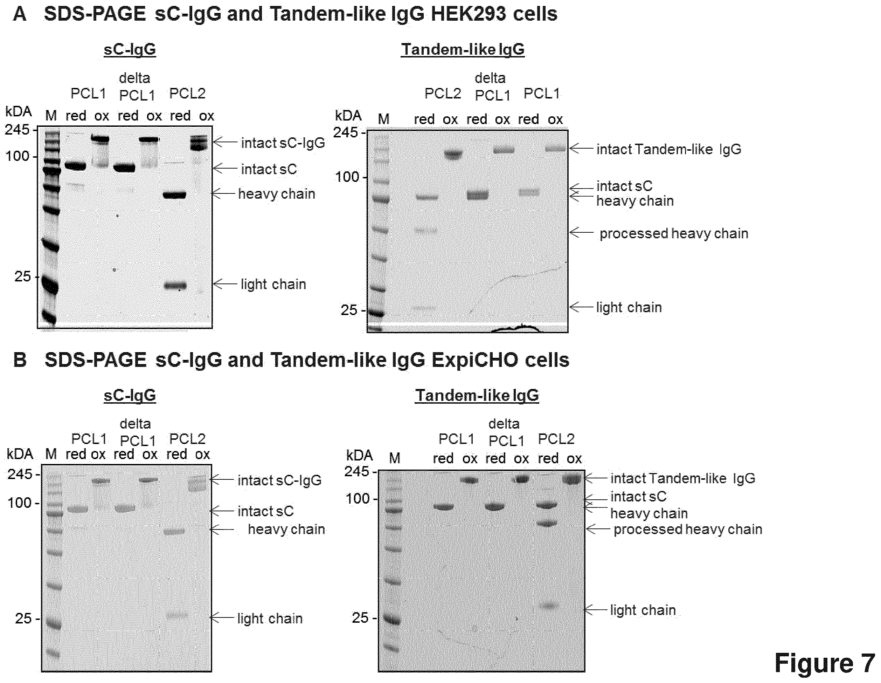

**Figure 7****: Analysis of sC-lgG and Tandem-like IgGs expressed in HEK293-FS and ExpiCHO cells with SDS-PAGE.** Panel **(A)** shows the reduced (one heavy chain and one light chain/ one sC (left), one heavy chain and un-/processed sC (right)) and oxidized form (intact Tandem-lgG) of the two antibody formats expressed in HEK293-FS cells and panel **(B)** corresponding constructs expressed in ExpiCHO cells using 4-12% Bis/Tris MOPS sodium dodecyl sulfate polyacrylamide gel electrophoresis (SDS-PAGE) after Protein A and SEC purification.

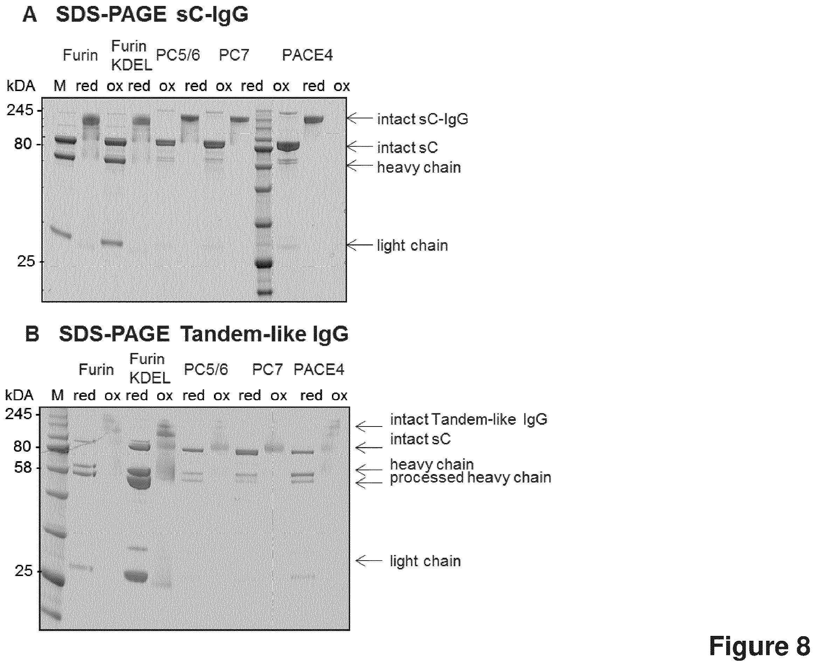

**Figure 8****: Analysis of sC-IgG and Tandem-like IgGs co-expressed with proteases in HEK293-FS cells.** Panel **(A)** shows the reduced (one heavy chain and one light chain/ one sC) and oxidized form of sC-lgG and panel **(B)** the reduced (one heavy chain and un-/processed sLC) and oxidized form (intact Tandem-like IgG) of the Tandem-like IgG after co-expression with PCSK family members in HEK293-FS cells using 4-12% Bis/Tris MOPS sodium dodecyl sulfate polyacrylamide gel electrophoresis (SDS-PAGE) after Protein A and SEC purification.

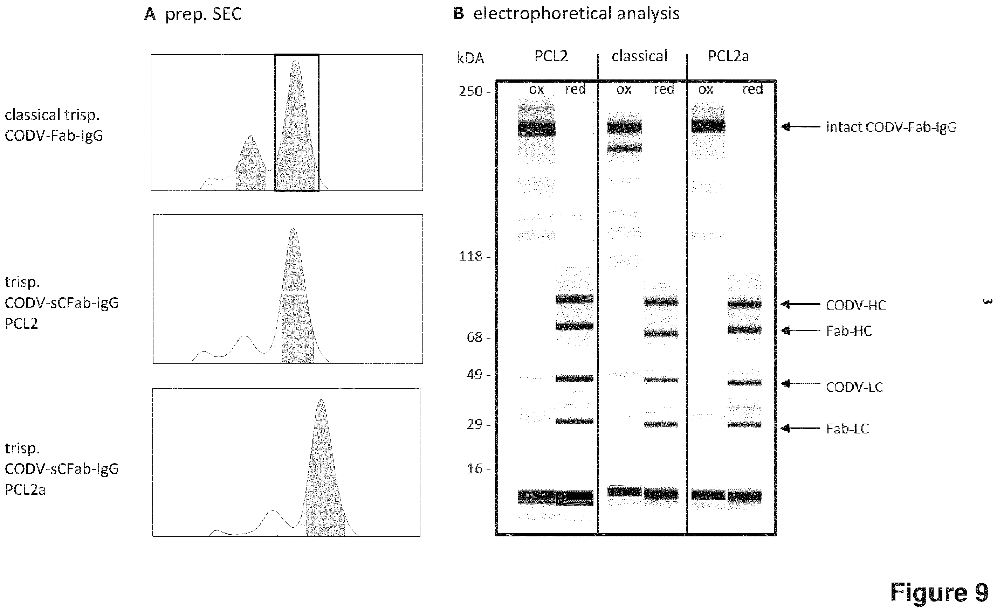

**Figure 9****: Analysis of trispecific CODV-sCFab-IgG expressed in HEK293FS cells.** Panel **(A)** shows the preparative SEC chromatograms and panel **(B)** shows the oxidized (intact trispecific CODV-Fab-lgG and reduced (CODV heavy and light chains and single/processed Fab heavy and light chain) after preparative SEC using LabCHip electrophoresis device.

### Detailed Description of the Invention

Before the present invention is described in detail below, it is to be understood that this invention is not limited to the particular methodology, protocols and reagents described herein as these may vary. It is also to be understood that the terminology used herein is for the purpose of describing particular embodiments only, and is not intended to limit the scope of the present invention which will be limited only by the appended claims. Unless defined otherwise, all technical and scientific terms used herein have the same meanings as commonly understood by one of ordinary skill in the art. Preferably, the terms used herein are defined as described in "A multilingual glossary of biotechnological terms: (IUPAC Recommendations)", Leuenberger, H.G.W, Nagel, B. and Kölbl, H. eds. (1995), Helvetica Chimica Acta, CH-4010 Basel, Switzerland).

In the following, the elements of the present invention will be described. These elements are listed with specific embodiments, however, it should be understood that they may be combined in any manner and in any number to create additional embodiments. The variously described examples and preferred/particular embodiments should not be construed to limit the present invention to only the explicitly described embodiments. This description should be understood to support and encompass embodiments which combine the explicitly described embodiments with any number of the disclosed and/or preferred elements. Furthermore, any permutations and combinations of all described elements in this application should be considered disclosed by the description of the present application unless the context indicates otherwise.

Throughout this specification and the claims which follow, unless the context requires otherwise, the word "comprise", and variations such as "comprises" and "comprising", are to be understood to imply the inclusion of a stated integer or step or group of integers or steps but not the exclusion of any other integer or step or group of integer or step. As used in this specification and the appended claims, the singular forms "a", "an", and "the" include plural referents, unless the content clearly dictates otherwise.

In one aspect, the present invention relates to a protein comprising at least one polypeptide chain having the formula

PP₁-PCL-PP₂,

wherein
PP₁ is a first polypeptide,
PP₂ is a second polypeptide, and
PCL is a protease-cleavable linker comprising at least one protease-cleavage site comprising the amino acid sequence HRRX₁X₂RSVDE (SEQ ID NO: 42), wherein X₁ and X₂ are independently selected from the group consisting of KK, KQ, KR, QK, QQ, QR, RK, RQ and RR.

If the protein comprises more than one polypeptide chain as defined above, these polypeptide chains may be the same or different.

The term "peptide" according to the invention refers to substances comprising two or more, preferably 3 or more, preferably 4 or more, preferably 6 or more, preferably 8 or more, preferably 9 or more, preferably 10 or more, preferably 13 or more, preferably 16 more, preferably 21 or more and up to preferably 8, 10, 20, 30, 40 or 50, in particular 100 amino acids joined covalently by peptide bonds. The term "protein" or "polypeptide" refers to large peptides, preferably to peptides with more than 100 amino acid residues, but in general the terms "peptides", "polypeptides" and "proteins" are synonyms and are used interchangeably herein.

In one embodiment, the protein is a recombinant protein. In one embodiment, the protein is a fusion protein.

The term "recombinant" in the context of the present invention means "made through genetic engineering". Preferably, a "recombinant object" is not naturally occurring.

The term "naturally occurring", as used herein, as applied to an object refers to the fact that an object can be found in nature. For example, a polypeptide or polynucleotide sequence that is present in an organism (including viruses) that can be isolated from a source in nature and which has not been intentionally modified by man in the laboratory is naturally occurring.

The term "fusion protein" (or "chimeric protein") generally refers to proteins created by joining, in particular covalently linking, two or more distinct proteins and/or peptides resulting in a single protein with functional properties derived from each of the original proteins and/or peptides.

The term "linker", as used herein, preferably refers to a peptide linker, i.e., a linker composed of amino acids connected via peptide bonds. A linker in accordance with the present invention may, however, also include non-peptidic components, such as non-peptidic polymers (e.g., PEG).

A peptide linker in accordance with the present invention may have any length, i.e., comprise any number of amino acid residues. However, it is preferably long enough to provide an adequate degree of flexibility so that the connected/linked moieties can, for example, interact/pair with each other or other moieties, and to allow for proper protein folding; yet it is preferably short enough to provide stability (e.g., proteolytic stability) in the cell. Suitable peptide linkers are described in, e.g., Chen et al., Adv Drug Deliv Rev. 2013, 65(10):1357-69. Preferably, a peptide linker according to the present invention has a length of 1 to 100 amino acids.

Flexibility of a peptide linker is generally increased if its amino acids are small and do not have bulky side chains that impede rotation or bending of the amino acid chain. Thus, a peptide linker of the present invention preferably has an increased content of small amino acids, in particular of glycines, alanines, serines, threonines, leucines and isoleucines. Preferably, at least 20%, 30%, 40%, 50%, 60%, 70%, 80%, 90% or more of the amino acids of the peptide linker are such small amino acids. In particular embodiments, the peptide linkers according to the present invention are glycine-serine-rich linkers, wherein at least 50%, preferably at least 60%, more preferably at least 70%, more preferably at least 80%, even more preferably at least 85% of the amino acids are a glycine or serine residue, respectively. Peptide linkers in accordance with the present invention can also be exclusively composed of glycine and/or serine residues (referred to as glycine linkers, serine linkers or glycine-serine linkers, respectively). Exemplary peptide linkers comprise a sequence of the amino acid formula (GₗSₘ)ₙ, wherein l is an integer from 1 to 4, m is 1 or 2, and n is an integer from 1 to 12, preferably 1 to 10, e.g., 2 to 10 or 4 to 10. Peptide linkers according to the present invention may also comprise or consist of other sequence elements, e.g., a Histag.

In some embodiments, a peptide linker according to the present invention may also be a rigid peptide linker. Such rigid peptide linkers are known to a person skilled in the art and include, for example, proline-rich peptide linkers with the general formula (XP)ₙ, with X designating any amino acid, preferably alanine, lysine or glutamic acid, and n being an integer, preferably an integer from 1 to 12. A particular example of this motif is (AP)ₙA (SEQ ID NO: 53). Another example of a rigid peptide linker sequence motif is (EAAAK)ₙ (SEQ ID NO: 52), wherein n is an integer, preferably an integer from 1 to 12.

The term "protease-cleavable linker" ("PCL"), as used herein, refers to a linker comprising at least one protease-cleavage site.

A "protease-cleavage site" ("PCS"; also referred to as protease recognition site herein) according to the present invention is a type of enzymatic cleavage site in a protein which is the target for enzymes (proteases) that function after translation of the protein. In one embodiment, such enzymes function during transport from the Golgi lumen to the trans-Golgi compartment. Intracellular processing enzymes (proteases) cleave polypeptides prior to secretion of the protein from the cell.

In one embodiment, the protease is a furin and/or furin-like protease. The terms "furin" and "furin-like protease" refer to the enzymes corresponding to EC No. 3.4.21.75. Furin is subtilisin-like proprotein convertase, which is also known as PACE (Paired basic Amino acid Cleaving Enzyme). Furin deletes sections of inactive precursor proteins to convert them into biologically active proteins. Examples of furin and/or furin-like proteases (which can also be referred to as members of the furin family of proteases) include, but are not limited to PCSK1 (also known as PC1/Pc3), PCSK2 (also known as PC2), PCSK3 (also known as furin or PACE), PCSK4 (also known as PC4), PCSK5 (also known as PCS or PC6), PCSK6 (also known as PACE4) and PCSK7 (also known as PC7/LPC, PC8, or SPC7).

The term "amino acid" or "amino acid residue", as used herein, refers to naturally occurring amino acids, unnatural amino acids, amino acid analogues and amino acid mimetics that function in a manner similar to the naturally occurring amino acids, all in their D and L stereoisomers if their structure allows such stereoisomeric forms. Amino acids are referred to herein by either their name, their commonly known three letter symbols or by the one-letter symbols recommended by the IUPAC-IUB Biochemical Nomenclature Commission.

When used in connection with amino acids, the term "naturally occurring" refers to the 20 conventional amino acids (i.e., alanine (A), cysteine (C), aspartic acid (D), glutamic acid (E), phenylalanine (F), glycine (G), histidine (H), isoleucine (I), lysine (K), leucine (L), methionine (M), asparagine (N), proline (P), glutamine (Q), arginine (R), serine (S), threonine (T), valine (V), tryptophan (W), and tyrosine (Y)), as well as selenocysteine, pyrrolysine, and pyrroline-carboxylysine.

The term "unnatural amino acid", as used herein, is meant to refer to amino acids that are not naturally encoded or found in the genetic code of any organism. They may, for example, be purely synthetic compounds. Examples of unnatural amino acids include, but are not limited to, hydroxyproline, gamma-carboxyglutamate, O-phosphoserine, azetidinecarboxylic acid, 2-aminoadipic acid, 3-aminoadipic acid, beta-alanine, aminopropionic acid, 2-aminobutyric acid, 4-aminobutyric acid, 6-aminocaproic acid, 2-aminoheptanoic acid, 2-aminoisobutyric acid, 3-aminoisobutyric acid, 2-aminopimelic acid, tertiary-butylglycine, 2,4-diaminoisobutyric acid, desmosine, 2,2'-diaminopimelic acid, 2,3-diaminoproprionic acid, N-ethylglycine, N-methylglycine, N-ethylasparagine, homoproline, hydroxylysine, allo-hydroxylysine, 3-hydroxyproline, 4-hydroxyproline, isodesmosine, allo-isoleucine, N-methylalanine, N-methylglycine, N-methylisoleucine, N-methylpentylglycine, N-methylvaline, naphthalanine, norvaline, norleucine, ornithine, D-ornithine, D-arginine, p-aminophenylalanine, pentylglycine, pipecolic acid and thioproline.

The term "amino acid analogue", as used herein, refers to compounds that have the same basic chemical structure as a naturally occurring amino acid. Amino acid analogues include the natural and unnatural amino acids which are chemically blocked, reversibly or irreversibly, or their C-terminal carboxy group, their N-terminal amino group and/or their side-chain functional groups are chemically modified. Such analogues include, but are not limited to, methionine sulfoxide, methionine sulfone, S-(carboxymethyl)-cysteine, S-(carboxymethyl)-cysteine sulfoxide, S-(carboxymethyl)-cysteine sulfone, aspartic acid-(betamethylester), N-ethylglycine, alanine carboxamide, homoserine, norleucine and methionine methyl sulfonium.

The term "amino acid mimetics", as used herein, refers to chemical compounds that have a structure that is different from the general chemical structure of an amino acid, but function in a manner similar to a naturally occurring amino acid.

According to the invention, X₁ and X₂ are selected from the group consisting of KK, KQ, KR, QK, QQ, QR, RK, RQ and RR.

In one embodiment, the at least one protease-cleavage site comprises the amino acid sequence HRRRKRSVDE (SEQ ID NO: 43) or the amino acid sequence HRRQQRSVDE (SEQ ID NO: 44).

In one embodiment, protease-cleavage of the linker results in a change in activity of the protein.

In one embodiment, the change in activity is binding or increased binding to at least one antigen.

The term "binding" according to the invention preferably relates to a specific binding. A binding agent, such as an antibody or antibody derivative, is specific for a predetermined target if it is capable of binding to said predetermined target while it is not (substantially) capable of binding to other targets.

According to the present invention, a binding agent, such as an antibody or antibody derivative, is capable of binding to a predetermined target if it has a significant affinity for said predetermined target and binds to said predetermined target in standard assays. "Affinity" or "binding affinity" is often measured by equilibrium dissociation constant (K_{D}). Preferably, the term "significant affinity" refers to the binding to a predetermined target with a dissociation constant (K_{D}) of 10⁻⁵ M or lower, 10⁻⁶ M or lower, 10⁻⁷ M or lower, 10⁻⁸ M or lower, 10⁻⁹ M or lower, 10⁻¹⁰ M or lower, 10⁻¹¹ M or lower, or 10⁻¹² M or lower.

An agent is not (substantially) capable of binding to a target if it has no significant affinity for said target and does not bind significantly, in particular does not bind detectably, to said target in standard assays. Preferably, the agent does not detectably bind to said target if present in a concentration of up to 2, preferably 10, more preferably 20, in particular 50 or 100 µg/ml or higher. Preferably, an agent has no significant affinity for a target if it binds to said target with a K_{D} that is at least 10-fold, 100-fold, 10³-fold, 10⁴-fold, 10⁵-fold, or 10⁶-fold higher than the K_{D} for binding to the predetermined target to which the agent is capable of binding. For example, if the K_{D} for binding of an agent to the target to which the agent is capable of binding is 10⁻⁷ M, the K_{D} for binding to a target for which the agent has no significant affinity would be at least 10⁻⁶ M, 10⁻⁵ M, 10⁻⁴ M, 10⁻³ M, 10⁻² M, or 10⁻¹ M.

Binding of an agent to a target can be determined experimentally using any suitable method; see, for example, Berzofsky et al., "Antibody-Antigen Interactions" In Fundamental Immunology, Paul, W. E., Ed., Raven Press New York, N Y (1984), Kuby, Janis Immunology, W. H. Freeman and Company New York, N Y (1992), and methods described herein. Affinities may be readily determined using conventional techniques, such as by equilibrium dialysis; by using the BIAcore 2000 instrument, using general procedures outlined by the manufacturer; by radioimmunoassay using radiolabeled target antigen; or by another method known to the skilled artisan. The affinity data may be analyzed, for example, by the method of Scatchard et al., Ann N.Y. Acad. ScL, 51:660 (1949). The measured affinity of a particular antibody-antigen interaction can vary if measured under different conditions, e.g., salt concentration, pH. Thus, measurements of affinity and other antigen-binding parameters, e.g., K_{D}, IC₅₀, are preferably made with standardized solutions of antibody and antigen, and a standardized buffer.

The term "increased binding" may refer to binding which is increased by at least 50%, at least 100%, at least 200%, at least 300%, at least 400% or at least 500% as compared to binding prior to protease-cleavage.

In one embodiment, protease-cleavage occurs intracellularly, e.g., within host cells as defined herein. Thus, the protease-cleavage site contained in the protease-cleavable linker may also be referred to as an intracellular processing site. A protease-cleavable linker according to the present invention may also be referred to as an *in vivo* cleavable linker.

Preferably, a protease-cleavable linker according to the present invention comprising at least one protease-cleavage site according to the present invention is cleaved/processed intracellularly/in *vivo* without co-expression of a protease (e.g., a furin and/or furin-like protease), in particular an exogenous protease. The term "co-expression", as used herein, is meant to refer to an artificial co-expression, e.g., by genetically manipulating host cells to express the protease, for example via transfection of host cells with a nucleic acid encoding the protease.

In one embodiment, the protease-cleavable linker comprises two protease-cleavage sites, wherein the two protease-cleavage sites are located at the N-terminus and at the C-terminus of the protease-cleavable linker, respectively, wherein the two protease-cleavage sites may be the same or different.

In one embodiment, PP₁ comprises at least one immunoglobulin constant region and/or at least one immunoglobulin variable region, and/or PP₂ comprises at least one immunoglobulin constant region and/or at least one immunoglobulin variable region.

In one embodiment, the protein is an antibody or antibody derivative.

In one embodiment, the protein is a single chain antibody, preferably a multispecific (e.g., bispecific, trispecific, tetraspecific, pentaspecific, hexaspecific etc.) single chain antibody. The term "single chain antibody", as used herein, also refers to antibodies or antibody derivatives comprising more than one (e.g., two) single chains, which, preferably, are associated with each other via at least one covalent or non-covalent bond (e.g., two disulfide bonds).

The term "antibody" (or "immunoglobulin") refers to a glycoprotein comprising at least two heavy (H) chains and two light (L) chains inter-connected by disulfide bonds. The term "antibody" includes monoclonal antibodies, recombinant antibodies, human antibodies, humanized antibodies, chimeric antibodies and combinations of any of the foregoing. Each heavy chain is comprised of a heavy chain variable region (VH) and a heavy chain constant region (CH). Each light chain is comprised of a light chain variable region (VL) and a light chain constant region (CL). The variable regions and constant regions are also referred to herein as variable domains and constant domains, respectively. The VH and VL regions can be further subdivided into regions of hypervariability, termed complementarity determining regions (CDRs), interspersed with regions that are more conserved, termed framework regions (FRs). Each VH and VL is composed of three CDRs and four FRs, arranged from amino-terminus to carboxy-terminus in the following order: FR1, CDR1, FR2, CDR2, FR3, CDR3, FR4. The CDRs of a VH are termed HCDR1, HCDR2 and HCDR3, the CDRs of a VL are termed LCDR1, LCDR2 and LCDR3. The variable regions of the heavy and light chains contain a binding domain that interacts with an antigen. The constant regions of an antibody comprise the heavy chain constant region (CH) and the light chain constant region (CL), wherein CH can be further subdivided into constant domain CH1, a hinge region, and constant domains CH2 and CH3 (arranged from amino-terminus to carboxy-terminus in the following order: CH1, CH2, CH3). The constant regions of the antibodies may mediate the binding of the immunoglobulin to host tissues or factors, including various cells of the immune system (e.g., effector cells) and the first component (Clq) of the classical complement system. In one embodiment, the CL region/domain referred to herein is a Cκ region/domain.

Antibodies may be derived from different species, including but not limited to mouse, rat, rabbit, guinea pig and human.

Antibodies described herein include IgA such as IgA1 or IgA2, IgG1, IgG2, IgG3, IgG4, IgE, IgM, and IgD antibodies. In various embodiments, the antibody is an IgG1 antibody, more particularly an IgG1 kappa or IgG1 lambda isotype (i.e. IgG1, κ, λ), an IgG2a antibody (e.g. IgG2a, κ, λ), an IgG2b antibody (e.g. IgG2b, κ, λ), an IgG3 antibody (e.g. IgG3, κ, λ) or an IgG4 antibody (e.g. IgG4, κ, λ). In one embodiment, the antibody or antibody derivative is an IgG1 antibody or IgG1 antibody derivative, respectively.

As used herein, "isotype" refers to the antibody class (e.g., IgM or IgG1) that is encoded by heavy chain constant region genes. "Isotype switching" refers to the phenomenon by which the class, or isotype, of an antibody changes from one Ig class to one of the other Ig classes.

The term "monoclonal antibody", as used herein, refers to a preparation of antibody molecules of single molecular composition. A monoclonal antibody displays a single binding specificity and affinity. In one embodiment, the monoclonal antibodies are produced by a hybridoma which includes a B-cell obtained from a non-human animal, e.g., a mouse, fused to an immortalized cell.

The term "hinge" or "hinge region", as used herein, refers to the flexible amino acid stretch in the central part of the heavy chains of the IgG and IgA, in particular the IgG (i.e., IgG1, IgG2, IgG3 or IgG4, especially IgG1) immunoglobulin classes, which links these two chains by disulfide bonds.

The term "antibody derivative", as used herein, refers to a molecule comprising at least the domains it is specified to comprise, but not having the overall structure of an antibody such as IgA, IgD, IgE, IgG, IgM, IgY or IgW, although still being capable of binding a target molecule. Said derivatives may be, but are not limited to functional (i.e. target binding, particularly specific target binding) antibody fragments thereof, such as Fab2, or combinations of such derivatives, for example bivalent Fabs. It also relates to an antibody to which further antibody domains have been added, such as further variable domains. In one embodiment, the term "antibody derivative" refers to the single chain antibodies as described herein. Thus, the term "antibody derivative" also includes multispecific (e.g., bispecific, trispecific, tetraspecific, pentaspecific, hexaspecific etc.) and multivalent (e.g., bivalent, trivalent, tetravalent etc.) antibodies.

In one embodiment,
PP₁ comprises an immunoglobulin constant region and PP₂ comprises an immunoglobulin variable region,
PP₁ comprises an immunoglobulin variable region and PP₂ comprises an immunoglobulin constant region,
PP₁ comprises an immunoglobulin constant region and PP₂ comprises an immunoglobulin constant region, or
PP₁ comprises an immunoglobulin variable region and PP₂ comprises an immunoglobulin variable region.

In one embodiment, the protein is selected from the group consisting of a single chain (sC) monoclonal IgG (e.g., as described in Spies et al., J Mol Imm. 2015, 67:95-106), a single light chain (sLC) bivalent, bispecific Tandem-lgG (e.g., as described in WO 2009/052081 A2), a single light chain (sLC) multispecific Tandem-IgG (e.g., as described in Wu et al., Nat. Biotechnol. 2007, 25:1290-1297), a single chain (sC) monovalent, bispecific Tandem-like-lgG (e.g., as described in Brinkmann & Kontermann, MAbs 2017, 9:182-212), a trispecific CODV-sC-Fab-lgG (e.g., as described in WO 2017/180913 A2 and/or Xu et al., Science 2017, 358(6359):85-90) and a single chain (sC) bispecific IgG (e.g., as described in Fitzgerald et al., Mol Cancer Ther. 2013, 13:410-25).

In one embodiment, the protein comprises a polypeptide chain having a formula selected from the group consisting of:
(a)

   VL-CL-PCL-VH-CH1-hinge-CH2-CH3,

   wherein
   VL is an immunoglobulin light chain variable region;
   CL is an immunoglobulin light chain constant region;
   VH is an immunoglobulin heavy chain variable region;
   CH1 is an immunoglobulin CH1 heavy chain constant region;
   hinge is an immunoglobulin hinge region;
   CH2 is an immunoglobulin CH2 heavy chain constant region;
   CH3 is an immunoglobulin CH3 heavy chain constant region; and
   PCL is the protease-cleavable linker;
(b)

   VLₐ-CLₐ-PCL-VL_{b}-CL_{b},

   wherein
   VLₐ is a first immunoglobulin light chain variable region;
   VL_{b} is a second immunoglobulin light chain variable region;
   CLₐ is a first immunoglobulin light chain constant region;
   CL_{b} is a second immunoglobulin light chain constant region; and
   PCL is the protease-cleavable linker; and
(c)

   VLₐ-CLₐ-PCL-VL_{b}-CL_{b}-L-hinge-CH2-CH3

   wherein
   VLₐ is a first immunoglobulin light chain variable region;
   VL_{b} is a second immunoglobulin light chain variable region;
   CLₐ is a first immunoglobulin light chain constant region;
   CL_{b} is a second immunoglobulin light chain constant region;
   L is an optional linker;
   hinge is an immunoglobulin hinge region;
   CH2 is an immunoglobulin CH2 heavy chain constant region;
   CH3 is an immunoglobulin CH3 heavy chain constant region; and
   PCL is the protease-cleavable linker.

The symbol (-), when used in connection with the formulas disclosed herein, is meant to refer to a covalent bond, in particular a peptide bond, or one or more amino acid residues, e.g., a peptide linker as described herein.

In one embodiment, L is a peptide linker as defined herein, preferably having a length of 5 to 25 amino acid residues, preferably 10 to 20 amino acid residues, preferably 12 to 18 amino acid residues, more preferably 13 to 17 amino acid residues, even more preferably 14 to 16 amino acid residues, e.g., 15 amino acid residues. In one embodiment, L is a glycine-serine-rich linker, wherein at least 50%, preferably at least 60%, more preferably at least 70%, more preferably at least 80%, even more preferably at least 85% of the amino acids are a glycine or serine residue, respectively. In one embodiment, L is a glycine linker, serine linker or glycine-serine linker. In one embodiment, L has the sequence (G₄S)ₙ (SEQ ID NO: 51), wherein n is an integer selected from 1 to 5, e.g., 1 to 3 or 2 to 4. In one embodiment, L has the sequence (G₄S)₃ (SEQ ID NO: 55).

In one embodiment, the protein comprises a polypeptide chain having the formula VL-CL-PCL-VH-CH1-hinge-CH2-CH3, wherein VL pairs with VH to form an antigen binding site.

In one embodiment, the antigen binding site becomes active or exhibits increased activity upon protease-cleavage of the protease-cleavable linker.

Preferably, the term "pairs with", as used herein, refers to the dimerization of immunoglobulin constant regions or immunoglobulin variable regions as it occurs in a regular, e.g., naturally occurring, immunoglobulin, in particular IgG.

The term "antigen binding site" (also called "paratope"), as used herein, refers to the part of an antibody which recognizes and binds to an antigen.

In one embodiment, the protein comprises two copies of the polypeptide chain having the formula VL-CL-PCL-VH-CH1-hinge-CH2-CH3, wherein the two copies are associated with each other via at least two disulfide bonds. In one embodiment, the protein comprises a first polypeptide chain having the formula VLₐ-CLₐ-PCL-VHₐ-CH1ₐ-hinge-CH2-CH3, and a second polypeptide chain having the formula VL_{b}-CL_{b}-PCL-VH_{b}-CH1_{b}-hinge-CH2-CH3,
wherein
VLₐ is a first immunoglobulin light chain variable region;
VL_{b} is a second immunoglobulin light chain variable region;
CLₐ is a first immunoglobulin light chain constant region;
CL_{b} is a second immunoglobulin light chain constant region;
VHₐ is a first immunoglobulin heavy chain variable region;
VH_{b} is a second immunoglobulin heavy chain variable region;
CH1ₐ is a first immunoglobulin CH1 heavy chain constant region; and
CH1_{b} is a second immunoglobulin CH1 heavy chain constant region;
wherein VLₐ pairs with VHₐ to form an antigen binding site binding to antigen A, and wherein VL_{b} pairs with VH_{b} to form an antigen binding site binding to antigen B; and
wherein the first polypeptide chain is associated with the second polypeptide chain via at least two disulfide bonds and, optionally, via at least one additional Fc interaction.

In one embodiment, the antigen binding site A and/or the antigen binding site B become active or exhibit increased activity upon protease-cleavage of the protease-cleavable linkers.

The term "additional Fc interaction", as used herein, refers to an interaction between two Fc molecules (e.g., two heterologous Fc molecules), in particular between the CH3 domains of two Fc molecules, in addition to the at least two disulfide bonds referred to herein. Examples of additional Fc interactions include, but are not limited to, knob-into-hole interactions (see below for details), hydrophobic interactions (e.g., due to the introduction of specific mutations; see, e.g., Von Kreudenstein et al., MAbs 2013, 5(5):646-654), electrostatic interactions (e.g., due to electrostatic steering; see, e.g., Gunasekaran et al., J Biol Chem 2010, 285(25):19637-19646), interactions due to alternating CH3 segments of IgG and IgA (SEED technology; see, e.g., Davis et al., Protein Eng Des Sel 2010, 23(4):195-202), interactions due to the fusion of a heterodimeric module, such as a cleavable leucine zipper, in the C-terminus of the CH3 domain (LUZ-Y technology; see, e.g., Wranik et al., J Biol Chem 2012, 287(52):43331-43339).

In one embodiment, the at least one additional Fc interaction is at least one knob-into-hole interaction (e.g., two knob-into-hole interactions).

The term "knob-into-hole interaction", as used herein, refers to an interaction which is based on knob-into-hole amino acid changes. Such changes represent a well-known rational design strategy in antibody engineering used for heterodimerization of the heavy (H) immunoglobulin chains, e.g., in the production of bispecific antibodies, as described in, e.g., Merchant et al., Nat. Biotechnol. 1998, 16:677-681. Amino acid changes are engineered in order to create a knob on the constant region (e.g., on the CH3) of a first immunoglobulin chain or first antibody and a hole on the constant region (e.g., on the CH3) of a second immunoglobulin chain or second antibody. The knob is represented by an amino acid that belongs to the "very large" IMGT volume class of amino acids (e.g., tyrosine, Y), whereas the hole is represented by an amino acid that belongs to the "small" IMGT volume class (e.g., threonine; T) - for the IMGT classes of the 20 conventional amino acids, see Pommié et al., J. Mol. Recognit. 2004, 17:17-32. Particularly preferred knob-into-hole amino acid changes, in accordance with the present invention, include, but are not limited to those described in Spies et al., J Mol Imm. 2015, 67:95-106.

In one embodiment, the protein comprises a first polypeptide chain having the formula VL_{b}-L1-VLₐ-L2-CL, a second polypeptide chain having the formula VHₐ-L3-VH_{b}-L4-CH1-hinge-CH2-CH3 and a third polypeptide chain having the formula VL_{c}-CL_{c}-PCL-VH_{c}-CH1_{c}-hinge-CH2-CH3, wherein
VLₐ is a first immunoglobulin light chain variable region;
VL_{b} is a second immunoglobulin light chain variable region;
VL_{c} is a third immunoglobulin light chain variable region;
CL is an immunoglobulin light chain constant region;
CL_{c} is an immunoglobulin light chain constant region;
VHₐ is a first immunoglobulin heavy chain variable region;
VH_{b} is a second immunoglobulin heavy chain variable region;
VH_{c} is a third immunoglobulin heavy chain variable region;
CH1 is an immunoglobulin CH1 heavy chain constant region;
CH1_{c} is an immunoglobulin CH1 heavy chain constant region;
hinge is an immunoglobulin hinge region;
CH2 is an immunoglobulin CH2 heavy chain constant region; and
CH3 is an immunoglobulin CH3 heavy chain constant region;
L1, L2, L3 and L4 are linkers;
wherein VLₐ pairs with VHₐ to form an antigen binding site binding to antigen A, wherein VL_{b} pairs with VH_{b} to form an antigen binding site binding to antigen B, and wherein VL_{c} pairs with VH_{c} to form an antigen binding site binding to antigen C; and
wherein the second polypeptide chain is associated with the third polypeptide chain via at least two disulfide bonds and, optionally, via at least one additional Fc interaction.

In one embodiment, the antigen binding site C becomes active or exhibits increased activity upon protease-cleavage of the protease-cleavable linker.

In one embodiment, the at least one additional Fc interaction is at least one knob-into-hole interaction (e.g., two knob-into-hole interactions).

In one embodiment, L1, L2, L3 and L4 are independently selected from peptide linkers as defined herein, preferably having a length of between 1 to 25, preferably 2 to 25, more preferably 5 to 20, more preferably 10 to 20 amino acid residues. In one embodiment, L1, L2, L3 and L4 are glycine-serine-rich linkers, wherein at least 50%, preferably at least 60%, more preferably at least 70%, more preferably at least 80%, even more preferably at least 85% of the amino acids are a glycine or serine residue, respectively. In one embodiment, L1, L2, L3 and L4 are glycine linkers, serine linkers or glycine-serine linkers, respectively. In one embodiment, L1, L2, L3 and L4 have the sequence (G₄S)ₙ (SEQ ID NO: 51), wherein n is an integer independently selected from 1 to 5, e.g., 1 to 3 or 2 to 4.

In one embodiment, the protein comprises a first polypeptide chain having the formula VLₐ-CLₐ-PCL-VL_{b}-CL_{b}, and a second polypeptide chain having the formula VHₐ-CH1ₐ-L-VH_{b}-CH1_{b}-hinge-CH2-CH3,
wherein
VHₐ is a first immunoglobulin heavy chain variable region;
VH_{b} is a second immunoglobulin heavy chain variable region;
CH1ₐ is a first immunoglobulin CH1 heavy chain constant region;
CH1_{b} is a second immunoglobulin CH1 heavy chain constant region;
L is an optional linker;
hinge is an immunoglobulin hinge region;
CH2 is an immunoglobulin CH2 heavy chain constant region; and
CH3 is an immunoglobulin CH3 heavy chain constant region;
wherein VLₐ pairs with VHₐ to form an antigen binding site binding to antigen A, and wherein VL_{b} pairs with VH_{b} to form an antigen binding site binding to antigen B.

In one embodiment, the antigen binding site B becomes active or exhibits increased activity upon protease-cleavage of the protease-cleavable linker.

In one embodiment, L is a peptide linker as defined herein. In one embodiment, L has a length of 5 to 25 amino acid residues, preferably 10 to 20 amino acid residues.

In one embodiment, the protein comprises two copies of the first polypeptide chain and two copies of the second polypeptide chain, wherein the two copies of the second polypeptide chain are associated with each other via at least two disulfide bonds.

In one embodiment, the protein comprises a first polypeptide chain having the formula VLₐ-CLₐ-PCL-VL_{b}-CL_{b}, a second polypeptide chain having the formula VL_{c}-CL_{c}-PCL-VL_{d}-CL_{d}, a third polypeptide chain having the formula VHₐ-CH1ₐ-L-VH_{b}-CH1_{b}-hinge-CH2-CH3, and a fourth polypeptide chain having the formula VH_{c}-CH1_{c}-L-VH_{d}-CH1_{d}-hinge-CH2-CH3,
wherein
VLₐ is a first immunoglobulin light chain variable region;
VL_{b} is a second immunoglobulin light chain variable region;
VL_{c} is a third immunoglobulin light chain variable region;
VL_{d} is a fourth immunoglobulin light chain variable region;
CLₐ is a first immunoglobulin light chain constant region;
CL_{b} is a second immunoglobulin light chain constant region;
CL_{c} is a third immunoglobulin light chain constant region;
CL_{d} is a fourth immunoglobulin light chain constant region;
VHₐ is a first immunoglobulin heavy chain variable region;
VH_{b} is a second immunoglobulin heavy chain variable region;
VH_{c} is a third immunoglobulin heavy chain variable region;
VH_{d} is a fourth immunoglobulin heavy chain variable region;
CH1ₐ is a first immunoglobulin CH1 heavy chain constant region;
CH1_{b} is a second immunoglobulin CH1 heavy chain constant region;
CH1_{c} is a third immunoglobulin CH1 heavy chain constant region;
CH1_{d} is a fourth immunoglobulin CH1 heavy chain constant region;
L is an optional linker;
hinge is an immunoglobulin hinge region;
CH2 is an immunoglobulin CH2 heavy chain constant region; and
CH3 is an immunoglobulin CH3 heavy chain constant region;
wherein VLₐ pairs with VHₐ to form an antigen binding site binding to antigen A, wherein VL_{b} pairs with VH_{b} to form an antigen binding site binding to antigen B, wherein VL_{c} pairs with VH_{c} to form an antigen binding site binding to antigen C, and wherein VL_{d} pairs with VH_{d} to form an antigen binding site binding to antigen D; and
wherein the third polypeptide chain is associated with the fourth polypeptide chain via at least two disulfide bonds and, optionally, via at least one additional Fc interaction.

In one embodiment, the antigen binding site B and/or the antigen binding site D become active or exhibit increased activity upon protease-cleavage of the protease-cleavable linkers.

In one embodiment, the at least one additional Fc interaction is at least one knob-into-hole interaction (e.g., two knob-into-hole interactions).

In one embodiment, L is a peptide linker as defined herein. In one embodiment, L has a length of 5 to 25 amino acid residues, preferably 10 to 20 amino acid residues.

In one embodiment, the protein comprises a first polypeptide chain having the formula VLₐ-CLₐ-PCL-VL_{b}-CL_{b}-L-hinge-CH2-CH3, and a second polypeptide chain having the formula VHₐ-CH1ₐ-L-VH_{B}-CH 1 _{b}-hinge-CH2-CH3,
wherein
VH, is a first immunoglobulin heavy chain variable region;
VH_{b} is a second immunoglobulin heavy chain variable region;
CH1ₐ is a first immunoglobulin CH1 heavy chain constant region;
CH1_{b} is a second immunoglobulin CH1 heavy chain constant region;
L is an optional linker;
hinge is an immunoglobulin hinge region;
CH2 is an immunoglobulin CH2 heavy chain constant region; and
CH3 is an immunoglobulin CH3 heavy chain constant region;
wherein VLₐ pairs with VHₐ to form an antigen binding site binding to antigen A, and wherein VL_{b} pairs with VH_{b} to form an antigen binding site binding to antigen B; and
wherein the first polypeptide chain is associated with the second polypeptide chain via at least two disulfide bonds and, optionally, via at least one additional Fc interaction.

In one embodiment, the antigen binding site B becomes active or exhibits increased activity upon protease-cleavage of the protease-cleavable linker.

In one embodiment, the at least one additional Fc interaction is at least one knob-into-hole interaction (e.g., two knob-into-hole interactions).

In one embodiment, L is a peptide linker as defined herein. In one embodiment, L has a length of 5 to 25 amino acid residues, preferably 10 to 20 amino acid residues.

In another aspect, the present invention relates to a nucleic acid or set of nucleic acids encoding the protein as defined herein.

A "nucleic acid" (or "nucleic acid molecule") is, according to the invention, preferably deoxyribonucleic acid (DNA) or ribonucleic acid (RNA). A nucleic acid molecule may according to the invention be in the form of a molecule, which is single-stranded or double-stranded and linear or covalently closed to form a circle.

The term "DNA" relates to a molecule which comprises deoxyribonucleotide residues and preferably is entirely or substantially composed of deoxyribonucleotide residues. "Deoxyribonucleotide" relates to a nucleotide which lacks a hydroxyl group at the 2'-position of a beta-D-ribofuranosyl group. The term "DNA" comprises isolated DNA such as partially or completely purified DNA, essentially pure DNA, synthetic DNA, and recombinantly generated DNA and includes modified DNA which differs from naturally occurring DNA by addition, deletion, substitution and/or alteration of one or more nucleotides. Such alterations can include addition of non-nucleotide material, such as to the end(s) of a DNA or internally, for example at one or more nucleotides of the DNA. Nucleotides in DNA molecules can also comprise non-standard nucleotides, such as non-naturally occurring nucleotides or chemically synthesized nucleotides. These altered DNAs can be referred to as analogues or analogues of naturally-occurring DNA. When used in connection with nucleotides, the term "naturally occurring" refers to the bases adenine (A), cytosine (C), guanine (G), thymine (T), and uracil (U).

The term "RNA" relates to a molecule which comprises ribonucleotide residues and preferably is entirely or substantially composed of ribonucleotide residues. "Ribonucleotide" relates to a nucleotide with a hydroxyl group at the 2'-position of a beta-D-ribofuranosyl group. The term "RNA" comprises isolated RNA such as partially or completely purified RNA, essentially pure RNA, synthetic RNA, and recombinantly generated RNA and includes modified RNA which differs from naturally occurring RNA by addition, deletion, substitution and/or alteration of one or more nucleotides. Such alterations can include addition of non-nucleotide material, such as to the end(s) of a RNA or internally, for example at one or more nucleotides of the RNA. Nucleotides in RNA molecules can also comprise non-standard nucleotides, such as non-naturally occurring nucleotides or chemically synthesized nucleotides or deoxynucleotides. These altered RNAs can be referred to as analogues or analogues of naturally-occurring RNA. According to the invention, "RNA" refers to single-stranded RNA or double-stranded RNA. In one embodiment, the RNA is mRNA, e.g., *in vitro* transcribed RNA (IVT RNA) or synthetic RNA. The RNA may also be modified, e.g., with one or more modifications increasing the stability (e.g., the half-life) of the RNA. Such modifications are known to a person skilled in the art and include, for example, 5'-caps or 5'cap analogues

In another aspect, the present invention relates to a vector or set of vectors comprising the nucleic acid or set of nucleic acids as defined herein.

The term "vector", as used herein, includes all vectors known to the skilled person, including plasmid vectors, cosmid vectors, phage vectors, such as lambda phage, viral vectors, such as adenoviral or baculoviral vectors, or artificial chromosome vectors such as bacterial artificial chromosomes (BAC), yeast artificial chromosomes (YAC), or P1 artificial chromosomes (PAC).

Said vectors include expression as well as cloning vectors. Expression vectors comprise plasmids as well as viral vectors and generally contain a desired coding sequence and appropriate DNA sequences necessary for the expression of the operably linked coding sequence in a particular host organism (e.g., bacteria, yeast, plant, insect, or mammal) or in *in vitro* expression systems. Cloning vectors are generally used to engineer and amplify a certain desired DNA fragment and may lack functional sequences needed for expression of the desired DNA fragments.

Alternatively, the nucleic acid molecule according to the present invention may be integrated into a genome, e.g., the genome of a host cell. Means and methods to integrate a particular nucleic acid molecule into a genome are known to a person skilled in the art.

In another aspect, the present invention relates to a host cell comprising a protein as defined herein, a nucleic acid or set of nucleic acids as defined herein, or a vector or set of vectors as defined herein.

The term "cell" or "host cell" preferably relates to an intact cell, i.e. a cell with an intact membrane that has not released its normal intracellular components such as enzymes, organelles, or genetic material. An intact cell preferably is a viable cell, i.e. a living cell capable of carrying out its normal metabolic functions. Preferably said term relates according to the invention to any cell which can be transfected with an exogenous nucleic acid. Preferably, the cell when transfected with an exogenous nucleic acid and transferred to a recipient can express the nucleic acid in the recipient. The term "cell" includes bacterial cells; other useful cells are yeast cells, fungal cells or mammalian cells. Suitable bacterial cells include cells from gram-negative bacterial strains such as strains of *Escherichia coli, Proteus,* and *Pseudomonas,* and gram-positive bacterial strains such as strains of *Bacillus, Streptomyces, Staphylococcus,* and *Lactococcus.* Suitable fungal cell include cells from species of *Trichoderma,* Neurospora, and *Aspergillus.* Suitable yeast cells include cells from species of *Saccharomyces* (Tor example Saccharomyces cerevisiae), *Schizosaccharomyces* (for example *Schizosaccharomyces pombe), Pichia* (for example *Pichia pastoris* and *Pichia methanolica),* and *Hansenula.* Suitable mammalian cells include for example CHO cells, BHK cells, HeLa cells, COS cells, HEK293 (e.g., HEK 293-FS) and the like. However, amphibian cells, insect cells, plant cells, and any other cells used in the art for the expression of heterologous proteins can be used as well. The "cell" or "host cell" may be isolated or part of a tissue or organism, in particular a "non-human organism". The term "non-human organism", as used herein, is meant to include non-human primates or other animals, in particular mammals, such as cows, horses, pigs, sheep, goats, dogs, cats, rabbits or rodents, such as mice, rats, guinea pigs and hamsters.

In one embodiment, the host cell expresses an endogenous or exogenous furin and/or furin-like protease.

In one embodiment, the host cell is a mammalian cell. In one embodiment, the mammalian cell is a CHO cell, preferably selected from the group consisting of CHO 9E4, ExpiCHO, CHO DG44 and CHO K1.

In one aspect, the present invention relates to a method of producing a protein, e.g., a protein as defined herein, comprising the steps:
i) culturing a host cell as defined herein; and
ii) isolating the protein from the host cell.

In one embodiment, said host cell is cultured under conditions allowing the expression of said protein.

In another aspect, the present invention relates to a protein obtainable (or obtained) by the method as defined herein.

In yet another aspect, the present invention relates to a protein obtainable (or obtained) by furin and/or furin-like protease-cleavage of a protein as defined herein.

In one embodiment, the protein as defined herein is a therapeutically active protein.

The term "therapeutically active protein", as used herein, refers to a protein which is suitable for therapy, i.e., can be used to treat a disease or disorder. In one embodiment, the therapeutically active protein is an antibody or antibody-derivative binding to a therapeutically relevant antigen.

For all antibodies and antibody derivatives, as described herein, the therapeutically relevant antigen is for example independently selected for each specificity from the group consisting of IL-4, IL-13, PD-1, 4.1BB, OX40 and GITR. For all bispecific antibodies and antibody derivatives, as described herein, the antigen pair is for example selected from the group consisting of the antigen pairs IL-4 and IL-13, PD-1 and OX40, PD-1 and GITR, and PD-1 and 4.1BB. For all trispecific antibodies or antibody derivatives described herein, two antigens are for example selected from the group consisting of the antigen pairs IL-4 and IL-13, PD-1 and OX40, PD-1 and GITR, and PD-1 and 4.1BB. The third and fourth antigens can be another antigen selected from the list above. In one embodiment of a trispecific antibody or antibody derivative, as described herein, the antigens are PD-1, GITR and OX40.

In one aspect, the present invention relates to a protein as defined herein for use in therapy.

In another aspect, the present invention relates to the use of a protein as defined herein in the manufacture of a medicament.

Also disclosed but not forming part of the present invention is a method of treating a disease or disorder, comprising administering an effective amount of a protein as defined herein to a subject in need thereof.

The term "medicament", as used herein, refers to a substance/composition used in therapy, i.e., in the treatment of a disease or disorder.

By "treat" is meant to administer a compound or composition or a combination of compounds or compositions to a subject in order to prevent or eliminate a disease or disorder; arrest or slow a disease or disorder in a subject; inhibit or slow the development of a new disease or disorder in a subject; decrease the frequency or severity of symptoms and/or recurrences in a subject who currently has or who previously has had a disease or disorder; and/or prolong, i.e., increase, the lifespan of the subject.

In particular, the term "treating/treatment of a disease or disorder" includes curing, shortening the duration, ameliorating, preventing, slowing down or inhibiting progression or worsening, or preventing or delaying the onset of a disease or disorder or the symptoms thereof.

According to the invention, the term "disease" refers to any pathological state, in particular cancer, infectious diseases, inflammatory diseases, autoimmune disorders, and transplant rejections.

As used herein, the term "cancer" includes a disease characterized by aberrantly regulated cellular growth, proliferation, differentiation, adhesion, and/or migration. By "cancer cell" is meant an abnormal cell that grows by a rapid, uncontrolled cellular proliferation and continues to grow after the stimuli that initiated the new growth cease. The term "cancer" according to the invention comprises leukemias, seminomas, melanomas, teratomas, lymphomas, neuroblastomas, gliomas, rectal cancer, endometrial cancer, kidney cancer, adrenal cancer, thyroid cancer, blood cancer, skin cancer, cancer of the brain, cervical cancer, intestinal cancer, liver cancer, colon cancer, stomach cancer, intestine cancer, head and neck cancer, gastrointestinal cancer, lymph node cancer, esophagus cancer, colorectal cancer, pancreas cancer, ear, nose and throat (ENT) cancer, breast cancer, prostate cancer, cancer of the uterus, ovarian cancer and lung cancer and the metastases thereof. Examples thereof are lung carcinomas, mamma carcinomas, prostate carcinomas, colon carcinomas, renal cell carcinomas, cervical carcinomas, or metastases of the cancer types or tumors described above.

The term "cancer" according to the invention also comprises cancer metastases. By "metastasis" is meant the spread of cancer cells from its original site to another part of the body. The formation of metastasis is a very complex process and depends on detachment of malignant cells from the primary tumor, invasion of the extracellular matrix, penetration of the endothelial basement membranes to enter the body cavity and vessels, and then, after being transported by the blood, infiltration of target organs. Finally, the growth of a new tumor, i.e. a secondary tumor or metastatic tumor, at the target site depends on angiogenesis. Tumor metastasis often occurs even after the removal of the primary tumor because tumor cells or components may remain and develop metastatic potential. In one embodiment, the term "metastasis" according to the invention relates to "distant metastasis" which relates to a metastasis which is remote from the primary tumor and the regional lymph node system.

The term "infectious disease" refers to any disease which can be transmitted from individual to individual or from organism to organism, and is caused by a microbial agent (e.g. common cold). Examples of infectious diseases include viral infectious diseases, such as AIDS (HIV), hepatitis A, B or C, herpes, herpes zoster (chicken-pox), German measles (rubella virus), yellow fever, dengue etc. flaviviruses, influenza viruses, hemorrhagic infectious diseases (Marburg or Ebola viruses), and severe acute respiratory syndrome (SARS), bacterial infectious diseases, such as Legionnaire's disease *(Legionella),* sexually transmitted diseases (e.g. chlamydia or gonorrhea), gastric ulcer *(Helicobacter),* cholera *(Vibrio),* tuberculosis, diphtheria, infections by *E. coli, Staphylococci, Salmonella* or *Streptococci* (tetanus); infections by protozoan pathogens such as malaria, sleeping sickness, leishmaniasis; toxoplasmosis, i.e. infections by Plasmodium, Trypanosoma, Leishmania and Toxoplasma; or fungal infections, which are caused, e.g., by *Cryptococcus neoformans, Histoplasma capsulatum, Coccidioides immitis, Blastomyces dermatitidis* or *Candida albicans.*

The term "inflammatory disease" refers to any disease, which is characterized by or associated with high levels of inflammation in tissues, in particular connective tissues, or degeneration of these tissues. A chronic inflammatory disease is a medical condition which is characterized by persistent inflammation. Examples of (chronic) inflammatory diseases include celiac disease, vasculitis, lupus, chronic obstructive pulmonary disease (COPD), irritable bowel disease, atherosclerosis, arthritis, ankylosing spondylitis, Crohn's disease, colitis, chronic active hepatitis, dermatitis and psoriasis.

The term "autoimmune disorder" refers to any disease/disorder in which the body produces an immunogenic (i.e. immune system) response to some constituent of its own tissue. In other words, the immune system loses its ability to recognize some tissue or system within the body as self and targets and attacks it as if it were foreign. Autoimmune diseases can be classified into those in which predominantly one organ is affected (e.g. hemolytic anemia and anti-immune thyroiditis), and those in which the autoimmune disease process is diffused through many tissues (e.g. systemic lupus erythematosus). For example, multiple sclerosis is thought to be caused by T cells attacking the sheaths that surround the nerve fibers of the brain and spinal cord. This results in loss of coordination, weakness, and blurred vision. Autoimmune diseases are known in the art and include, for instance, Hashimoto's thyroiditis, Grave's disease, lupus, multiple sclerosis, rheumatic arthritis, hemolytic anemia, anti-immune thyroiditis, systemic lupus erythematosus, celiac disease, Crohn's disease, colitis, diabetes, scleroderma, psoriasis, and the like.

The term "transplant rejection" refers to the rejection of a transplanted tissue or organ by the recipient's immune system, which may, ultimately, destroy the transplanted tissue or organ.

The term "effective amount", as used herein, refers, in particular, to a "therapeutically effective amount", which is an amount that achieves a desired therapeutic reaction or a desired therapeutic effect alone or together with further doses, preferably without causing unacceptable side-effects. In the case of treatment of a particular disease or of a particular condition, the desired reaction preferably relates to inhibition of the course of the disease. This comprises slowing down the progress of the disease and, in particular, interrupting or reversing the progress of the disease. The desired reaction in a treatment of a disease or of a condition may also be delay of the onset or a prevention of the onset of said disease or said condition. An effective amount of a protein described herein will depend on the condition to be treated, the severeness of the disease, the individual parameters of the subject, including age, physiological condition, size and weight, the duration of treatment, the type of an accompanying therapy (if present), the specific route of administration and similar factors. Accordingly, the doses administered of the agents described herein may depend on several of such parameters. In the case that a reaction in a subject is insufficient with an initial dose, higher doses (or effectively higher doses achieved by a different, more localized route of administration) may be used.

The term "subject" means according to the invention a subject for treatment, in particular a diseased subject (also referred to as "patient"), including human beings, non-human primates or other animals, in particular mammals, such as cows, horses, pigs, sheep, goats, dogs, cats, rabbits or rodents, such as mice, rats, guinea pigs and hamsters. In one embodiment, the subject/patient is a human being.

In yet another aspect, the present invention relates to a pharmaceutical composition or kit comprising a protein as defined above. In one embodiment, the pharmaceutical composition further comprises a pharmaceutically acceptable carrier.

The pharmaceutical compositions of the invention can be selected for parenteral delivery. Alternatively, the compositions can be selected for inhalation or for delivery through the digestive tract, such as orally. The preparation of such pharmaceutically acceptable compositions is within the skill of the art.

The term "pharmaceutically acceptable carrier" or "physiologically acceptable carrier" as used herein refers to one or more formulation materials suitable for accomplishing or enhancing the delivery of an antibody-like binding protein. The primary carrier in a pharmaceutical composition can be either aqueous or non-aqueous in nature. For example, a suitable vehicle or carrier for injection can be water, physiological saline solution, or artificial cerebrospinal fluid, possibly supplemented with other materials common in compositions for parenteral administration. Neutral buffered saline or saline mixed with serum albumin are further exemplary vehicles. Other exemplary pharmaceutical compositions comprise Tris buffer of about pH 7.0-8.5, or acetate buffer of about pH 4.0-5.5, which can further include sorbitol or a suitable substitute. In one embodiment of the invention, antibody-like binding protein compositions can be prepared for storage by mixing the selected composition having the desired degree of purity with optional formulation agents in the form of a lyophilized cake or an aqueous solution. Further, the antibody-like binding protein can be formulated as a lyophilizate using appropriate excipients such as sucrose.

The pharmaceutical composition can contain formulation materials for modifying, maintaining, or preserving, for example, the pH, osmolarity, viscosity, clarity, color, isotonicity, odor, sterility, stability, rate of dissolution or release, adsorption, or penetration of the composition. Suitable formulation materials include, but are not limited to, amino acids (such as glycine, glutamine, asparagine, arginine, or lysine), antimicrobials, antioxidants (such as ascorbic acid, sodium sulfite, or sodium hydrogen-sulfite ), buffers (such as borate, bicarbonate, Tris-HCl, citrates, phosphates, or other organic acids), bulking agents (such as mannitol or glycine), chelating agents (such as ethylenediaminetetraacetic acid (EDTA)), complexing agents (such as caffeine, polyvinylpyrrolidone, beta-cyclodextrin, or hydroxypropylbeta-cyclodextrin), fillers, monosaccharides, disaccharides, and other carbohydrates (such as glucose, mannose, or dextrins), proteins (such as serum albumin, gelatin, or immunoglobulins), coloring, flavoring and diluting agents, emulsifying agents, hydrophilic polymers (such as polyvinylpyrrolidone), low molecular weight polypeptides, salt-forming counterions (such as sodium), preservatives (such as benzalkonium chloride, benzoic acid, salicylic acid, thimerosal, phenethyl alcohol, methylparaben, propylparaben, chlorhexidine, sorbic acid, or hydrogen peroxide), solvents (such as glycerin, propylene glycol, or polyethylene glycol), sugar alcohols (such as mannitol or sorbitol), suspending agents, surfactants or wetting agents (such as pluronics; PEG; sorbitan esters; polysorbates such as polysorbate 20 or polysorbate 80; triton; tromethamine; lecithin; cholesterol or tyloxapal), stability enhancing agents (such as sucrose or sorbitol), tonicity enhancing agents (such as alkali metal halides, preferably sodium or potassium chloride - or mannitol sorbitol), delivery vehicles, diluents, excipients and/or pharmaceutical adjuvants (see, e.g., REMINGTON's PHARMACEUTICAL SCIENCES (18th Ed., A.R. Gennaro, ed., Mack Publishing Company 1990), and subsequent editions of the same).

As used herein, the term "kit of parts (in short: kit)" refers to an article of manufacture comprising one or more containers and, optionally, a data carrier. Said one or more containers may be filled with one or more of the above mentioned (re-)agents. Additional containers may be included in the kit that contain, e.g., diluents, buffers and further reagents. Said data carrier may be a non-electronical data carrier, e.g., a graphical data carrier such as an information leaflet, an information sheet, a bar code or an access code, or an electronical data carrier such as a compact disk (CD), a digital versatile disk (DVD), a microchip or another semiconductor-based electronical data carrier. The access code may allow the access to a database, e.g., an internet database, a centralized, or a decentralized database. Said data carrier may comprise instructions for the use of the agents of the present invention, e.g., proteins and pharmaceutical compositions as well as related agents, such as nucleic acid molecules and host cells, as described herein.

In another aspect, the present invention relates to a protein comprising a first polypeptide chain having the formula VL_{b}-L1-VLₐ-L2-CL, a second polypeptide chain having the formula VHₐ-L3-VH_{b}-L4-CH1-hinge-CH2-CH3 and a third polypeptide chain having the formula VL_{c}-CL_{c}-PCL-VH_{c}-CH1_{c}-hinge-CH2-CH3,
wherein
VLₐ is a first immunoglobulin light chain variable region;
VL_{b} is a second immunoglobulin light chain variable region;
VL_{c} is a third immunoglobulin light chain variable region;
CL is an immunoglobulin light chain constant region;
CL_{c} is an immunoglobulin light chain constant region;
VHₐ is a first immunoglobulin heavy chain variable region;
VH_{b} is a second immunoglobulin heavy chain variable region;
VH_{c} is a third immunoglobulin heavy chain variable region;
CH1 is an immunoglobulin CH1 heavy chain constant region;
CH1_{c} is an immunoglobulin CH1 heavy chain constant region;
hinge is an immunoglobulin hinge region;
CH2 is an immunoglobulin CH2 heavy chain constant region; and
CH3 is an immunoglobulin CH3 heavy chain constant region;
PCL is a protease-cleavable linker;
L1, L2, L3 and L4 are linkers;
wherein VLₐ pairs with VHₐ to form an antigen binding site binding to antigen A, wherein VL_{b} pairs with VH_{b} to form an antigen binding site binding to antigen B, and wherein VL_{c} pairs with VH_{c} to form an antigen binding site binding to antigen C; and
wherein the second polypeptide chain is associated with the third polypeptide chain via at least two disulfide bonds and, optionally, via at least one additional Fc interaction.

In one embodiment, the antigen binding site C becomes active or exhibits increased activity upon protease-cleavage of the protease-cleavable linker.

The protease-cleavable linker is a protease-cleavable linker as defined herein.

In one embodiment, the at least one additional Fc interaction is at least one knob-into-hole interaction (e.g., two knob-into-hole interactions).

In one embodiment, L1, L2, L3 and L4 are independently selected from peptide linkers as defined herein, preferably having a length of between 1 to 25, preferably 2 to 25, more preferably 5 to 20, more preferably 10 to 20 amino acid residues. In one embodiment, L1, L2, L3 and L4 are glycine-serine-rich linkers, wherein at least 50%, preferably at least 60%, more preferably at least 70%, more preferably at least 80%, even more preferably at least 85% of the amino acids are a glycine or serine residue, respectively. In one embodiment, L1, L2, L3 and L4 are glycine linkers, serine linkers or glycine-serine linkers, respectively. In one embodiment, L1, L2, L3 and L4 have the sequence (G₄S)ₙ (SEQ ID NO: 51), wherein n is an integer independently selected from 1 to 5, e.g., 1 to 3 or 2 to 4.

Also disclosed is (the use of) a functional (i.e., protease-cleavable) variant of the amino acid sequence of any one of SEQ ID NOs: 42 to 44 in any aspect of the present invention as described herein. The variant may comprise up to 3, 2 or 1 amino acid substitution(s), preferably conservative amino acid substitution(s), a deletion of up to 3, 2 or 1 amino acid residues (e.g., at the N-terminus and/or C-terminus) and/or an addition of up to 3, 2 or 1 amino acid residues (e.g., to the N-terminus and/or C-terminus) in/from/to the amino acid sequence of any one of SEQ ID NOs: 42 to 44. A conservative amino acid substitution involves substitution of an amino acid with another one of the same family of amino acids, i.e., amino acids which are related in their side chains (e.g., in terms of the electrical charge and/or size). Naturally occurring amino acids are generally divided into four families: acidic (aspartate, glutamate), basic (lysine, arginine, histidine), non-polar (alanine, valine, leucine, isoleucine, proline, phenylalanine, methionine, tryptophan), and uncharged polar (glycine, asparagine, glutamine, cysteine, serine, threonine, tyrosine) amino acids. Phenylalanine, tryptophan, and tyrosine are sometimes classified jointly as aromatic amino acids. Conservative substitutions are well known in the art (see for example Creighton (1984) Proteins. W.H. Freeman and Company). The variant may comprise one or more (e.g., up to 3, 2 or 1) of the following amino acid substitutions: R→K, S→T/A (i.e., S→T or S→A), V→A/I/L (i.e., V→A or V→I or V→L), D→E, E→D. The variant (e.g., a variant of SEQ ID NO: 42) may comprise one or more (e.g., up to 3, 2 or 1) of the following amino acid substitutions: S→T/A, V→A/I/L, D→E, E→D.

The present invention is now further described by reference to the following Examples, which are intended to illustrate, not to limit the scope of the present invention.

### Examples

### Example 1: Materials and Methods

### Expression of protease-cleavable monospecific and bispecific molecules in HEK 293-FS, ExpiCHO cells and stable CHO 9E4 cell pools

For monoclonal antibodies, the expression plasmids encoded a single chain comprising the heavy and light chain (anti-IL4-hlgG1) linked via protease-cleavable linkers; for bispecific Tandem-lgG antibodies, expression plasmids encoded either heavy chain or single-light chains (2 Fab fragments linked with protease-cleavable linkers); for bispecific Tandem-like antibodies, expression plasmids encoded either heavy chain or heavy-light chain linked constructs. For proteases, the expression plasmids encoded the human propeptide sequences of either Furin, PCSK5, PCSK6, PCSK7, or Furin with an additional KDEL-signal peptide. All expression plasmids were propagated in *E. coli* DH5a. Plasmids used for transfection were prepared from E. *coli* using the Qiagen EndoFree Plasmid Mega Kit.

HEK 293-FS cells growing in F17 serum free suspension culture (Invitrogen) were transfected with indicated single chain or LC and HC plasmids using polyethylenimine transfection reagent. ExpiCHO cells growing in F17 serum free suspension cultures (Invitrogen) were transfected with indicated plasmids using a suitable transfection reagent.

After 7 days of cultivation at 37°C, cells were removed by centrifugation and the supernatant was passed over a 0.22 µm filter to remove particles.

For co-transfections of HEK293-FS or ExpiCHO cells, plasmids for the antibody constructs were mixed in a 1:1 molar ratio with individual expression plasmids encoding the pro-peptide sequences of the proteases.

For the generation of stable CHO 9E4 cell pools, CHO 9E4 cells were cultured in CD CHO serum-free media (Invitrogen) and electroporated using the MaxCyte STX instrument and buffer reagents (MaxCyte Inc.) with transposon-based plasmids encoding single light chains and heavy chains, respectively. For protease co-expression, 30% of plasmid content were transposon-based protease-encoding plasmids. Stable cell pools were selected for 6 days in CD CHO media containing MSX selection marker. For expression of antibodies, cells were cultivated for 13 days at 37°C in OPTi CHO media (Invitrogen) containing FeedB (Invitrogen) under selection pressure, before supernatants were separated by centrifugation and passed over a 0.22 µm filter to remove particles.

For purification, the antibody was captured on a MabSelect SuRe column (Cat. No.: 11-0034-93, GE Healthcare) and eluted with 0.1 M Citrate buffer pH 3.0. Elution fractions were directly neutralized by adding 1:6 (v/v) Trizma pH 8.0 (Sigma Aldrich). After polishing the protein by size exclusion chromatography (SEC) using a Superdex200 16/60 (GE) and a final ultrafiltration concentration step, the protein was used for further characterization.

### Sodium dodecyl sulfate polyacrylamide gel electrophoresis (SDS-PAGE) and capillary electrophoresis

For SDS-PAGE, 2 µg protein sample was mixed with NuPage LDS Sample Buffer (ThermoFisher Scientific); for reducing conditions, 2 µg protein was mixed with NuPage LDS Sample Buffer containing DTT (Dithiothreitol) and heated for five minutes at 95°C. Samples and Color Protein Standard Broad Range ladder (New England Biolabs) were loaded on 4-12% Bis/Tris gels (Invitrogen) and run in NuPage MOPS SDS Running Buffer (ThermoFisher Scientific) at 200 V for 45 minutes. Gels were stained with Instant Blue Coomassie protein stain (Expedeon). For capillary electrophoreses, protein samples were prepared under reducing and oxidizing conditions according to manufacturer's protocol using Protein Clear HR Assay Kit and LabChip Touch SP2 instrument (Perkin Elmer).

### Analytical Size-exclusion chromatography (SEC)

Analytical SEC was performed using a BioSECcurity instrument (PSS Polymer) with a AdvanceBio 300 column (4.6 mm x 300 mm) and AdvanceBio 300 guard column (Agilent Technologies) at 25°C. The analysis was run at a flow rate of 0.5 ml/min using 2x concentrated D-PBS buffer (Thermo Fisher Scientific) with detection at 280 nm. 10 µl of protein sample (at 1 mg/ml) were applied onto the column. Data evaluation was performed using WinGPC software v8.1 (PSS Polymer). For estimation of the molecular weight, the SEC column was calibrated with a protein calibration standard mix (Agilent Technologies).

### Analytical Hydrophobic-interaction chromatography (HIC)

Analytical HIC was performed using a LC10 HPLC instrument (Shimadzu) or a Vanquish HPLC instrument (Thermo Fisher Scientific) equipped with a TSKgel Butyl-NPR column (2.5 µm, 4.6 x 35 mm) (Tosoh Bioscience) at 25°C. The analysis was run at a flow rate of 1 ml/min with detection at 280 nm. 5 µg of undiluted protein sample were applied onto the column. Gradient elution was from 15% B to 85% B in 7 min followed by 1 min to 100% B, then 1 min to 15% B and then 3 minutes equilibration at 15% B. Buffer A was composed of 1.5 M ammonium sulfate, 25 mM sodium phosphate pH 7.0. Buffer B was composed of 25 mM sodium phosphate pH 7.0. Data evaluation was performed using either LabSolutions software v5.85 (Shimadzu) or Chromeleon 7 software (Thermo Fisher Scientific).

### Mass spectrometry (MS)

Protein integrity and potential mispairing of heterodimeric constructs was analyzed by LC-mass spectrometry (LC-MS). Protein samples were deglycosylated with 12.5 µg of protein diluted to 0.17 mg/ml in LC-MS grade water (Thermo Scientific) treated with 0.5 µl PNGaseF (glycerol free, New England Biolabs) at 37°C for 16 hours. The LC-MS analysis was performed using a Thermo Fisher Orbitrap Lumos LC/MS instrument. Reversed phase (RP) chromatography was done using a MabPac RP HPLC column, analytical 4 µm particle size, 2.1 x 100mm (Thermo Scientific) at 300 µL/min. Eluents were LC water, 0.1% formic acid (A) and 90% acetonitrile, 10% LC water, 0.1% formic acid (B). 2 µg of protein were injected onto the column and eluted using a linear gradient from 0% to 95% B in 12 minutes. Data analysis was done using Expressionist software 13.0.3 (Genedata). Molecular masses were calculated based on the amino acid sequences of the proteins using GPMAW software version 10.32b1 (Lighthouse data).

### Surface plasmon resonance (SPR)

Binding of antigens to the antibody constructs was measured using surface plasmon resonance (SPR) with a BIAcore 3000 instrument (GE Healthcare) with HBS-EP buffer (GE Healthcare). As antigens, human IL4 (IL004, Millipore) and human IL13 (IL012, Millipore) were used. The antihuman Fc capture antibody (human antibody capture kit, GE Life Sciences) was immobilized via primary amine groups (11000 RU) on a research grade CM5 chip (GE Life Sciences) using standard procedures. The ligands were captured at a flow rate of 10 µl/min with an adjusted RU value that resulted in maximal analyte binding of 30 RU. The tested antibody constructs were used as analytes and injected at 100 nM concentration for 240 sec with a dissociation time of 300 sec at a flow rate of 30 µL/min. For human IL4 and IL13, a dilution series of 0.1 nM to 3 nM and 0.8 nM to 25 nM, respectively were used. Chip surfaces were regenerated with 2 min injects of the regeneration buffer provided with the capture kit. Sensorgrams were double-referenced with a blank chip surface and HBS-EP buffer blanks. Data analysis was performed using the BIAevaluation software v4.1.

### Example 2: Expression and in vivo processing of protease-cleavable Tandem-IgGs in HEK293-FS cells

Bispecific Tandem-lgG (Figure 1B; Table 1) constructs were expressed in HEK293-FS cells after transient transfection of two plasmids encoding the heavy chain (HC) and a single light chain (sLC) wherein the VLa-Cκ and VLb-Cκ chains were connected via different protease-cleavable linker sequences. As control, a Tandem-(anti-IL4xanti-IL13)-hulgG1 with VLa-Cκ and VLb-Cκ expressed as two separate chains (Tandem-IgG control) was used. The sLC Tandem-IgGs showed comparable expression and purification characteristics as the control Tandem-lgG (Figure 3A-B; selected constructs depicted in bold in Table 1).

**Table 1: Expression and purification results for Tandem-IgG constructs with protease-cleavable linkers from HEK293-FS cultures.**

| **SEQ ID NO** | **Construct Name** | **Monomeric Fraction after Protein A [%]** | **Yield after prep. SEC [mg/L]** |
|---|---|---|---|
| 11, 12, 13 | **Tandem-(anti-IL4xanti-IL13)-huIgG1** | 100.0 | 71.3 |
| 14, 13 | sLC-Tandem-(anti-IL4xanti-IL13)-singlePCL1-huIgG1 | 80.9 | 44.8 |
| 15, 13 | **sLC-Tandem-(anti-IL4xanti-IL13)-PCL1-huIgG1** | 83.1 | 34.8 |
| 16, 13 | sLC-Tandem-(anti-IL4xanti-IL13)-ΔPCL1-huIgG1 | 63.2 | 78.4 |
| 17, 13 | sLC-Tandem-(anti-IL4xanti-IL13)-His8-PCL1-huIgG1 | 91.0 | 33.1 |
| 18, 13 | sLC-Tandem-(anti-IL4xanti-IL13)-His8-ΔPCL1-huIgG1 | 83.1 | 28.3 |
| 19, 13 | **sLC-Tandem-(anti-IL4xanti-IL13)-PCL2-huIgG1** | 100.0 | 38.2 |
| 20, 13 | sLC-Tandem-(anti-IL4xanti-IL13)-PCL3-huIgG1 | 84.3 | 20.4 |
| 21, 22 | sLC-Tandem-(anti-lL4xanti-lL13)-rigidL1-PCL1-huIgG1 | 73.0 | 21.4 |
| 23, 13 | sLC-Tandem-(anti-IL4xanti-IL13)-rigidL2-PCL1-huIgG1 | 84.8 | 18.3 |
| 24, 13 | sLC-Tandem-(anti-IL4xanti-IL13)-charlL-PCL1-huIgG1 | 100.0 | 18.3 |
| 70, 71 | sLC-Tandem-(anti-PD-1)x(anti-4.1BB)-PCL2-huIgG1-LALA_ABO-2 | 100.0 | 7.5 |
| 72, 71 | sLC-Tandem-(anti-PD-1)x(anti-4.1BB)-PCL2a huIgG1-LALA_AB0-2 | 100.0 | 6.9 |
| 73, 74 | sLC-Tandem-(anti-PD-1)x(anti-OX40)-PCL2 huIgG1-LALA_AC0-2 | 100.0 | 3.7 |
| 75, 74 | sLC-Tandem-(anti-PD-1)x(anti-OX40)-PCL2a huIgG1-LALA_ACO-2 | 100.0 | 7.2 |

SDS-PAGE analysis under reducing conditions of *in vivo* processing of the Tandem-IgG constructs with protease-cleavable linkers revealed that the sLCs containing the linker sequences with the modified protease recognition/cleavage site HRRRKRSVDE (PCS2; SEQ ID NO: 43; "PCL2" refers to a PCL containing PCS2) showed two separate light chains (Figure 3C) as seen for the Tandem-lgG control. Similar results were obtained with the modified protease recognition/cleavage site HRRQQRSVDE (PCS2a; SEQ ID NO: 44; "PCL2a" refers to a PCL containing PCS2a). In contrast, all constructs containing the minimal protease recognition sequence (PCS1; SEQ ID NO: 49; "PCL1" refers to a PCL containing PCS1), different linker sequences or the deleted recognition sequence (deltaPCS/PCL) were not processed *in vivo* during expression in HEK293-FS cells and showed the same profile of a single LC and a HC under reducing conditions (Figure 3C; exemplary profile shown for sLC PCL1 Tandem-IgG).

Protein integrity and *in vivo* processing of Tandem-IgG constructs was further analyzed by LC-mass spectrometry (LC-MS). Samples were measured after deglycosylation under reducing and non-reducing conditions. Molecular masses were calculated based on the amino acid sequences of the proteins (Table 2). LC-MS analysis confirmed complete *in vivo* processing of linker sequences in Tandem-lgG constructs containing PCS2 recognition sites. In contrast, no processing was detected in protein samples of Tandem-IgGs with deleted (deltaPCS/PCL) or minimal PCS (PCS1) recognition sites. Furthermore, control Tandem-IgG consisting of two separate LCs showed mispairing of LCs to HCs (3*LC1 + 1*LC2 + 2*HC), which was not observed in sLC Tandem-IgG-PCL2 after processing of the sLC into LC1 and LC2. Thus, sLC Tandem-lgG constructs reduce/avoid LC-HC mispairing in this bispecific IgG-format. Moreover, in fully processed samples, further processing of the C-terminal charged amino acids comprising the protease recognition site RRKR by carboxy-terminal proteases was observed (RRKR-loss). In contrast, no further N-terminal processing could be seen by Edman-Sequencing (data not shown).

**Table 2: LC-MS results of HEK293-FS expressed Tandem-IgG under deglycosylated reducing or non-reducing conditions.**

| **SE Q ID NO** | **Construct Name** | **Expecte d (Da) *** | **Measure d (Da)** | **Differen ce (Da)** | **Comment** | **Pairing** | **Processi ng** |
|---|---|---|---|---|---|---|---|
| 11, 12, 13 | Tandem-(anti-IL4xanti-IL13)-huIgG1 | 243319. 08 | 243343. 67 | 24.59 | correct | 2*LC1 | ctrl. |
| | | | | | | 2*LC2 | |
| | | | | | | 2*HC | |
| | | | 242905. 05 | -414.03 | minor peak - mispairing | 3*LC1 | |
| | | | | | | 1*LC2 | |
| | | | | | | 2*HC | |
| | | | 243203. 21 | -115.87 | minor preak 2*Gly-loss | 2*LC1 | |
| | | | | | | 1*LC2 | |
| | | | | | | 2*HC | |
| 15, 13 | sLC-Tandem-(anti-IL4xanti-IL13)-PCL1-huIgG1 | 49185.1 1 | 49180.3 4 | -4.77 | correct | sLC | no |
| | | 74621.9 2 | 74621.9 2 | 6.18 | correct | HC | |
| 16, 13 | sLC-Tandem-(anti-IL4xanti-IL13)-ΔPCL1-huIgG1 | 49128.9 1 | 49124.1 0 | -4.81 | correct | sLC | no |
| | | 74621.9 2 | 74617.4 8 | -4.44 | correct | HC | |
| 19, 13 | sLC-Tandem-(anti-IL4xanti-IL13)-PCL2-huIgG1 | 24176.4 6 | 24173.9 4 | -2.52 | correct | LC1 | yes |
| | | 24207.8 3 | 23608.7 1 | -599.12 | correct but RRKR-loss | LC2 | |
| | | 24207.8 3 | 23452.2 4 | -755.59 | correct but RRKR-loss | LC2 | |
| | | 74621.9 2 | 746625. 48 | 3.56 | correct | HC | |
| 72, 71 | sLC-Tandem-(anti-PD-1)x(anti-4.1BB)-PCL2a huIgG1-LALA_AB 0-2 | 244161. 57 | 244169. 00 | 30 | correct | 2*LC1 | yes |
| | | | | | | 2*LC2 | |
| | | | | | | HC | |
| | | 244161. 57 | 244007. 00 | -154.57 | correct but RR-loos on 1LC1 | 2*LC1 | |
| | | | | | | 2*LC2 | |
| | | | | | | HC | |
| | | 244161. 57 | 243850. 00 | -1276 | correct but R-loss on 2LC1 | 2*LC1 | |
| | | | | | | 2*LC2 | |
| | | | | | | HC | |
| 75, 74 | sLC-Tandem- | 243246. 66 | 242933. 00 | -3 | correct | 2*LC1 | yes |
| | | | | | | 2*LC2 | |
| | (anti-PD-1)x(anti-OX40)-PCL2a huIgG1-LALA_AC 0-2 | | | | | HC | |
| | | 243246. 66 | 243092. 00 | -636 | correct but RR-loos on 1LC1 | 2*LC1 | |
| | | | | | | 2*LC2 | |
| | | | | | | HC | |
| | | 243246. 66 | 243246. 00 | -1289 | correct but R-loss on 2LC1 | 2*LC1 | |
| | | | | | | 2*LC2 | |
| | | | | | | HC | |

### Example 3: Expression and in vivo processing of protease-cleavable Tandem-IgGs in ExpiCHO cells

For transient expression of Tandem-IgGs in ExpiCHO cells, selected sLC constructs are shown in Table 3. The sLC Tandem-IgGs with different protease recognition sites showed comparable expression and purification characteristics as the control Tandem-lgG (Figure 4A-B). However, expression levels were lower compared to transient transfection and expression in HEK293-FS cells.

**Table 3: Expression and purification results Tandem-IgG constructs with protease-cleavable linkers from ExpiCHO cultures.**

| **SEQ ID NO** | **Construct Name** | **Monomeric Fraction after Protein A [%]** | **Yield after prep. SEC [mg/L]** |
|---|---|---|---|
| 15, 13 | sLC-Tandem-(anti-IL4xanti-IL13)-PCL1-huIgG1 | 80.1 | 1.4 |
| 16, 13 | sLC-Tandem-(anti-IL4xanti-IL13)-ΔPCL1-huIgG1 | 90.3 | 3.4 |
| 19, 13 | sLC-Tandem-(anti-IL4xanti-IL13)-PCL2-huIgG1 | 95.4 | 4.1 |

SDS-PAGE analysis under reducing conditions of *in vivo* processing of the Tandem-lgG revealed, that the sLCs containing the linker sequences with the modified protease recognition site HRRRKRSVDE (PCS2; SEQ ID NO: 43) showed two separate light chains (Figure 4C). Constructs containing the minimal protease recognition sequence (PCS1; SEQ ID NO: 49) or the deleted recognition sequence (deltaPCS/PCL) were not processed *in vivo* by endogenously expressed proteases in ExpiCHO cells. Thus, expression of protease-cleavable sLC Tandem-IgGs in ExpiCHO cells confirmed the results from HEK293-FS cultures.

LC-MS analysis confirmed SDS-PAGE results of fully processed sLC-PCL2 Tandem-lgG in ExpiCHO cells with further processing profile of C-terminal charged amino acids by carboxy-terminal proteases (RRKR-loss).

**Table 4: LC-MS results of ExpiCHO expressed Tandem-IgG under deglycosylated reducing conditions.**

| **SEQ ID NO** | **Construct Name** | **Expected (Da) *** | **Measured (Da)** | **Difference (Da)** | **Comment** | **Pairing** | **Processing** |
|---|---|---|---|---|---|---|---|
| 15, 13 | sLC-Tandem-(anti-IL4xanti-IL13)-PCL1-huIgG1 | 49185.11 | 49179.68 | -5.43 | correct | sLC | no |
| | | 74621.92 | 74620.47 | -1.45 | correct | HC | |
| 16, 13 | sLC-Tandem-(anti-IL4xanti-IL13)-ΔPCL1-huIgG1 | 49128.91 | 49123.63 | -5.28 | correct | sLC | no |
| | | 74621.92 | 74620.93 | -0.99 | correct | HC | |
| 19, 13 | sLC-Tandem-(anti-IL4xanti-IL13)-PCL2-huIgG1 | 24176.46 | 24174.19 | -2.27 | correct | LC1 | yes |
| | | 24207.83 | 24212.20 | 4.37 | correct | LC2 | |
| | | 24207.83 | 24049.16 | -158.67 | correct but R-loss | LC2 | |
| | | 24207.83 | 23764.18 | -443.65 | correct but RKR-loss | LC2 | |
| | | 24207.83 | 23608.55 | -599.28 | correct but RRKR-loss | LC2 | |
| | | 24207.83 | 23452.18 | -755.65 | correct but RRRKR-loss | LC2 | |
| | | 74621.92 | 74622.13 | 0.25 | correct | HC | |

### Example 4: Co-Transfection of proteases and protease-cleavable constructs in HEK293-FS cells

Tandem-IgG constructs with sLC containing PCS1 and deltaPCS recognition sites in the linker sequence of the sLC were co-transfected with proteases of the PCSK family in HEK293-FS cells (Table 5) to induce *in vivo* processing by overexpression of the proteases. The sLC Tandem-lgG constructs showed comparable expression and purification characteristics as the single transfected antibodies (Figure 5A and B). Reduced expression yields were due to a lower plasmid amount in the co-transfection (1:1 molar ratio with plasmids encoding individual proteases). Analysis of *in vivo* processing in SDS-PAGE and LC-MS confirmed processing of sLC Tandem-IgGs containing PCS1 recognition sites with recombinantly expressed human Furin and human Furin with a KDEL-retention sequence (Figure 5C and D). In contrast, overexpression of other protease family members did not lead to sLC Tandem-lgG processing. Comparable results were obtained for sC-lgG and Tandem-like IgG constructs (Figure 7A and B).

**Table 5: Expression and purification results Tandem-IgG constructs with protease-cleavable linkers from HEK293-FS cultures after co-expression with proteases of the PCSK family.**

| **SEQ ID NO** | **Construct Name** | **Yield after prep. SEC [mg/L]** |
|---|---|---|
| 17, 13 with 37 | sLC-Tandem-(anti-IL4xanti-IL13)-His8-PCL1-huIgG1 | 12.5 |
| 17, 13 with 38 | sLC-Tandem-(anti-IL4xanti-IL13)-His8-PCL1-huIgG1 | 12.5 |
| 17, 13 with 39 | sLC-Tandem-(anti-IL4xanti-IL13)-His8-PCL1-huIgG1 | 7.8 |
| 17, 13 with 40 | sLC-Tandem-(anti-IL4xanti-IL13)-His8-PCL1-huIgG1 | 3.8 |
| 17, 13 with 41 | sLC-Tandem-(anti-IL4xanti-IL13)-His8-PCL1-huIgG1 | 9.0 |
| 18, 13 with 37 | sLC-Tandem-(anti-IL4xanti-IL13)-His8-ΔPCL1-huIgG1 | 5.8 |
| 18, 13 with 38 | sLC-Tandem-(anti-IL4xanti-IL13)-His8-ΔPCL1-huIgG1 | 8.8 |
| 18, 13 with 39 | sLC-Tandem-(anti-IL4xanti-IL13)-His8-ΔPCL1-huIgG1 | 5.3 |
| 18, 13 with 40 | sLC-Tandem-(anti-IL4xanti-IL13)-His8-ΔPCL1-huIgG1 | 3.8 |
| 18, 13 with 41 | sLC-Tandem-(anti-IL4xanti-IL13)-His8-ΔPCL1-huIgG1 | 7.5 |

For generation of stable CHO cell pools, transposon-based plasmids encoding Tandem-lgG constructs with sLC containing PCS1, PCS2 and deltaPCS recognition sites in the linker sequence of the sLC, as well as the Tandem-lgG control (2 LC plasmids) were used for electroporation of CHO 9E4 cells with and without protease co-expression. The sLC Tandem-lgG constructs showed comparable expression and purification characteristics as the Tandem-IgG control. Analysis of *in vivo* processing in SDS-PAGE confirmed processing of sLC Tandem-IgGs containing PCS2 recognition sites in the samples expressed without protease co-expression and processed sLC for Tandem-IgGs with PCS1 and PCS2 recognition sites with recombinantly expressed human Furin (Figure 6).

### Example 5: Evaluation of antigen binding of bispecific sLC Tandem-IgGs with protease-cleavable linkers

The binding of the different bispecific Tandem-lgG constructs expressed in HEK293-FS or ExpiCHO cells as well as *in vivo* processed Tandem-IgG from the co-transfection with Furin variants was measured via surface plasmon resonance (SPR). The binding to the two human antigens IL4 and IL13 were compared to the binding characteristics of the individual monoclonal antibodies. The binding characteristics of the monoclonal anti-IL4 and anti-IL13 mAbs and bispecific Tandem-IgGs are shown in Table 6. Specific binding to IL4 was measured for all Tandem-IgGs and was comparable to the specific binding of anti-IL4 control antibody. In contrast, specific binding to IL13 was only detectable for *in vivo* processed Tandem-IgGs as well as the Tandem-lgG control comprising two LCs. Unprocessed sLC Tandem-IgGs showed no binding. Thus, processing of the linker sequence connecting the VLb (VL against IL13) at the inner position of the Tandem-lgG with the Cκ domain of the IL4 binding Fab at the outer position is required for a functional IL13 binding site. *In vivo* processing can thereby be mediated via endogenously expressed proteases or via co-transfected Furin variants.

**Table 6: Binding kinetics by SPR: Comparison of anti-IL4 and anti-IL13 mAbs with bispecific Tandem-IgGs**

| | | **Antigen IL4** | | | **Antigen IL13** | | |
|---|---|---|---|---|---|---|---|
| **SEQ ID NO** | **Construct Name** | **KD [M]** | **ka [1/Ms]** | **kd [1/s]** | **KD [M]** | **ka [1/Ms]** | **kd [1/s]** |
| **HEK293-FS expression** | | | | | | | |
| | anti-IL4 mAb ctrl | 8.87E-13 | 7.90E+08 | 7.01E-04 | - | - | - |
| | anti-IL13 mAb ctrl | - | | | 8.88E-11 | 5.08E+05 | 4.52E-05 |
| 11, 12, 13 | Tandem-(anti-IL4)xanti-IL13)-huIgG1 | 2.32E-12 | 6.39E+07 | 1.49E-04 | 1.14E-10 | 2.25E+05 | 2.56E-05 |
| 15, 13 | sLC-Tandem-(anti-IL4)xanti-IL13)-PCL1-huIgG1 | 9.01E-13 | 4.20E+09 | 3.79E-04 | - | - | - |
| 16, 13 | sLC-Tandem-(anti-IL4xanti-IL13)-ΔPCL1-huIgG1 | 2.29E-12 | 6.75E+07 | 1.55E-04 | - | - | - |
| 19, 13 | sLC-Tandem-(anti-IL4xanti-IL13)-PCL2-huIgG1 | 1.05E-12 | 1.79E+08 | 1.89E-04 | 1.81E-10 | 2.00E+05 | 3.62E-05 |
| 17, 13 with 37 | sLC-Tandem-(anti-IL4xanti-IL13)-His8-PCL1-huIgG1 | 1.53E-12 | 1.38E+08 | 2.11E-04 | 8.70E-11 | 1.79E+05 | 1.56E-05 |
| 17, 13 with 38 | sLC-Tandem-(anti-IL4xanti-IL13)-His8-PCL1-huIgG1 | 1.86E-12 | 1.61E+08 | 2.99E-04 | 1.49E-10 | 1.89E+05 | 2.82E-05 |

| **ExpiCHO expression** | | | | | | | |
|---|---|---|---|---|---|---|---|
| 15, 13 | sLC-Tandem-(anti-IL4xanti-IL13)-PCL1-huIgG1 | 1.42E-12 | 1.13E+08 | 1.61E-04 | - | - | - |
| 16, 13 | sLC-Tandem-(anti-IL4xanti-IL13)-ΔPCL1-huIgG1 | 2.75E-12 | 5.43E+07 | 1.49E-04 | - | - | - |
| 19, 13 | sLC-Tandem-(anti-IL4xanti-IL13)-PCL2-huIgG1 | 1.33E-12 | 1.87E+08 | 2.49E-04 | 6.33E-12 | 1.27E+05 | 8.05E-07 |

### Example 6: Expression and purification of sC-IgGs and Tandem-like IgGs with protease-cleavable linkers in HEK293-FS and ExpiCHO cells

Table 6 and Table 7 show expression and purification data of sC-lgG and Tandem-like IgG constructs with protease-cleavable linker sequence from HEK293-FS and ExpiCHO cultures after transient transfection, respectively.

After Protein A purification and preparative SEC, sC-IgGs showed high purity with 83-99% monomeric peak fractions. Protein yield dropped for sC-lgGs with decreasing length of the (G₄S)ₙ (SEQ ID NO: 51) linker sequences.

In contrast to sC-lgGs, the Tandem-like IgG format showed only low protein purity (23-75% monomeric peak fraction) with high aggregation potential as seen in high molecular weight species in preparative SEC.

**Table 7: Expression and purification results of sc-IgGs and Tandem-like IgG constructs with protease-cleavable linkers from HEK293-FS cultures.**

| **SEQ ID NO** | **Construct Name** | **Yield after Protein A [mg/L]** | **Monomeric Fraction after Protein A [%]** |
|---|---|---|---|
| 1 | sc-anti-IL4-(G4S)10-PCL1-hulgG1 | 32.3 | 98.4 |
| 2 | sc-anti-IL4-(G4S)8-PCLl-huIgG1 | 13.8 | 99.1 |
| 3 | sc-anti-IL4-(G4S)6-PCLl-huIgG1 | 8.3 | 92.3 |
| 4 | sc-anti-IL4-(G4S)4-PCL1-hulgG1 | 9.4 | 93.2 |
| 5 | sc-anti-IL4-(G4S)2-PCL1-hulgG1 | 1.6 | 88.5 |
| 6 | sc-anti-IL4-ΔPCL1-hulgG1 | 94.3 | 83.8 |
| 7 | sc-anti-IL4-His8-PCLl-huIgG1 | 8.1 | 87.1 |
| 8 | sc-anti-IL4-His8-ΔPCL1-hulgG1 | 43.9 | 90.4 |
| 9 | sc-anti-IL4-His8-PCL2-hulgG1 | 61.8 | 98.7 |
| 10 | sc-anti-IL4-His8-PCL3-hulgG1 | 22.8 | 98.2 |
| 25, 26 | scTandem-(anti-IL4xanti-IL13)-PCL1-hulgG1-OL-KIH | 36.7 | 39.6 |
| 27, 26 | sctandem-(anti-IL4xanti-IL13)-singlePCLl-huIgG1-OL-KIH | 19.2 | 33.9 |
| 28, 26 | scTandem-(anti-IL4xanti-IL13)-ΔPCL1-hulgGl-OL-KIH | 15.6 | 23.6 |
| 29, 26 | scTandem-(anti-IL4xanti-1L13)-His8-PCLl-huIgG1-OL-KIH | 5.9 | 73.3 |
| 30, 26 | scTandem-(anti-IL4xanti-1L13)-His8-ðPCLl-huIgG1-OL-KIH | 6.3 | 69.3 |
| 31, 26 | scTandem-(anti-IL4xanti-IL13)-PCL2-hulgG1-OL-KIH | 17.6 | 54 |
| 32, 26 | scTandem-(anti-IL4xanti-IL13)-PCL3-hulgG1-OL-KIH | 13.0 | 61.9 |
| 33, 34 | scTandem-(anti-IL4xanti-1L13)-rigidL-PCL1-hulgG1-OL-KIH | 20.2 | 29 |
| 35, 26 | scTandem-(anti-IL4xanti-IL13)-rigidL2-PCL1-hulgG1-OL-KIH | 25.0 | 60 |
| 36, 26 | scTandem-(anti-IL4xanti-1L13)-charL-PCL1-hulgG1-OL-KIH | 6.3 | 75.1 |

**Table 8: Expression and purification results of sc-IgGs and Tandem-like IgG constructs with protease-cleavable linkers from ExpiCHO cultures.**

| **SEQ ID NO** | **Construct name** | **Monomeric Fraction after Protein A [%]** | **Yield after prep. SEC [mg/L]** |
|---|---|---|---|
| 3 | sc-anti-IL4-(G4S)6-PCLl-huIgG1 | 94.6 | 1.1 |
| 6 | sc-anti-IL4-ΔPCL1-hulgG1 | 96.3 | 1.6 |
| 9 | sc-anti-IL4-His8-PCL2-hulgG1 | 100.0 | 3.7 |
| 25, 26 | scTandem-(anti-IL4xanti-IL13)-PCL1-hulgG1-OL-KIH | | 1.2 |
| 28, 26 | scTandem-(anti-IL4xanti-IL13)-APCL1-hulgG1-OL-KIH | | 2.1 |
| 31, 26 | scTandem-(anti-IL4xanti-IL13)-PCL2-hulgG1-OL-KIH | | 3.5 |

In both cell lines, only sC-IgGs and Tandem-like IgGs with PCS2 recognition sites were cleaved into the respective chain fragments by endogenous proteases during protein expression (Figure 7A and B) as shown for the sLC Tandem-lgG format.

### Example 7: Co-Transfection of proteases and protease-cleavable constructs in HEK293-FS cells

Single chain Ig and Tandem-like IgG constructs with sLC containing PCS1 and deltaPCS recognition sites in the linker sequence were co-transfected with proteases of the PCSK family in HEK293-FS cells to induce *in vivo* processing by overexpression of the proteases. Analysis of *in vivo* processing in SDS-PAGE confirmed induced processing of sC-lgG and Tandem-like IgG containing PCS1 recognition sites with recombinantly expressed human Furin and human Furin with a KDEL-retention sequence (Figure 8A and B). Furthermore, overexpression of PACE4 lead to partially processed constructs.

### Example 8: Expression and in vivo processing of protease-cleavable CODV-scFab-IgG in HEK293-FS cells

Trispecific CODV-scFab-IgG (Figure 2C; Table 8) constructs were expressed in HEK293-FS cells after transient transfection of three plasmids encoding the CODV HC, CODV LC and a single chain (sC) wherein the VLc-Cκ and VHc-CH1-CH2-CH3 chains were connected via different protease-cleavable linker sequences. As control, a trispecific CODV-Fab-IgG with Fab-LC and HC expressed as two separate chains (classical CODV-Fab-IgG control) was used. The single chain Fab CODVs showed comparable expression and purification characteristics as the control, with reduction of aggregate peaks in SEC (Table 9, Figure 9A).

**Table 9: Expression and purification results of trispecific CODV-Fab-IgG constructs with protease-cleavable linkers from HEK293-FS cultures.**

| **SEQ ID NO** | **Construct Name** | **Monomeric Fraction after Protein A [%]** | **Yield after prep. SEC [mg/L]** |
|---|---|---|---|
| 56, 57, 58, 59 | TRI-(CODV- anti-PD-1 x anti-OX40) x anti-GITR-hulgG1-LALA-KIH-RF | 56.3 | 40.3 |
| 56, 57, 60 | TRI-(CODV- anti-PD-1 x anti-OX40) x sc-PCL2-anti-GITR-hulgG1-LALA-KIH-RF | 73.6 | 24.5 |
| 56, 57, 61 | TRI-(CODV) x sc-PCL2a-anti-GITR-hulgG1-LALA-KIH-RF | 75.8 | 19.0 |
| 62, 63, 64 | TRI-(CODV- anti-GITR x anti-PD-1) x sc-PCL2- anti-OX40-hulgG1-LALA-KIH-RF | 89.4 | 30.7 |
| 62, 63, 65 | TRI-(CODV- anti-GITR x anti-PD-1)x sc-PCL2a- anti-OX40-hulgG1-LALA-KIH-RF | 92.3 | 28.0 |
| 66, 67, 68 | TRI-(CODV- anti-PD-1 x anti-GITR) x sc-PCL2- anti-OX40-hulgG1-LALA-KIH-RF | 88.1 | 33.7 |
| 66, 67, 69 | TRI-(CODV- anti-PD-1 x anti-GITR) x sc-PCL2a- anti-OX40-hulgG1-LALA-KIH-RF | 93.7 | 28.2 |

Electrophoretic analysis under reducing conditions showed that the single chains of the Fab arm containing either PCS2 (SEQ ID NO: 43) or PCS2a (SEQ ID NO: 44) sequences were processed after expression in HEK293-FS cells leading to Fab-LC and Fab-HC parts (Figure 9B).

**Table 10: List of Sequences/Constructs.**

| **Name** | **SEQ ID NO** | **Amino acid sequence** | **Annotation / Comments** |
|---|---|---|---|
| sc-anti-IL4-(G4S)10-PCL1-huIgG1 | 1 | DIQMTQSPASLSVSVGDTITLTCHASQNIDVWLSWFQQK PGNIPKLLIYKASNLHTGVPSRFSGSGSGTGFTLTISSLQPED IATYYCQQAHSYPFTFGGGTKLEIKRTVAAPSVFIFPPSDEQ LKSGTASVVCLLNNFYPREAKVQWKVDNALQSGNSQESVT EQDSKDSTYSLSSTLTLSKADYEKHKVYACEVTHQGLSSPVT KSFNRGECRRKRGGGGSGGGGSGGGGSGGGGSGGGGSG GGGSGGGGSGGGGSGGGGSGGGGSRRKRQVQLQQSGP ELVKPGASVKISCKASGYSFTSYWIHWIKQRPGQGLEWIG MIDPSDGETRLNQRFQGRATLTVDESTSTAYMQLRSPTSE DSAVYYCTRLKEYGNYDSFYFDVWGAGTLVTVSSASTKGP SVFPLAPSSKSTSGGTAALGCLVKDYFPEPVTVSWNSGALTS GVHTFPAVLQSSGLYSLSSVVTVPSSSLGTQTYICNVNHKPS NTKVDKKVEPKSCDKTHTCPPCPAPELLGGPSVFLFPPKPKD TLMISRTPEVTCVVVDVSHEDPEVKFNWYVDGVEVHNAKT KPREEQYNSTYRVVSVLTVLHQDWLNGKEYKCKVSNKALP APIEKTISKAKGQPREPQVYTLPPSRDELTKNQVSLTCLVKGF YPSDIAVEWESNGQPENNYKTTPPVLDSDGSFFLYSKLTVD KSRWQQGNVFSCSVMHEALHNHYTQKSLSLSPG | **anti-IL4-VL-**huIGKC-*[PCS1]*-<(G4S)10>-*[PCS1]***-anti-IL4-VH-**hulGHG1 |
| sc-anti-IL4-(G4S)8-PCL1-huIgG1 | 2 | DIQMTQSPASLSVSVGDTITLTCHASQNIDVWLSWFQQK PGNIPKLLIYKASNLHTGVPSRFSGSGSGTGFTLTISSLQPED IATYYCQQAHSYPFTFGGGTKLEIKRTVAAPSVFIFPPSDEQ LKSGTASVVCLLNNFYPREAKVQWKVDNALQSGNSQESVT EQDSKDSTYSLSSTLTLSKADYEKHKVYACEVTHQGLSSPVT KSFNRGECRRKRGGGGSGGGGSGGGGSGGGGSGGGGSG GGGSGGGGSGGGGSRRKRQVQLQQSGPELVKPGASVKIS CKASGYSFTSYWIHWIKQRPGQGLEWIGMIDPSDGETRL NQRFQGRATLTVDESTSTAYMQLRSPTSEDSAVYYCTRLK EYGNYDSFYFDVWGAGTLVTVSSASTKGPSVFPLAPSSKST SGGTAALGCLVKDYFPEPVTVSWNSGALTSGVHTFPAVLQS SGLYSLSSVVTVPSSSLGTQTYICNVNHKPSNTKVDKKVEPK SCDKTHTCPPCPAPELLGGPSVFLFPPKPKDTLMISRTPEVTC VVVDVSHEDPEVKFNWYVDGVEVHNAKTKPREEQYNSTY RVVSVLTVLHQDWLNGKEYKCKVSNKALPAPIEKTISKAKG QPREPQVYTLPPSRDELTKNQVSLTCLVKGFYPSDIAVEWES NGQPENNYKTTPPVLDSDGSFFLYSKLTVDKSRWQQGNVF SCSVMHEALHNHYTQKSLSLSPG | **anti-IL4-VL**-huIGKC-*[PCS1]-*<(G4S)8>-*[PCS1]***-anti-IL4-VH-**huIGHG1 |
| sc-anti-IL4-(G4S)6-PCL1-huIgG1 | 3 | DIQMTQSPASLSVSVGDTITLTCHASQNIDVWLSWFQQK PGNIPKLLIYKASNLHTGVPSRFSGSGSGTGFTLTISSLQPED IATYYCQQAHSYPFTFGGGTKLEIKRTVAAPSVFIFPPSDEQ LKSGTASVVCLLNNFYPREAKVQWKVDNALQSGNSQESVT EQDSKDSTYSLSSTLTLSKADYEKHKVYACEVTHQGLSSPVT KSFNRGECRRKRGGGGSGGGGSGGGGSGGGGSGGGGSG GGGSRRKRQVQLQQSGPELVKPGASVKISCKASGYSFTSY WIHWIKQRPGQGLEWIGMIDPSDGETRLNQRFQGRATL TVDESTSTAYMQLRSPTSEDSAVYYCTRLKEYGNYDSFYFD VWGAGTLVTVSSASTKGPSVFPLAPSSKSTSGGTAALGCLV KDYFPEPVTVSWNSGALTSGVHTFPAVLQSSGLYSLSSVVT VPSSSLGTQTYICNVNHKPSNTKVDKKVEPKSCDKTHTCPPC PAPELLGGPSVFLFPPKPKDTLMISRTPEVTCVVVDVSHEDP EVKFNWYVDGVEVHNAKTKPREEQYNSTYRVVSVLTVLHQ DWLNGKEYKCKVSNKALPAPIEKTISKAKGQPREPQVYTLPP SRDELTKNQVSLTCLVKGFYPSDIAVEWESNGQPENNYKTT PPVLDSDGSFFLYSKLTVDKSRWQQGNVFSCSVMHEALHN HYTQKSLSLSPG | **anti-IL4-VL-**huIGKC-*[PCS1]-*<(G4S)6>-*[PCS1]*-**anti-IL4-VH-**huIGHG1 |
| sc-anti-IL4-(G4S)4-PCL1-huIgG1 | 4 | DIQMTQSPASLSVSVGDTITLTCHASQNIDVWLSWFQQK PGNIPKLLIYKASNLHTGVPSRFSGSGSGTGFTLTISSLQPED IATYYCQQAHSYPFTFGGGTKLEIKRTVAAPSVFIFPPSDEQ LKSGTASVVCLLNNFYPREAKVQWKVDNALQSGNSQESVT EQDSKDSTYSLSSTLTLSKADYEKHKVYACEVTHQGLSSPVT KSFNRGECRRKRGGGGSGGGGSGGGGSGGGGSRRKRQV QLQQSGPELVKPGASVKISCKASGYSFTSYWIHWIKQRPG QGLEWIGMIDPSDGETRLNQRFQGRATLTVDESTSTAYM QLRSPTSEDSAVYYCTRLKEYGNYDSFYFDVWGAGTLVTV SSASTKGPSVFPLAPSSKSTSGGTAALGCLVKDYFPEPVTVS WNSGALTSGVHTFPAVLQSSGLYSLSSVVTVPSSSLGTQTYI CNVNHKPSNTKVDKKVEPKSCDKTHTCPPCPAPELLGGPSV FLFPPKPKDTLMISRTPEVTCVVVDVSHEDPEVKFNWYVDG VEVHNAKTKPREEQYNSTYRVVSVLTVLHQDWLNGKEYKC KVSNKALPAPIEKTISKAKGQPREPQVYTLPPSRDELTKNQV SLTCLVKGFYPSDIAVEWESNGQPENNYKTTPPVLDSDGSF FLYSKLTVDKSRWQQGNVFSCSVMHEALHNHYTQKSLSLS PG | **anti-IL4-VL-**huIGKC-*[PCS1]-*<(G4S)4>-*[PCS1]*-**anti-IL4-VH-**huIGHG1 |
| sc-anti-IL4-(G4S)2-PCL1-huIgG1 | 5 | DIQMTQSPASLSVSVGDTITLTCHASQNIDVWLSWFQQK PGNIPKLLIYKASNLHTGVPSRFSGSGSGTGFTLTISSLQPED IATYYCQQAHSYPFTFGGGTKLEIKRTVAAPSVFIFPPSDEQ LKSGTASVVCLLNNFYPREAKVQWKVDNALQSGNSQESVT EQDSKDSTYSLSSTLTLSKADYEKHKVYACEVTHQGLSSPVT KSFNRGECRRKRGGGGSGGGGSRRKRQVQLQQSGPELVK PGASVKISCKASGYSFTSYWIHWIKQRPGQGLEWIGMIDP SDGETRLNQRFQGRATLTVDESTSTAYMQLRSPTSEDSAV YYCTRLKEYGNYDSFYFDVWGAGTLVTVSSASTKGPSVFPL APSSKSTSGGTAALGCLVKDYFPEPVTVSWNSGALTSGVHT FPAVLQSSGLYSLSSVVTVPSSSLGTQTYICNVNHKPSNTKV DKKVEPKSCDKTHTCPPCPAPELLGGPSVFLFPPKPKDTLMI SRTPEVTCVVVDVSHEDPEVKFNWYVDGVEVHNAKTKPRE EQYNSTYRVVSVLTVLHQDWLNGKEYKCKVSNKALPAPIEK TISKAKGQPREPQVYTLPPSRDELTKNQVSLTCLVKGFYPSDI AVEWESNGQPENNYKTTPPVLDSDGSFFLYSKLTVDKSRW QQGNVFSCSVMHEALHNHYTQKSLSLSPG | **anti-IL4-VL-**huIGKC-*[PCS1]-*<(G4S)2>-*[PCS1]*-**anti-IL4-VH-**huIGHG1 |
| sc-anti-IL4-ΔPCL1-huIgG1 | 6 | DIQMTQSPASLSVSVGDTITLTCHASQNIDVWLSWFQQK PGNIPKLLIYKASNLHTGVPSRFSGSGSGTGFTLTISSLQPED IATYYCQQAHSYPFTFGGGTKLEIKRTVAAPSVFIFPPSDEQ LKSGTASVVCLLNNFYPREAKVQWKVDNALQSGNSQESVT EQDSKDSTYSLSSTLTLSKADYEKHKVYACEVTHQGLSSPVT KSFNRGECRQQRGGGGSGGGGSGGGGSGGGGSGGGGS GGGGSGGGGSGGGGSGGGGSGGGGSRQQRQVQLQQSG PELVKPGASVKISCKASGYSFTSYWIHWIKQRPGQGLEWI GMIDPSDGETRLNQRFQGRATLTVDESTSTAYMQLRSPTS EDSAVYYCTRLKEYGNYDSFYFDVWGAGTLVTVSSASTKG PSVFPLAPSSKSTSGGTAALGCLVKDYFPEPVTVSWNSGALT SGVHTFPAVLQSSGLYSLSSVVTVPSSSLGTQTYICNVNHKP SNTKVDKKVEPKSCDKTHTCPPCPAPELLGGPSVFLFPPKPK DTLMISRTPEVTCVVVDVSHEDPEVKFNWYVDGVEVHNAK TKPREEQYNSTYRVVSVLTVLHQDWLNGKEYKCKVSNKALP APIEKTISKAKGQPREPQVYTLPPSRDELTKNQVSLTCLVKGF YPSDIAVEWESNGQPENNYKTTPPVLDSDGSFFLYSKLTVD KSRWQQGNVFSCSVMHEALHNHYTQKSLSLSPG | **anti-IL4-VL-**huIGKC-*[deltaPCS]-*<(G4S)10>-*[deltaPCS]*-**anti-IL4-VH**-huIGHG1 |
| sc-anti-IL4-His8-PCL1-huIgG1 | 7 | DIQMTQSPASLSVSVGDTITLTCHASQNIDVWLSWFQQK PGNIPKLLIYKASNLHTGVPSRFSGSGSGTGFTLTISSLQPED IATYYCQQAHSYPFTFGGGTKLEIKRTVAAPSVFIFPPSDEQ LKSGTASVVCLLNNFYPREAKVQWKVDNALQSGNSQESV TEQDSKDSTYSLSSTLTLSKADYEKHKVYACEVTHQGLSSP VTKSFNRGECRRKRGGGGSGGGGSGGGGSGGGGSHHHH HHHHGGGGSGGGGSGGGGSGGGGSRRKRQVQLQQSGP ELVKPGASVKISCKASGYSFTSYWIHWIKQRPGQGLEWIG MIDPSDGETRLNQRFQGRATLTVDESTSTAYMQLRSPTSE DSAVYYCTRLKEYGNYDSFYFDVWGAGTLVTVSSASTKGP SVFPLAPSSKSTSGGTAALGCLVKDYFPEPVTVSWNSGALTS GVHTFPAVLQSSGLYSLSSVVTVPSSSLGTQTYICNVNHKPS NTKVDKKVEPKSCDKTHTCPPCPAPELLGGPSVFLFPPKPKD TLMISRTPEVTCVVVDVSHEDPEVKFNWYVDGVEVHNAKT KPREEQYNSTYRVVSVLTVLHQDWLNGKEYKCKVSNKALP APIEKTISKAKGQPREPQVYTLPPSRDELTKNQVSLTCLVKGF YPSDIAVEWESNGQPENNYKTTPPVLDSDGSFFLYSKLTVD KSRWQQGNVFSCSVMHEALHNHYTQKSLSLSPG | **anti-IL4-VL**-huIGKC-*[PCS1]-*<(G4S)4>-His(8)-<(G4S)4>-*[PCS1]*-**anti-IL4-VH-**huIGHG1 |
| sc-anti-IL4-His8-ΔPCL1-huIgG1 | 8 | DIQMTQSPASLSVSVGDTITLTCHASQNIDVWLSWFQQK PGNIPKLLIYKASNLHTGVPSRFSGSGSGTGFTLTISSLQPED IATYYCQQAHSYPFTFGGGTKLEIKRTVAAPSVFIFPPSDEQ LKSGTASVVCLLNNFYPREAKVQWKVDNALQSGNSQESVT EQDSKDSTYSLSSTLTLSKADYEKHKVYACEVTHQGLSSPVT KSFNRGECRQQRGGGGSGGGGSGGGGSGGGGSHHHHH HHHGGGGSGGGGSGGGGSGGGGSRQQRQVQLQQSGPE LVKPGASVKISCKASGYSFTSYWIHWIKQRPGQGLEWIG MIDPSDGETRLNQRFQGRATLTVDESTSTAYMQLRSPTSE DSAVYYCTRLKEYGNYDSFYFDVWGAGTLVTVSSASTKGP SVFPLAPSSKSTSGGTAALGCLVKDYFPEPVTVSWNSGALTS GVHTFPAVLQSSGLYSLSSVVTVPSSSLGTQTYICNVNHKPS NTKVDKKVEPKSCDKTHTCPPCPAPELLGGPSVFLFPPKPKD TLMISRTPEVTCVVVDVSHEDPEVKFNWYVDGVEVHNAKT KPREEQYNSTYRVVSVLTVLHQDWLNGKEYKCKVSNKALP APIEKTISKAKGQPREPQVYTLPPSRDELTKNQVSLTCLVKGF YPSDIAVEWESNGQPENNYKTTPPVLDSDGSFFLYSKLTVD KSRWQQGNVFSCSVMHEALHNHYTQKSLSLSPG | **anti-IL4-VL**-huIGKC-*[deltaPCS]*-<(G4S)4>-His(8)-<(G4S)4>-*[deltaPCS]*-**anti-IL4-VH**-huIGHG1 |
| sc-anti-IL4-His8-PCL2-huIgG1 | 9 | DIQMTQSPASLSVSVGDTITLTCHASQNIDVWLSWFQQK PGNIPKLLIYKASNLHTGVPSRFSGSGSGTGFTLTISSLQPED IATYYCQQAHSYPFTFGGGTKLEIKRTVAAPSVFIFPPSDEQ LKSGTASVVCLLNNFYPREAKVQWKVDNALQSGNSQESVT EQDSKDSTYSLSSTLTLSKADYEKHKVYACEVTHQGLSSPVT KSFNRGECHRRRKRSVDEGGGGSGGGGSGGGGSHHHHH HHHGGGGSGGGGSGGGGSHRRRKRSVDEQVQLQQSGPE LVKPGASVKISCKASGYSFTSYWIHWIKQRPGQGLEWIG MIDPSDGETRLNQRFQGRATLTVDESTSTAYMQLRSPTSE DSAVYYCTRLKEYGNYDSFYFDVWGAGTLVTVSSASTKGP SVFPLAPSSKSTSGGTAALGCLVKDYFPEPVTVSWNSGALTS GVHTFPAVLQSSGLYSLSSVVTVPSSSLGTQTYICNVNHKPS NTKVDKKVEPKSCDKTHTCPPCPAPELLGGPSVFLFPPKPKD TLMISRTPEVTCVVVDVSHEDPEVKFNWYVDGVEVHNAKT KPREEQYNSTYRVVSVLTVLHQDWLNGKEYKCKVSNKALP APIEKTISKAKGQPREPQVYTLPPSRDELTKNQVSLTCLVKGF YPSDIAVEWESNGQPENNYKTTPPVLDSDGSFFLYSKLTVD KSRWQQGNVFSCSVMHEALHNHYTQKSLSLSPG | **anti-IL4-VL**-huIGKC-*[PCS2]*-<(G4S)4>-His(8)-<(G4S)4>-*[PCS2]*-**anti-IL4-VH-**huIGHG1 |
| sc-anti-IL4-His8-PCL3-huIgG1 | 10 | DIQMTQSPASLSVSVGDTITLTCHASQNIDVWLSWFQQK PGNIPKLLIYKASNLHTGVPSRFSGSGSGTGFTLTISSLQPED IATYYCQQAHSYPFTFGGGTKLEIKRTVAAPSVFIFPPSDEQ LKSGTASVVCLLNNFYPREAKVQWKVDNALQSGNSQESVT EQDSKDSTYSLSSTLTLSKADYEKHKVYACEVTHQGLSSPVT KSFNRGECPMKKRRAGVPGGGGSGGGGSGGGGSHHHHH HHHGGGGSGGGGSGGGGSPMKKRRAGVPQVQLQQSGP ELVKPGASVKISCKASGYSFTSYWIHWIKQRPGQGLEWIG MIDPSDGETRLNQRFQGRATLTVDESTSTAYMQLRSPTSE DSAVYYCTRLKEYGNYDSFYFDVWGAGTLVTVSSASTKGP SVFPLAPSSKSTSGGTAALGCLVKDYFPEPVTVSWNSGALTS GVHTFPAVLQSSGLYSLSSVVTVPSSSLGTQTYICNVNHKPS NTKVDKKVEPKSCDKTHTCPPCPAPELLGGPSVFLFPPKPKD TLMISRTPEVTCVVVDVSHEDPEVKFNWYVDGVEVHNAKT KPREEQYNSTYRVVSVLTVLHQDWLNGKEYKCKVSNKALP APIEKTISKAKGQPREPQVYTLPPSRDELTKNQVSLTCLVKGF YPSDIAVEWESNGQPENNYKTTPPVLDSDGSFFLYSKLTVD KSRWQQGNVFSCSVMHEALHNHYTQKSLSLSPG | **anti-IL4-VL**-huIGKC-*[PCS3]*-<(G4S)4>-His(8)-<(G4S)4>-*[PCS3]*-**anti-IL4-VH-**huIGHG1 |
| Tandem-(anti-IL4xanti-IL13)-huIgG1 | 11 | DIQMTQSPASLSVSVGDTITLTCHASQNIDVWLSWFQQK PGNIPKLLIYKASNLHTGVPSRFSGSGSGTGFTLTISSLQPED IATYYCQQAHSYPFTFGGGTKLEIKRTVAAPSVFIFPPSDEQ LKSGTASVVCLLNNFYPREAKVQWKVDNALQSGNSQESVT EQDSKDSTYSLSSTLTLSKADYEKHKVYACEVTHQGLSSPVT KSFNRGEC | **anti-IL4-VL**-huIGKC |
| | 12 | DIVLTQSPASLAVSLGQRATISCRASESVDSYGQSYMHWY QQKAGQPPKLLIYLASNLESGVPARFSGSGSRTDFTLTIDP VQAEDAATYYCQQNAEDSRTFGGGTKLEIKRTVAAPSVFIF PPSDEQLKSGTASVVCLLNNFYPREAKVQWKVDNALQSGN SQESVTEQDSKDSTYSLSSTLTLSKADYEKHKVYACEVTHQG LSSPVTKSFNRGEC | **anti-IL13**-IGKC |
| | 13 | QVQLQQSGPELVKPGASVKISCKASGYSFTSYWIHWIKQR PGQGLEWIGMIDPSDGETRLNQRFQGRATLTVDESTSTA YMQLRSPTSEDSAVYYCTRLKEYGNYDSFYFDVWGAGTL VTVSSASTKGPSVFPLAPSSKSTSGGTAALGCLVKDYFPEPV TVSWNSGALTSGVHTFPAVLQSSGLYSLSSVVTVPSSSLGTQ TYICNVNHKPSNTKVDKKVEPKSCDKTHTGGGGSGGGGSG GGGSEVQLKESGPGLVAPGGSLSITCTVSGFSLTDSSINWV RQPPGKGLEWLGMIWGDGRIDYADALKSRLSISKDSSKS QVFLEMTSLRTDDTATYYCARDGYFPYAMDFWGQGTSV TVSSASTKGPSVFPLAPSSKSTSGGTAALGCLVKDYFPEPVT VSWNSGALTSGVHTFPAVLQSSGLYSLSSVVTVPSSSLGTQT YICNVNHKPSNTKVDKKVEPKSCDKTHTCPPCPAPELLGGP SVFLFPPKPKDTLMISRTPEVTCVVVDVSHEDPEVKFNWYV DGVEVHNAKTKPREEQYNSTYRVVSVLTVLHQDWLNGKEY KCKVSNKALPAPIEKTISKAKGQPREPQVYTLPPSRDELTKN QVSLTCLVKGFYPSDIAVEWESNGQPENNYKTTPPVLDSDG SFFLYSKLTVDKSRWQQGNVFSCSVMHEALHNHYTQKSLS LSPG | **anti-IL4-VH**-huIGHG11-huIGH-H1-<*(G4S)3>***-anti-IL13-VH**-huIGHG1 |
| sLC-Tandem-(anti-IL4xanti-IL13)-singlePCL1-1-huIgG1 | 14 | DIQMTQSPASLSVSVGDTITLTCHASQNIDVWLSWFQQK PGNIPKLLIYKASNLHTGVPSRFSGSGSGTGFTLTISSLQPED IATYYCQQAHSYPFTFGGGTKLEIKRTVAAPSVFIFPPSDEQ LKSGTASVVCLLNNFYPREAKVQWKVDNALQSGNSQESVT EQDSKDSTYSLSSTLTLSKADYEKHKVYACEVTHQGLSSPVT KSFNRGECGGGGSRRKRGGGGSDIVLTQSPASLAVSLGQR ATISCRASESVDSYGQSYMHWYQQKAGQPPKLLIYLASNL ESGVPARFSGSGSRTDFTLTIDPVQAEDAATYYCQQNAED SRTFGGGTKLEIKRTVAAPSVFIFPPSDEQLKSGTASVVCLLN NFYPREAKVQWKVDNALQSGNSQESVTEQDSKDSTYSLSS TLTLSKADYEKHKVYACEVTHQGLSSPVTKSFNRGEC | **anti-IL4-VL**-huIGKC-<G4S>-*[PCS*1*]*-<G4S>-**anti-IL13-VL**-huIGKC |
| | 13 | QVQLQQSGPELVKPGASVKISCKASGYSFTSYWIHWIKQR PGQGLEWIGMIDPSDGETRLNQRFQGRATLTVDESTSTA YMQLRSPTSEDSAVYYCTRLKEYGNYDSFYFDVWGAGTL VTVSSASTKGPSVFPLAPSSKSTSGGTAALGCLVKDYFPEPV TVSWNSGALTSGVHTFPAVLQSSGLYSLSSVVTVPSSSLGTQ TYICNVNHKPSNTKVDKKVEPKSCDKTHTGGGGSGGGGSG GGGSEVQLKESGPGLVAPGGSLSITCTVSGFSLTDSSINWV RQPPGKGLEWLGMIWGDGRIDYADALKSRLSISKDSSKS QVFLEMTSLRTDDTATYYCARDGYFPYAMDFWGQGTSV TVSSASTKGPSVFPLAPSSKSTSGGTAALGCLVKDYFPEPVT VSWNSGALTSGVHTFPAVLQSSGLYSLSSVVTVPSSSLGTQT YICNVNHKPSNTKVDKKVEPKSCDKTHTCPPCPAPELLGGP SVFLFPPKPKDTLMISRTPEVTCVVVDVSHEDPEVKFNWYV DGVEVHNAKTKPREEQYNSTYRVVSVLTVLHQDWLNGKEY KCKVSNKALPAPIEKTISKAKGQPREPQVYTLPPSRDELTKN QVSLTCLVKGFYPSDIAVEWESNGQPENNYKTTPPVLDSDG SFFLYSKLTVDKSRWQQGNVFSCSVMHEALHNHYTQKSLS LSPG | **anti-IL4-VH**-huIGHG11-huIGH-H1-<*(G4S)3*>**-anti-IL13-VH**-huIGHG1 |
| sLC-Tandem-(anti-IL4xanti-IL13)-PCL1-huIgG1 | 15 | DIQMTQSPASLSVSVGDTITLTCHASQNIDVWLSWFQQK PGNIPKLLIYKASNLHTGVPSRFSGSGSGTGFTLTISSLQPED IATYYCQQAHSYPFTFGGGTKLEIKRTVAAPSVFIFPPSDEQ LKSGTASVVCLLNNFYPREAKVQWKVDNALQSGNSQESVT EQDSKDSTYSLSSTLTLSKADYEKHKVYACEVTHQGLSSPVT KSFNRGECRRKRGGGGSGGGGSGGGGSRRKRDIVLTQSPA SLAVSLGQRATISCRASESVDSYGQSYMHWYQQKAGQP PKLLIYLASNLESGVPARFSGSGSRTDFTLTIDPVQAEDAAT YYCQQNAEDSRTFGGGTKLEIKRTVAAPSVFIFPPSDEQLKS GTASVVCLLNNFYPREAKVQWKVDNALQSGNSQESVTEQ DSKDSTYSLSSTLTLSKADYEKHKVYACEVTHQGLSSPVTKSF NRGEC | **anti-IL4-VL**-huIGKC-*[PCS1]-<*(G4S)3>-*[PCS1]*-**anti-IL13-VL-**huIGKC |
| | 13 | QVQLQQSGPELVKPGASVKISCKASGYSFTSYWIHWIKQR PGQGLEWIGMIDPSDGETRLNQRFQGRATLTVDESTSTA YMQLRSPTSEDSAVYYCTRLKEYGNYDSFYFDVWGAGTL VTVSSASTKGPSVFPLAPSSKSTSGGTAALGCLVKDYFPEPV TVSWNSGALTSGVHTFPAVLQSSGLYSLSSVVTVPSSSLGTQ TYICNVNHKPSNTKVDKKVEPKSCDKTHTGGGGSGGGGSG GGGSEVQLKESGPGLVAPGGSLSITCTVSGFSLTDSSINWV RQPPGKGLEWLGMIWGDGRIDYADALKSRLSISKDSSKS QVFLEMTSLRTDDTATYYCARDGYFPYAMDFWGQGTSV TVSSASTKGPSVFPLAPSSKSTSGGTAALGCLVKDYFPEPVT VSWNSGALTSGVHTFPAVLQSSGLYSLSSVVTVPSSSLGTQT YICNVNHKPSNTKVDKKVEPKSCDKTHTCPPCPAPELLGGP SVFLFPPKPKDTLMISRTPEVTCVVVDVSHEDPEVKFNWYV DGVEVHNAKTKPREEQYNSTYRVVSVLTVLHQDWLNGKEY KCKVSNKALPAPIEKTISKAKGQPREPQVYTLPPSRDELTKN QVSLTCLVKGFYPSDIAVEWESNGQPENNYKTTPPVLDSDG SFFLYSKLTVDKSRWQQGNVFSCSVMHEALHNHYTQKSLS LSPG | **anti-IL4-VH**-huIGHG11-huIGH-H1-<*(G4S)3>*-**anti-IL13-VH**-huIGHG1 |
| sLC-Tandem-(anti-IL4xanti-IL13)-ΔPCL1-huIgG1 | 16 | DIQMTQSPASLSVSVGDTITLTCHASQNIDVWLSWFQQK PGNIPKLLIYKASNLHTGVPSRFSGSGSGTGFTLTISSLQPED IATYYCQQAHSYPFTFGGGTKLEIKRTVAAPSVFIFPPSDEQ LKSGTASVVCLLNNFYPREAKVQWKVDNALQSGNSQESVT EQDSKDSTYSLSSTLTLSKADYEKHKVYACEVTHQGLSSPVT KSFNRGECRQQRGGGGSGGGGSGGGGSRQQRDIVLTQSP ASLAVSLGQRATISCRASESVDSYGQSYMHWYQQKAGQ PPKLLIYLASNLESGVPARFSGSGSRTDFTLTIDPVQAEDAA TYYCQQNAEDSRTFGGGTKLEIKRTVAAPSVFIFPPSDEQLK SGTASVVCLLNNFYPREAKVQWKVDNALQSGNSQESVTEQ DSKDSTYSLSSTLTLSKADYEKHKVYACEVTHQGLSSPVTKSF NRGEC | **anti-IL4-VL**-huIGKC-*[deltaPCS]-*<(G4S)3>-*[deltaPCS]-***anti-IL13-**VL-huIGKC |
| | 13 | QVQLQQSGPELVKPGASVKISCKASGYSFTSYWIHWIKQR PGQGLEWIGMIDPSDGETRLNQRFQGRATLTVDESTSTA YMQLRSPTSEDSAVYYCTRLKEYGNYDSFYFDVWGAGTL VTVSSASTKGPSVFPLAPSSKSTSGGTAALGCLVKDYFPEPV TVSWNSGALTSGVHTFPAVLQSSGLYSLSSVVTVPSSSLGTQ TYICNVNHKPSNTKVDKKVEPKSCDKTHTGGGGSGGGGSG GGGSEVQLKESGPGLVAPGGSLSITCTVSGFSLTDSSINWV RQPPGKGLEWLGMIWGDGRIDYADALKSRLSISKDSSKS QVFLEMTSLRTDDTATYYCARDGYFPYAMDFWGQGTSV TVSSASTKGPSVFPLAPSSKSTSGGTAALGCLVKDYFPEPVT VSWNSGALTSGVHTFPAVLQSSGLYSLSSVVTVPSSSLGTQT YICNVNHKPSNTKVDKKVEPKSCDKTHTCPPCPAPELLGGP SVFLFPPKPKDTLMISRTPEVTCVVVDVSHEDPEVKFNWYV DGVEVHNAKTKPREEQYNSTYRVVSVLTVLHQDWLNGKEY KCKVSNKALPAPIEKTISKAKGQPREPQVYTLPPSRDELTKN QVSLTCLVKGFYPSDIAVEWESNGQPENNYKTTPPVLDSDG SFFLYSKLTVDKSRWQQGNVFSCSVMHEALHNHYTQKSLS LSPG | **anti-IL4-VH**-huIGHG11-huIGH-H1-<*(G4S)3>*-a**nti-IL13-VH**-huIGHG1 |
| sLC-Tandem-(anti-IL4xanti-IL13)-His8-PCL1-huIgG1 | 17 | DIQMTQSPASLSVSVGDTITLTCHASQNIDVWLSWFQQK PGNIPKLLIYKASNLHTGVPSRFSGSGSGTGFTLTISSLQPED IATYYCQQAHSYPFTFGGGTKLEIKRTVAAPSVFIFPPSDEQ LKSGTASVVCLLNNFYPREAKVQWKVDNALQSGNSQESVT EQDSKDSTYSLSSTLTLSKADYEKHKVYACEVTHQGLSSPVT KSFNRGECRRKRGSHHHHHHHHGGGGSRRKRDIVLTQSP ASLAVSLGQRATISCRASESVDSYGQSYMHWYQQKAGQ PPKLLIYLASNLESGVPARFSGSGSRTDFTLTIDPVQAEDAA TYYCQQNAEDSRTFGGGTKLEIKRTVAAPSVFIFPPSDEQLK SGTASVVCLLNNFYPREAKVQWKVDNALQSGNSQESVTEQ DSKDSTYSLSSTLTLSKADYEKHKVYACEVTHQGLSSPVTKSF NRGEC | **anti-IL4-VL**-huIGKC-*[PCS1]-*<GS>-His(8)-<G4S>-*[PCS1]-***anti-IL13-VL**-huIGKC |
| | 13 | QVQLQQSGPELVKPGASVKISCKASGYSFTSYWIHWIKQR PGQGLEWIGMIDPSDGETRLNQRFQGRATLTVDESTSTA YMQLRSPTSEDSAVYYCTRLKEYGNYDSFYFDVWGAGTL VTVSSASTKGPSVFPLAPSSKSTSGGTAALGCLVKDYFPEPV TVSWNSGALTSGVHTFPAVLQSSGLYSLSSVVTVPSSSLGTQ TYICNVNHKPSNTKVDKKVEPKSCDKTHTGGGGSGGGGSG GGGSEVQLKESGPGLVAPGGSLSITCTVSGFSLTDSSINWV RQPPGKGLEWLGMIWGDGRIDYADALKSRLSISKDSSKS QVFLEMTSLRTDDTATYYCARDGYFPYAMDFWGQGTSV TVSSASTKGPSVFPLAPSSKSTSGGTAALGCLVKDYFPEPVT VSWNSGALTSGVHTFPAVLQSSGLYSLSSVVTVPSSSLGTQT YICNVNHKPSNTKVDKKVEPKSCDKTHTCPPCPAPELLGGP SVFLFPPKPKDTLMISRTPEVTCVVVDVSHEDPEVKFNWYV DGVEVHNAKTKPREEQYNSTYRVVSVLTVLHQDWLNGKEY KCKVSNKALPAPIEKTISKAKGQPREPQVYTLPPSRDELTKN QVSLTCLVKGFYPSDIAVEWESNGQPENNYKTTPPVLDSDG SFFLYSKLTVDKSRWQQGNVFSCSVMHEALHNHYTQKSLS LSPG | **anti-IL4-VH**-huIGHG11-huIGH-H1-<*(G4S)3*>**-anti-IL13-VH**-huIGHG1 |
| sLC-Tandem-(anti-IL4xanti-IL13)-His8-ΔPCL1-huIgG1 | 18 | DIQMTQSPASLSVSVGDTITLTCHASQNIDVWLSWFQQK PGNIPKLLIYKASNLHTGVPSRFSGSGSGTGFTLTISSLQPED IATYYCQQAHSYPFTFGGGTKLEIKRTVAAPSVFIFPPSDEQ LKSGTASVVCLLNNFYPREAKVQWKVDNALQSGNSQESVT EQDSKDSTYSLSSTLTLSKADYEKHKVYACEVTHQGLSSPVT KSFNRGECRQQRGSHHHHHHHHGGGGSRQQRDIVLTQS PASLAVSLGQRATISCRASESVDSYGQSYMHWYQQKAG QPPKLLIYLASNLESGVPARFSGSGSRTDFTLTIDPVQAEDA ATYYCQQNAEDSRTFGGGTKLEIKRTVAAPSVFIFPPSDEQ LKSGTASVVCLLNNFYPREAKVQWKVDNALQSGNSQESVT EQDSKDSTYSLSSTLTLSKADYEKHKVYACEVTHQGLSSPVT KSFNRGEC | **anti-IL4-VL**-huIGKC-*[deltaPCS]-*<GS>-His(8)-<G4S>-*[deltaPCS]*-**anti-IL13-VL**-huIGKC |
| | 13 | QVQLQQSGPELVKPGASVKISCKASGYSFTSYWIHWIKQR PGQGLEWIGMIDPSDGETRLNQRFQGRATLTVDESTSTA YMQLRSPTSEDSAVYYCTRLKEYGNYDSFYFDVWGAGTL VTVSSASTKGPSVFPLAPSSKSTSGGTAALGCLVKDYFPEPV TVSWNSGALTSGVHTFPAVLQSSGLYSLSSVVTVPSSSLGTQ TYICNVNHKPSNTKVDKKVEPKSCDKTHTGGGGSGGGGSG GGGSEVQLKESGPGLVAPGGSLSITCTVSGFSLTDSSINWV RQPPGKGLEWLGMIWGDGRIDYADALKSRLSISKDSSKS QVFLEMTSLRTDDTATYYCARDGYFPYAMDFWGQGTSV TVSSASTKGPSVFPLAPSSKSTSGGTAALGCLVKDYFPEPVT VSWNSGALTSGVHTFPAVLQSSGLYSLSSVVTVPSSSLGTQT YICNVNHKPSNTKVDKKVEPKSCDKTHTCPPCPAPELLGGP SVFLFPPKPKDTLMISRTPEVTCVVVDVSHEDPEVKFNWYV DGVEVHNAKTKPREEQYNSTYRVVSVLTVLHQDWLNGKEY KCKVSNKALPAPIEKTISKAKGQPREPQVYTLPPSRDELTKN QVSLTCLVKGFYPSDIAVEWESNGQPENNYKTTPPVLDSDG SFFLYSKLTVDKSRWQQGNVFSCSVMHEALHNHYTQKSLS LSPG | **anti-IL4-VH**-huIGHG11-huIGH-H1-**< (G4S)3>-anti-IL13-**VH-huIGHG1 |
| sLC-Tandem-(anti-IL4xanti-IL13)-PCL2-huIgG1 | 19 | DIQMTQSPASLSVSVGDTITLTCHASQNIDVWLSWFQQK PGNIPKLLIYKASNLHTGVPSRFSGSGSGTGFTLTISSLQPED IATYYCQQAHSYPFTFGGGTKLEIKRTVAAPSVFIFPPSDEQ LKSGTASVVCLLNNFYPREAKVQWKVDNALQSGNSQESVT EQDSKDSTYSLSSTLTLSKADYEKHKVYACEVTHQGLSSPVT KSFNRGECHRRRKRSVDEGGGGSHRRRKRSVDEDIVLTQS PASLAVSLGQRATISCRASESVDSYGQSYMHWYQQKAG QPPKLLIYLASNLESGVPARFSGSGSRTDFTLTIDPVQAEDA ATYYCQQNAEDSRTFGGGTKLEIKRTVAAPSVFIFPPSDEQ LKSGTASVVCLLNNFYPREAKVQWKVDNALQSGNSQESVT EQDSKDSTYSLSSTLTLSKADYEKHKVYACEVTHQGLSSPVT KSFNRGEC | **anti-IL4-VL**-huIGKC-*[PCS2]*-<(G4S)3>-*[PCS2]*-**anti-IL13-VL-**huIGKC |
| | 13 | QVQLQQSGPELVKPGASVKISCKASGYSFTSYWIHWIKQR PGQGLEWIGMIDPSDGETRLNQRFQGRATLTVDESTSTA YMQLRSPTSEDSAVYYCTRLKEYGNYDSFYFDVWGAGTL VTVSSASTKGPSVFPLAPSSKSTSGGTAALGCLVKDYFPEPV TVSWNSGALTSGVHTFPAVLQSSGLYSLSSVVTVPSSSLGTQ TYICNVNHKPSNTKVDKKVEPKSCDKTHTGGGGSGGGGSG GGGSEVQLKESGPGLVAPGGSLSITCTVSGFSLTDSSINWV RQPPGKGLEWLGMIWGDGRIDYADALKSRLSISKDSSKS QVFLEMTSLRTDDTATYYCARDGYFPYAMDFWGQGTSV TVSSASTKGPSVFPLAPSSKSTSGGTAALGCLVKDYFPEPVT VSWNSGALTSGVHTFPAVLQSSGLYSLSSVVTVPSSSLGTQT YICNVNHKPSNTKVDKKVEPKSCDKTHTCPPCPAPELLGGP SVFLFPPKPKDTLMISRTPEVTCVVVDVSHEDPEVKFNWYV DGVEVHNAKTKPREEQYNSTYRVVSVLTVLHQDWLNGKEY KCKVSNKALPAPIEKTISKAKGQPREPQVYTLPPSRDELTKN QVSLTCLVKGFYPSDIAVEWESNGQPENNYKTTPPVLDSDG SFFLYSKLTVDKSRWQQGNVFSCSVMHEALHNHYTQKSLS LSPG | **anti-IL4-VH**-huIGHG11-huIGH-H1-<*(G4S)3>*-a**nti-IL13-VH**-huIGHG1 |
| sLC-Tandem-(anti-IL4xanti-IL13)-PCL3-huIgG1 | 20 | DIQMTQSPASLSVSVGDTITLTCHASQNIDVWLSWFQQK PGNIPKLLIYKASNLHTGVPSRFSGSGSGTGFTLTISSLQPED IATYYCQQAHSYPFTFGGGTKLEIKRTVAAPSVFIFPPSDEQ LKSGTASVVCLLNNFYPREAKVQWKVDNALQSGNSQESVT EQDSKDSTYSLSSTLTLSKADYEKHKVYACEVTHQGLSSPVT KSFNRGECPMKKRRAGVPGGGGSPMKKRRAGVPDIVLTQ SPASLAVSLGQRATISCRASESVDSYGQSYMHWYQQKAG QPPKLLIYLASNLESGVPARFSGSGSRTDFTLTIDPVQAEDA ATYYCQQNAEDSRTFGGGTKLEIKRTVAAPSVFIFPPSDEQ LKSGTASVVCLLNNFYPREAKVQWKVDNALQSGNSQESVT EQDSKDSTYSLSSTLTLSKADYEKHKVYACEVTHQGLSSPVT KSFNRGEC | **anti-IL4-VL**-huIGKC-*[PCS3]-*<(G4S)>-*[PCS3]***-anti-IL13-VL-**huIGKC |
| | 13 | QVQLQQSGPELVKPGASVKISCKASGYSFTSYWIHWIKQR PGQGLEWIGMIDPSDGETRLNQRFQGRATLTVDESTSTA YMQLRSPTSEDSAVYYCTRLKEYGNYDSFYFDVWGAGTL VTVSSASTKGPSVFPLAPSSKSTSGGTAALGCLVKDYFPEPV TVSWNSGALTSGVHTFPAVLQSSGLYSLSSVVTVPSSSLGTQ TYICNVNHKPSNTKVDKKVEPKSCDKTHTGGGGSGGGGSG GGGSEVQLKESGPGLVAPGGSLSITCTVSGFSLTDSSINWV RQPPGKGLEWLGMIWGDGRIDYADALKSRLSISKDSSKS QVFLEMTSLRTDDTATYYCARDGYFPYAMDFWGQGTSV TVSSASTKGPSVFPLAPSSKSTSGGTAALGCLVKDYFPEPVT VSWNSGALTSGVHTFPAVLQSSGLYSLSSVVTVPSSSLGTQT YICNVNHKPSNTKVDKKVEPKSCDKTHTCPPCPAPELLGGP SVFLFPPKPKDTLMISRTPEVTCVVVDVSHEDPEVKFNWYV DGVEVHNAKTKPREEQYNSTYRVVSVLTVLHQDWLNGKEY KCKVSNKALPAPIEKTISKAKGQPREPQVYTLPPSRDELTKN QVSLTCLVKGFYPSDIAVEWESNGQPENNYKTTPPVLDSDG SFFLYSKLTVDKSRWQQGNVFSCSVMHEALHNHYTQKSLS LSPG | **anti-IL4-VH**-huIGHG11-huIGH-H1-**< (G4S)3>-anti-IL13-VH**-huIGHG1 |
| sLC-Tandem-(anti-IL4xanti-IL13)-rigidL1-PCL1-huIgG1 | 21 | DIQMTQSPASLSVSVGDTITLTCHASQNIDVWLSWFQQK PGNIPKLLIYKASNLHTGVPSRFSGSGSGTGFTLTISSLQPED IATYYCQQAHSYPFTFGGGTKLEIKRTVAAPSVFIFPPSDEQ LKSGTASVVCLLNNFYPREAKVQWKVDNALQSGNSQESVT EQDSKDSTYSLSSTLTLSKADYEKHKVYACEVTHQGLSSPVT KSFNRGECRRKRGGGGSEAAAKEAAAKGGGGSRRKRDIVL TQSPASLAVSLGQRATISCRASESVDSYGQSYMHWYQQK AGQPPKLLIYLASNLESGVPARFSGSGSRTDFTLTIDPVQAE DAATYYCQQNAEDSRTFGGGTKLEIKRTVAAPSVFIFPPSD EQLKSGTASVVCLLNNFYPREAKVQWKVDNALQSGNSQES VTEQDSKDSTYSLSSTLTLSKADYEKHKVYACEVTHQGLSSP VTKSFNRGEC | **anti-IL4-VL**-huIGKC-*[PCS1]*-<G4S>-<(EAAAK)2>-<G4S>-*[PCS1]*-**anti-IL13-VL-**huIGKC |
| | 22 | QVQLQQSGPELVKPGASVKISCKASGYSFTSYWIHWIKQR PGQGLEWIGMIDPSDGETRLNQRFQGRATLTVDESTSTA YMQLRSPTSEDSAVYYCTRLKEYGNYDSFYFDVWGAGTL VTVSSASTKGPSVFPLAPSSKSTSGGTAALGCLVKDYFPEPV TVSWNSGALTSGVHTFPAVLQSSGLYSLSSVVTVPSSSLGTQ TYICNVNHKPSNTKVDKKVEPKSCDKTHTGGGGSEAAAKE AAAKGGGGSEVQLKESGPGLVAPGGSLSITCTVSGFSLTDS SINWVRQPPGKGLEWLGMIWGDGRIDYADALKSRLSISK DSSKSQVFLEMTSLRTDDTATYYCARDGYFPYAMDFWG QGTSVTVSSASTKGPSVFPLAPSSKSTSGGTAALGCLVKDYF PEPVTVSWNSGALTSGVHTFPAVLQSSGLYSLSSVVTVPSSS LGTQTYICNVNHKPSNTKVDKKVEPKSCDKTHTCPPCPAPE LLGGPSVFLFPPKPKDTLMISRTPEVTCVVVDVSHEDPEVKF NWYVDGVEVHNAKTKPREEQYNSTYRVVSVLTVLHQDWL NGKEYKCKVSNKALPAPIEKTISKAKGQPREPQVYTLPPSRD ELTKNQVSLTCLVKGFYPSDIAVEWESNGQPENNYKTTPPV LDSDGSFFLYSKLTVDKSRWQQGNVFSCSVMHEALHNHYT QKSLSLSPG | **anti-IL4-VH**-huIGHG11-hulGH-H1-<G4S>-<(EAAAK)2>-<G4S>-**anti-IL13-VH**-huIGHG1 |
| sLC-Tandem-(anti-IL4xanti-IL13)-rigidL2-PCL1-huIgG1 | 23 | DIQMTQSPASLSVSVGDTITLTCHASQNIDVWLSWFQQK PGNIPKLLIYKASNLHTGVPSRFSGSGSGTGFTLTISSLQPED IATYYCQQAHSYPFTFGGGTKLEIKRTVAAPSVFIFPPSDEQ LKSGTASVVCLLNNFYPREAKVQWKVDNALQSGNSQESVT EQDSKDSTYSLSSTLTLSKADYEKHKVYACEVTHQGLSSPVT KSFNRGECRRKRAPAPAPAPAPAPAPARRKRDIVLTQSPAS LAVSLGQRATISCRASESVDSYGQSYMHWYQQKAGQPP KLLIYLASNLESGVPARFSGSGSRTDFTLTIDPVQAEDAATY YCQQNAEDSRTFGGGTKLEIKRTVAAPSVFIFPPSDEQLKS GTASVVCLLNNFYPREAKVQWKVDNALQSGNSQESVTEQ DSKDSTYSLSSTLTLSKADYEKHKVYACEVTHQGLSSPVTKSF NRGEC | **anti-IL4-VL**-huIGKC-*[PCS1]*-<(AP)7A>-*[PCS1]*-**anti-IL13-VL-**huIGKC |
| | 13 | QVQLQQSGPELVKPGASVKISCKASGYSFTSYWIHWIKQR PGQGLEWIGMIDPSDGETRLNQRFQGRATLTVDESTSTA YMQLRSPTSEDSAVYYCTRLKEYGNYDSFYFDVWGAGTL VTVSSASTKGPSVFPLAPSSKSTSGGTAALGCLVKDYFPEPV TVSWNSGALTSGVHTFPAVLQSSGLYSLSSVVTVPSSSLGTQ TYICNVNHKPSNTKVDKKVEPKSCDKTHTGGGGSGGGGSG GGGSEVQLKESGPGLVAPGGSLSITCTVSGFSLTDSSINWV RQPPGKGLEWLGMIWGDGRIDYADALKSRLSISKDSSKS QVFLEMTSLRTDDTATYYCARDGYFPYAMDFWGQGTSV TVSSASTKGPSVFPLAPSSKSTSGGTAALGCLVKDYFPEPVT VSWNSGALTSGVHTFPAVLQSSGLYSLSSVVTVPSSSLGTQT YICNVNHKPSNTKVDKKVEPKSCDKTHTCPPCPAPELLGGP SVFLFPPKPKDTLMISRTPEVTCVVVDVSHEDPEVKFNWYV DGVEVHNAKTKPREEQYNSTYRVVSVLTVLHQDWLNGKEY KCKVSNKALPAPIEKTISKAKGQPREPQVYTLPPSRDELTKN QVSLTCLVKGFYPSDIAVEWESNGQPENNYKTTPPVLDSDG SFFLYSKLTVDKSRWQQGNVFSCSVMHEALHNHYTQKSLS LSPG | **anti-IL4-VH**-huIGHG11-huIGH-H1-<*(G4S)*3>-**anti-IL13-VH**-huIGHG1 |
| sLC-Tandem-(anti-IL4xanti-IL13)-charL-PCL1-huIgG1 | 24 | DIQMTQSPASLSVSVGDTITLTCHASQNIDVWLSWFQQK PGNIPKLLIYKASNLHTGVPSRFSGSGSGTGFTLTISSLQPED IATYYCQQAHSYPFTFGGGTKLEIKRTVAAPSVFIFPPSDEQ LKSGTASVVCLLNNFYPREAKVQWKVDNALQSGNSQESVT EQDSKDSTYSLSSTLTLSKADYEKHKVYACEVTHQGLSSPVT KSFNRGECRRKRGSKQGKQKQGKQGSRRKRDIVLTQSPAS LAVSLGQRATISCRASESVDSYGQSYMHWYQQKAGQPP KLLIYLASNLESGVPARFSGSGSRTDFTLTIDPVQAEDAATY YCQQNAEDSRTFGGGTKLEIKRTVAAPSVFIFPPSDEQLKS GTASVVCLLNNFYPREAKVQWKVDNALQSGNSQESVTEQ DSKDSTYSLSSTLTLSKADYEKHKVYACEVTHQGLSSPVTKSF NRGEC | **anti-IL4-VL**-huIGKC-*[PCS1]*-<GS>-<(KQGKQ)2>-<GS>-*[PCS1]***-anti-IL13-VL-**huIGKC |
| | 13 | QVQLQQSGPELVKPGASVKISCKASGYSFTSYWIHWIKQR PGQGLEWIGMIDPSDGETRLNQRFQGRATLTVDESTSTA YMQLRSPTSEDSAVYYCTRLKEYGNYDSFYFDVWGAGTL VTVSSASTKGPSVFPLAPSSKSTSGGTAALGCLVKDYFPEPV TVSWNSGALTSGVHTFPAVLQSSGLYSLSSVVTVPSSSLGTQ TYICNVNHKPSNTKVDKKVEPKSCDKTHTGGGGSGGGGSG GGGSEVQLKESGPGLVAPGGSLSITCTVSGFSLTDSSINWV RQPPGKGLEWLGMIWGDGRIDYADALKSRLSISKDSSKS QVFLEMTSLRTDDTATYYCARDGYFPYAMDFWGQGTSV TVSSASTKGPSVFPLAPSSKSTSGGTAALGCLVKDYFPEPVT VSWNSGALTSGVHTFPAVLQSSGLYSLSSVVTVPSSSLGTQT YICNVNHKPSNTKVDKKVEPKSCDKTHTCPPCPAPELLGGP SVFLFPPKPKDTLMISRTPEVTCVVVDVSHEDPEVKFNWYV DGVEVHNAKTKPREEQYNSTYRVVSVLTVLHQDWLNGKEY KCKVSNKALPAPIEKTISKAKGQPREPQVYTLPPSRDELTKN QVSLTCLVKGFYPSDIAVEWESNGQPENNYKTTPPVLDSDG SFFLYSKLTVDKSRWQQGNVFSCSVMHEALHNHYTQKSLS LSPG | **anti-IL4-VH**-huIGHG11-huIGH-H1-**< (G4S)3>-anti-IL13-**VH-huIGHG1 |
| scTandem-(anti-IL4xanti-IL13)-PCL1-huIgG1-OL-KIH | 25 | DIQMTQSPASLSVSVGDTITLTCHASQNIDVWLSWFQQK PGNIPKLLIYKASNLHTGVPSRFSGSGSGTGFTLTISSLQPED IATYYCQQAHSYPFTFGGGTKLEIKRTVAAPSVFIFPPSDEQ LKSGTASVVCLLNNFYPREAKVQWKVDNALQSGNSQESVT EQDSKDSTYSLSSTLTLSKADYEKHKVYACEVTHQGLSSPVT KSFNRGECRRKRGGGGSGGGGSGGGGSRRKRDIVLTQSPA SLAVSLGQRATISCRASESVDSYGQSYMHWYQQKAGQP PKLLIYLASNLESGVPARFSGSGSRTDFTLTIDPVQAEDAAT YYCQQNAEDSRTFGGGTKLEIKRTVAAPSVFIFPPSDEQLKS GTASVVCLLNNFYPREAKVQWKVDNALQSGNSQESVTEQ DSKDSTYSLSSTLTLSKADYEKHKVYACEVTHQGLSSPVTKSF NRGECGGGGSDKTHTCPPCPAPELLGGPSVFLFPPKPKDTL MISRTPEVTCVVVDVSHEDPEVKFNWYVDGVEVHNAKTKP REEQYNSTYRVVSVLTVLHQDWLNGKEYKCKVSNKALPAPI EKTISKAKGQPREPQVCTLPPSRDELTKNQVSLSCAVKGFYP SDIAVEWESNGQPENNYKTTPPVLDSDGSFFLVSKLTVDKS RWQQGNVFSCSVMHEALHNRFTQKSLSLSPG | **anti-IL4-VL**-huIGKC-*[PCS1]*-<(G4S)3>-*[PCS1]*-**anti-IL13-VL-**huIGKC-<G4S>-Fc-*huIgG1(hole-RF)* |
| | 26 | QVQLQQSGPELVKPGASVKISCKASGYSFTSYWIHWIKQR PGQGLEWIGMIDPSDGETRLNQRFQGRATLTVDESTSTA YMQLRSPTSEDSAVYYCTRLKEYGNYDSFYFDVWGAGTL VTVSSASTKGPSVFPLAPSSKSTSGGTAALGCLVKDYFPEPV TVSWNSGALTSGVHTFPAVLQSSGLYSLSSVVTVPSSSLGTQ TYICNVNHKPSNTKVDKKVEPKSCDKTHTGGGGSGGGGSG GGGSEVQLKESGPGLVAPGGSLSITCTVSGFSLTDSSINWV RQPPGKGLEWLGMIWGDGRIDYADALKSRLSISKDSSKS QVFLEMTSLRTDDTATYYCARDGYFPYAMDFWGQGTSV TVSSASTKGPSVFPLAPSSKSTSGGTAALGCLVKDYFPEPVT VSWNSGALTSGVHTFPAVLQSSGLYSLSSVVTVPSSSLGTQT YICNVNHKPSNTKVDKKVEPKSCDKTHTCPPCPAPELLGGP SVFLFPPKPKDTLMISRTPEVTCVVVDVSHEDPEVKFNWYV DGVEVHNAKTKPREEQYNSTYRVVSVLTVLHQDWLNGKEY KCKVSNKALPAPIEKTISKAKGQPREPQVYTLPPCRDELTKN QVSLWCLVKGFYPSDIAVEWESNGQPENNYKTTPPVLDSD GSFFLYSKLTVDKSRWQQGNVFSCSVMHEALHNHYTQKSL SLSPG | **anti-IL4-VH**-huIGHG11-huIGH-H1-<(G4S)3>-**anti-IL13-VH**-huIGHG1(knob) |
| scTandem-(anti-IL4xanti-IL13)-singlePCL1-huIgG1-OL-KIH | 27 | DIQMTQSPASLSVSVGDTITLTCHASQNIDVWLSWFQQK PGNIPKLLIYKASNLHTGVPSRFSGSGSGTGFTLTISSLQPED IATYYCQQAHSYPFTFGGGTKLEIKRTVAAPSVFIFPPSDEQ LKSGTASVVCLLNNFYPREAKVQWKVDNALQSGNSQESVT EQDSKDSTYSLSSTLTLSKADYEKHKVYACEVTHQGLSSPVT KSFNRGECGGGGSRRKRGGGGSDIVLTQSPASLAVSLGQR ATISCRASESVDSYGQSYMHWYQQKAGQPPKLLIYLASNL ESGVPARFSGSGSRTDFTLTIDPVQAEDAATYYCQQNAED SRTFGGGTKLEIKRTVAAPSVFIFPPSDEQLKSGTASVVCLLN NFYPREAKVQWKVDNALQSGNSQESVTEQDSKDSTYSLSS TLTLSKADYEKHKVYACEVTHQGLSSPVTKSFNRGECGGGG SDKTHTCPPCPAPELLGGPSVFLFPPKPKDTLMISRTPEVTCV VVDVSHEDPEVKFNWYVDGVEVHNAKTKPREEQYNSTYR VVSVLTVLHQDWLNGKEYKCKVSNKALPAPIEKTISKAKGQ PREPQVCTLPPSRDELTKNQVSLSCAVKGFYPSDIAVEWESN GQPENNYKTTPPVLDSDGSFFLVSKLTVDKSRWQQGNVFS CSVMHEALHNRFTQKSLSLSPG | **anti-IL4-VL**-huIGKC-<G4S>-*[PCS1]*-<G4S>-**anti-IL13-VL**-huIGKC-<G4S>-*Fc-huIgG1(hole-RF)* |
| | 26 | QVQLQQSGPELVKPGASVKISCKASGYSFTSYWIHWIKQR PGQGLEWIGMIDPSDGETRLNQRFQGRATLTVDESTSTA YMQLRSPTSEDSAVYYCTRLKEYGNYDSFYFDVWGAGTL VTVSSASTKGPSVFPLAPSSKSTSGGTAALGCLVKDYFPEPV TVSWNSGALTSGVHTFPAVLQSSGLYSLSSVVTVPSSSLGTQ TYICNVNHKPSNTKVDKKVEPKSCDKTHTGGGGSGGGGSG GGGSEVQLKESGPGLVAPGGSLSITCTVSGFSLTDSSINWV RQPPGKGLEWLGMIWGDGRIDYADALKSRLSISKDSSKS QVFLEMTSLRTDDTATYYCARDGYFPYAMDFWGQGTSV TVSSASTKGPSVFPLAPSSKSTSGGTAALGCLVKDYFPEPVT VSWNSGALTSGVHTFPAVLQSSGLYSLSSVVTVPSSSLGTQT YICNVNHKPSNTKVDKKVEPKSCDKTHTCPPCPAPELLGGP SVFLFPPKPKDTLMISRTPEVTCVVVDVSHEDPEVKFNWYV DGVEVHNAKTKPREEQYNSTYRVVSVLTVLHQDWLNGKEY KCKVSNKALPAPIEKTISKAKGQPREPQVYTLPPCRDELTKN QVSLWCLVKGFYPSDIAVEWESNGQPENNYKTTPPVLDSD GSFFLYSKLTVDKSRWQQGNVFSCSVMHEALHNHYTQKSL SLSPG | **anti-IL4-VH**-huIGHG11-huIGH-H1-<(G4S)3>-**anti-IL13-VH**-huIGHG1(knob) |
| scTandem-(anti-IL4xanti-IL13)-ΔPCL1-huIgG1-OL-KIH | 28 | DIQMTQSPASLSVSVGDTITLTCHASQNIDVWLSWFQQK PGNIPKLLIYKASNLHTGVPSRFSGSGSGTGFTLTISSLQPED IATYYCQQAHSYPFTFGGGTKLEIKRTVAAPSVFIFPPSDEQ LKSGTASVVCLLNNFYPREAKVQWKVDNALQSGNSQESVT EQDSKDSTYSLSSTLTLSKADYEKHKVYACEVTHQGLSSPVT KSFNRGECRQQRGGGGSGGGGSGGGGSRQQRDIVLTQSP ASLAVSLGQRATISCRASESVDSYGQSYMHWYQQKAGQ PPKLLIYLASNLESGVPARFSGSGSRTDFTLTIDPVQAEDAA TYYCQQNAEDSRTFGGGTKLEIKRTVAAPSVFIFPPSDEQLK SGTASVVCLLNNFYPREAKVQWKVDNALQSGNSQESVTEQ DSKDSTYSLSSTLTLSKADYEKHKVYACEVTHQGLSSPVTKSF NRGECGGGGSDKTHTCPPCPAPELLGGPSVFLFPPKPKDTL MISRTPEVTCVVVDVSHEDPEVKFNWYVDGVEVHNAKTKP REEQYNSTYRVVSVLTVLHQDWLNGKEYKCKVSNKALPAPI EKTISKAKGQPREPQVCTLPPSRDELTKNQVSLSCAVKGFYP SDIAVEWESNGQPENNYKTTPPVLDSDGSFFLVSKLTVDKS RWQQGNVFSCSVMHEALHNRFTQKSLSLSPG | **anti-IL4-VL**-huIGKC-*[deltaPCS]*-<(G4S)3>-*[deltaPCS]***-anti-IL13-VL**-huIGKC-<G4S>-Fc-*huIgG1(hole-RF)* |
| | 26 | QVQLQQSGPELVKPGASVKISCKASGYSFTSYWIHWIKQR PGQGLEWIGMIDPSDGETRLNQRFQGRATLTVDESTSTA YMQLRSPTSEDSAVYYCTRLKEYGNYDSFYFDVWGAGTL VTVSSASTKGPSVFPLAPSSKSTSGGTAALGCLVKDYFPEPV TVSWNSGALTSGVHTFPAVLQSSGLYSLSSVVTVPSSSLGTQ TYICNVNHKPSNTKVDKKVEPKSCDKTHTGGGGSGGGGSG GGGSEVQLKESGPGLVAPGGSLSITCTVSGFSLTDSSINWV RQPPGKGLEWLGMIWGDGRIDYADALKSRLSISKDSSKS QVFLEMTSLRTDDTATYYCARDGYFPYAMDFWGQGTSV TVSSASTKGPSVFPLAPSSKSTSGGTAALGCLVKDYFPEPVT VSWNSGALTSGVHTFPAVLQSSGLYSLSSVVTVPSSSLGTQT YICNVNHKPSNTKVDKKVEPKSCDKTHTCPPCPAPELLGGP SVFLFPPKPKDTLMISRTPEVTCVVVDVSHEDPEVKFNWYV DGVEVHNAKTKPREEQYNSTYRVVSVLTVLHQDWLNGKEY KCKVSNKALPAPIEKTISKAKGQPREPQVYTLPPCRDELTKN QVSLWCLVKGFYPSDIAVEWESNGQPENNYKTTPPVLDSD GSFFLYSKLTVDKSRWQQGNVFSCSVMHEALHNHYTQKSL SLSPG | **anti-IL4-VH**-huIGHG11-huIGH-H1-<(G4S)3>-anti-**IL13-VH**-huIGHG1(knob) |
| scTandem-(anti-IL4xanti-IL13)-His8-PCL1-huIgG1-OL-KIH | 29 | DIQMTQSPASLSVSVGDTITLTCHASQNIDVWLSWFQQK PGNIPKLLIYKASNLHTGVPSRFSGSGSGTGFTLTISSLQPED IATYYCQQAHSYPFTFGGGTKLEIKRTVAAPSVFIFPPSDEQ LKSGTASVVCLLNNFYPREAKVQWKVDNALQSGNSQESVT EQDSKDSTYSLSSTLTLSKADYEKHKVYACEVTHQGLSSPVT KSFNRGECRRKRGSHHHHHHHHGGGGSRRKRDIVLTQSP ASLAVSLGQRATISCRASESVDSYGQSYMHWYQQKAGQ PPKLLIYLASNLESGVPARFSGSGSRTDFTLTIDPVQAEDAA TYYCQQNAEDSRTFGGGTKLEIKRTVAAPSVFIFPPSDEQLK SGTASVVCLLNNFYPREAKVQWKVDNALQSGNSQESVTEQ DSKDSTYSLSSTLTLSKADYEKHKVYACEVTHQGLSSPVTKSF NRGECGGGGSDKTHTCPPCPAPELLGGPSVFLFPPKPKDTL MISRTPEVTCVVVDVSHEDPEVKFNWYVDGVEVHNAKTKP REEQYNSTYRVVSVLTVLHQDWLNGKEYKCKVSNKALPAPI EKTISKAKGQPREPQVCTLPPSRDELTKNQVSLSCAVKGFYP SDIAVEWESNGQPENNYKTTPPVLDSDGSFFLVSKLTVDKS RWQQGNVFSCSVMHEALHNRFTQKSLSLSPG | **anti-IL4-VL**-huIGKC-*[PCS1]*-<GS>-His(8)-<G4S>-*[PCS1]-***anti-IL13-VL**-huIGKC-<G4S>*-Fc-huIgG1(hole-RF)* |
| | 26 | QVQLQQSGPELVKPGASVKISCKASGYSFTSYWIHWIKQR PGQGLEWIGMIDPSDGETRLNQRFQGRATLTVDESTSTA YMQLRSPTSEDSAVYYCTRLKEYGNYDSFYFDVWGAGTL VTVSSASTKGPSVFPLAPSSKSTSGGTAALGCLVKDYFPEPV TVSWNSGALTSGVHTFPAVLQSSGLYSLSSVVTVPSSSLGTQ TYICNVNHKPSNTKVDKKVEPKSCDKTHTGGGGSGGGGSG GGGSEVQLKESGPGLVAPGGSLSITCTVSGFSLTDSSINWV RQPPGKGLEWLGMIWGDGRIDYADALKSRLSISKDSSKS QVFLEMTSLRTDDTATYYCARDGYFPYAMDFWGQGTSV TVSSASTKGPSVFPLAPSSKSTSGGTAALGCLVKDYFPEPVT VSWNSGALTSGVHTFPAVLQSSGLYSLSSVVTVPSSSLGTQT YICNVNHKPSNTKVDKKVEPKSCDKTHTCPPCPAPELLGGP SVFLFPPKPKDTLMISRTPEVTCVVVDVSHEDPEVKFNWYV DGVEVHNAKTKPREEQYNSTYRVVSVLTVLHQDWLNGKEY KCKVSNKALPAPIEKTISKAKGQPREPQVYTLPPCRDELTKN QVSLWCLVKGFYPSDIAVEWESNGQPENNYKTTPPVLDSD GSFFLYSKLTVDKSRWQQGNVFSCSVMHEALHNHYTQKSL SLSPG | **anti-IL4-VH**-huIGHG11-huIGH-H1-<(G4S)3>-**anti-IL13-VH**-huIGHG1(knob) |
| scTandem-(anti-IL4xanti-IL13)-His8-ΔPCL1-huIgG1-OL-KIH | 30 | DIQMTQSPASLSVSVGDTITLTCHASQNIDVWLSWFQQK PGNIPKLLIYKASNLHTGVPSRFSGSGSGTGFTLTISSLQPED IATYYCQQAHSYPFTFGGGTKLEIKRTVAAPSVFIFPPSDEQ LKSGTASVVCLLNNFYPREAKVQWKVDNALQSGNSQESVT EQDSKDSTYSLSSTLTLSKADYEKHKVYACEVTHQGLSSPVT KSFNRGECRQQRGSHHHHHHHHGGGGSRQQRDIVLTQS PASLAVSLGQRATISCRASESVDSYGQSYMHWYQQKAG QPPKLLIYLASNLESGVPARFSGSGSRTDFTLTIDPVQAEDA ATYYCQQNAEDSRTFGGGTKLEIKRTVAAPSVFIFPPSDEQ LKSGTASVVCLLNNFYPREAKVQWKVDNALQSGNSQESVT EQDSKDSTYSLSSTLTLSKADYEKHKVYACEVTHQGLSSPVT KSFNRGECGGGGSDKTHTCPPCPAPELLGGPSVFLFPPKPK DTLMISRTPEVTCVVVDVSHEDPEVKFNWYVDGVEVHNAK TKPREEQYNSTYRVVSVLTVLHQDWLNGKEYKCKVSNKALP APIEKTISKAKGQPREPQVCTLPPSRDELTKNQVSLSCAVKG FYPSDIAVEWESNGQPENNYKTTPPVLDSDGSFFLVSKLTV DKSRWQQGNVFSCSVMHEALHNRFTQKSLSLSPG | **anti-IL4-VL-huIGKC-***[deltaPCS]*-<GS>-His(8)-<G4S>-*[deltaPCS]*-**anti-IL13-VL**-huIGKC-<G4S>-*Fc-huIgG1(hole-RF)* |
| | 26 | QVQLQQSGPELVKPGASVKISCKASGYSFTSYWIHWIKQR PGQGLEWIGMIDPSDGETRLNQRFQGRATLTVDESTSTA YMQLRSPTSEDSAVYYCTRLKEYGNYDSFYFDVWGAGTL VTVSSASTKGPSVFPLAPSSKSTSGGTAALGCLVKDYFPEPV TVSWNSGALTSGVHTFPAVLQSSGLYSLSSVVTVPSSSLGTQ TYICNVNHKPSNTKVDKKVEPKSCDKTHTGGGGSGGGGSG GGGSEVQLKESGPGLVAPGGSLSITCTVSGFSLTDSSINWV RQPPGKGLEWLGMIWGDGRIDYADALKSRLSISKDSSKS QVFLEMTSLRTDDTATYYCARDGYFPYAMDFWGQGTSV TVSSASTKGPSVFPLAPSSKSTSGGTAALGCLVKDYFPEPVT VSWNSGALTSGVHTFPAVLQSSGLYSLSSVVTVPSSSLGTQT YICNVNHKPSNTKVDKKVEPKSCDKTHTCPPCPAPELLGGP SVFLFPPKPKDTLMISRTPEVTCVVVDVSHEDPEVKFNWYV DGVEVHNAKTKPREEQYNSTYRVVSVLTVLHQDWLNGKEY KCKVSNKALPAPIEKTISKAKGQPREPQVYTLPPCRDELTKN QVSLWCLVKGFYPSDIAVEWESNGQPENNYKTTPPVLDSD GSFFLYSKLTVDKSRWQQGNVFSCSVMHEALHNHYTQKSL SLSPG | **anti-IL4-VH**-huIGHG11-huIGH-H1-<(G4S)3>-**anti-IL13-VH**-huIGHG1(knob) |
| scTandem-(anti-IL4xanti-IL13)-PCL2-huIgG1-OL-KIH | 31 | DIQMTQSPASLSVSVGDTITLTCHASQNIDVWLSWFQQK PGNIPKLLIYKASNLHTGVPSRFSGSGSGTGFTLTISSLQPED IATYYCQQAHSYPFTFGGGTKLEIKRTVAAPSVFIFPPSDEQ LKSGTASVVCLLNNFYPREAKVQWKVDNALQSGNSQESVT EQDSKDSTYSLSSTLTLSKADYEKHKVYACEVTHQGLSSPVT KSFNRGECHRRRKRSVDEGGGGSHRRRKRSVDEDIVLTQS PASLAVSLGQRATISCRASESVDSYGQSYMHWYQQKAG QPPKLLIYLASNLESGVPARFSGSGSRTDFTLTIDPVQAEDA ATYYCQQNAEDSRTFGGGTKLEIKRTVAAPSVFIFPPSDEQ LKSGTASVVCLLNNFYPREAKVQWKVDNALQSGNSQESVT EQDSKDSTYSLSSTLTLSKADYEKHKVYACEVTHQGLSSPVT KSFNRGECGGGGSDKTHTCPPCPAPELLGGPSVFLFPPKPK DTLMISRTPEVTCVVVDVSHEDPEVKFNWYVDGVEVHNAK TKPREEQYNSTYRVVSVLTVLHQDWLNGKEYKCKVSNKALP APIEKTISKAKGQPREPQVCTLPPSRDELTKNQVSLSCAVKG FYPSDIAVEWESNGQPENNYKTTPPVLDSDGSFFLVSKLTV DKSRWQQGNVFSCSVMHEALHNRFTQKSLSLSPG | **anti-IL4-VL**-huIGKC-*[PCS2]*-<(G4S)3>-*[PCS2]***-anti-IL13-VL-**huIGKC-<G4S>*-Fc-huIgG1(hole-RF)* |
| | 26 | QVQLQQSGPELVKPGASVKISCKASGYSFTSYWIHWIKQR PGQGLEWIGMIDPSDGETRLNQRFQGRATLTVDESTSTA YMQLRSPTSEDSAVYYCTRLKEYGNYDSFYFDVWGAGTL VTVSSASTKGPSVFPLAPSSKSTSGGTAALGCLVKDYFPEPV TVSWNSGALTSGVHTFPAVLQSSGLYSLSSVVTVPSSSLGTQ TYICNVNHKPSNTKVDKKVEPKSCDKTHTGGGGSGGGGSG GGGSEVQLKESGPGLVAPGGSLSITCTVSGFSLTDSSINWV RQPPGKGLEWLGMIWGDGRIDYADALKSRLSISKDSSKS QVFLEMTSLRTDDTATYYCARDGYFPYAMDFWGQGTSV TVSSASTKGPSVFPLAPSSKSTSGGTAALGCLVKDYFPEPVT VSWNSGALTSGVHTFPAVLQSSGLYSLSSVVTVPSSSLGTQT YICNVNHKPSNTKVDKKVEPKSCDKTHTCPPCPAPELLGGP SVFLFPPKPKDTLMISRTPEVTCVVVDVSHEDPEVKFNWYV DGVEVHNAKTKPREEQYNSTYRVVSVLTVLHQDWLNGKEY KCKVSNKALPAPIEKTISKAKGQPREPQVYTLPPCRDELTKN QVSLWCLVKGFYPSDIAVEWESNGQPENNYKTTPPVLDSD GSFFLYSKLTVDKSRWQQGNVFSCSVMHEALHNHYTQKSL SLSPG | **anti-IL4-VH**-huIGHG11-huIGH-H1-<(G4S)3>-**anti-IL13-VH**-huIGHG1(knob) |
| scTandem-(anti-IL4xanti-IL13)-PCL3-huIgG1-OL-KIH | 32 | DIQMTQSPASLSVSVGDTITLTCHASQNIDVWLSWFQQK PGNIPKLLIYKASNLHTGVPSRFSGSGSGTGFTLTISSLQPED IATYYCQQAHSYPFTFGGGTKLEIKRTVAAPSVFIFPPSDEQ LKSGTASVVCLLNNFYPREAKVQWKVDNALQSGNSQESVT EQDSKDSTYSLSSTLTLSKADYEKHKVYACEVTHQGLSSPVT KSFNRGECPMKKRRAGVPGGGGSPMKKRRAGVPDIVLT QSPASLAVSLGQRATISCRASESVDSYGQSYMHWYQQKA GQPPKLLIYLASNLESGVPARFSGSGSRTDFTLTIDPVQAED AATYYCQQNAEDSRTFGGGTKLEIKRTVAAPSVFIFPPSDE QLKSGTASVVCLLNNFYPREAKVQWKVDNALQSGNSQESV TEQDSKDSTYSLSSTLTLSKADYEKHKVYACEVTHQGLSSPV TKSFNRGECGGGGSDKTHTCPPCPAPELLGGPSVFLFPPKP KDTLMISRTPEVTCVVVDVSHEDPEVKFNWYVDGVEVHNA KTKPREEQYNSTYRVVSVLTVLHQDWLNGKEYKCKVSNKAL PAPIEKTISKAKGQPREPQVCTLPPSRDELTKNQVSLSCAVK GFYPSDIAVEWESNGQPENNYKTTPPVLDSDGSFFLVSKLT VDKSRWQQGNVFSCSVMHEALHNRFTQKSLSLSPG | **anti-IL4-VL**-huIGKC-*[PCS3]*-<(G4S)3>-*[PCS3]*-**anti-IL13-VL-**huIGKC-<G4S>*-Fc-huIgG1(hole-RF)* |
| | 26 | QVQLQQSGPELVKPGASVKISCKASGYSFTSYWIHWIKQR PGQGLEWIGMIDPSDGETRLNQRFQGRATLTVDESTSTA YMQLRSPTSEDSAVYYCTRLKEYGNYDSFYFDVWGAGTL VTVSSASTKGPSVFPLAPSSKSTSGGTAALGCLVKDYFPEPV TVSWNSGALTSGVHTFPAVLQSSGLYSLSSVVTVPSSSLGTQ TYICNVNHKPSNTKVDKKVEPKSCDKTHTGGGGSGGGGSG GGGSEVQLKESGPGLVAPGGSLSITCTVSGFSLTDSSINWV RQPPGKGLEWLGMIWGDGRIDYADALKSRLSISKDSSKS QVFLEMTSLRTDDTATYYCARDGYFPYAMDFWGQGTSV TVSSASTKGPSVFPLAPSSKSTSGGTAALGCLVKDYFPEPVT VSWNSGALTSGVHTFPAVLQSSGLYSLSSVVTVPSSSLGTQT YICNVNHKPSNTKVDKKVEPKSCDKTHTCPPCPAPELLGGP SVFLFPPKPKDTLMISRTPEVTCVVVDVSHEDPEVKFNWYV DGVEVHNAKTKPREEQYNSTYRVVSVLTVLHQDWLNGKEY KCKVSNKALPAPIEKTISKAKGQPREPQVYTLPPCRDELTKN QVSLWCLVKGFYPSDIAVEWESNGQPENNYKTTPPVLDSD GSFFLYSKLTVDKSRWQQGNVFSCSVMHEALHNHYTQKSL SLSPG | **anti-IL4-VH**-huIGHG11-huIGH-H1-<(G4S)3>-**anti-IL13-VH**-huIGHG1(knob) |
| scTandem-(anti-IL4xanti-IL13)-rigidL-PCL1-huIgG1-OL-KIH | 33 | DIQMTQSPASLSVSVGDTITLTCHASQNIDVWLSWFQQK PGNIPKLLIYKASNLHTGVPSRFSGSGSGTGFTLTISSLQPED IATYYCQQAHSYPFTFGGGTKLEIKRTVAAPSVFIFPPSDEQ LKSGTASVVCLLNNFYPREAKVQWKVDNALQSGNSQESVT EQDSKDSTYSLSSTLTLSKADYEKHKVYACEVTHQGLSSPVT KSFNRGECRRKRGGGGSEAAAKEAAAKGGGGSRRKRDIVL TQSPASLAVSLGQRATISCRASESVDSYGQSYMHWYQQK AGQPPKLLIYLASNLESGVPARFSGSGSRTDFTLTIDPVQAE DAATYYCQQNAEDSRTFGGGTKLEIKRTVAAPSVFIFPPSD EQLKSGTASVVCLLNNFYPREAKVQWKVDNALQSGNSQES VTEQDSKDSTYSLSSTLTLSKADYEKHKVYACEVTHQGLSSP VTKSFNRGECGGGGSDKTHTCPPCPAPELLGGPSVFLFPPK PKDTLMISRTPEVTCVVVDVSHEDPEVKFNWYVDGVEVHN AKTKPREEQYNSTYRVVSVLTVLHQDWLNGKEYKCKVSNK ALPAPIEKTISKAKGQPREPQVCTLPPSRDELTKNQVSLSCAV KGFYPSDIAVEWESNGQPENNYKTTPPVLDSDGSFFLVSKLT VDKSRWQQGNVFSCSVMHEALHNRFTQKSLSLSPG | **anti-IL4-VL-huIGKC-***[PCS1]-<G4S>-*<(EAAAK)2>-<G4S>-*[PCS1]*-**anti-IL13-VL-**huIGKC-<G4S>-*Fc-huIgG1(hole-RF)* |
| | 34 | QVQLQQSGPELVKPGASVKISCKASGYSFTSYWIHWIKQR PGQGLEWIGMIDPSDGETRLNQRFQGRATLTVDESTSTA YMQLRSPTSEDSAVYYCTRLKEYGNYDSFYFDVWGAGTL VTVSSASTKGPSVFPLAPSSKSTSGGTAALGCLVKDYFPEPV TVSWNSGALTSGVHTFPAVLQSSGLYSLSSVVTVPSSSLGTQ TYICNVNHKPSNTKVDKKVEPKSCDKTHTGGGGSEAAAKE AAAKGGGGSEVQLKESGPGLVAPGGSLSITCTVSGFSLTDS SINWVRQPPGKGLEWLGMIWGDGRIDYADALKSRLSISK DSSKSQVFLEMTSLRTDDTATYYCARDGYFPYAMDFWG QGTSVTVSSASTKGPSVFPLAPSSKSTSGGTAALGCLVKDYF PEPVTVSWNSGALTSGVHTFPAVLQSSGLYSLSSVVTVPSSS LGTQTYICNVNHKPSNTKVDKKVEPKSCDKTHTCPPCPAPE LLGGPSVFLFPPKPKDTLMISRTPEVTCVVVDVSHEDPEVKF NWYVDGVEVHNAKTKPREEQYNSTYRVVSVLTVLHQDWL NGKEYKCKVSNKALPAPIEKTISKAKGQPREPQVYTLPPCRD ELTKNQVSLWCLVKGFYPSDIAVEWESNGQPENNYKTTPP VLDSDGSFFLYSKLTVDKSRWQQGNVFSCSVMHEALHNHY TQKSLSLSPG | **anti-IL4-VH**-huIGH-G11-huIGH-H1-<G4S>-<(EAAAK)2>-<G4S>-*anti-IL13-VH-*huIGHG1(knob) |
| scTandem-(anti-IL4xanti-IL13)-rigidL2-PCL1-huIgG1-OL-KIH | 35 | DIQMTQSPASLSVSVGDTITLTCHASQNIDVWLSWFQQK PGNIPKLLIYKASNLHTGVPSRFSGSGSGTGFTLTISSLQPED IATYYCQQAHSYPFTFGGGTKLEIKRTVAAPSVFIFPPSDEQ LKSGTASVVCLLNNFYPREAKVQWKVDNALQSGNSQESVT EQDSKDSTYSLSSTLTLSKADYEKHKVYACEVTHQGLSSPVT KSFNRGECRRKRAPAPAPAPAPAPAPAPARRKRDIVLTQSPAS LAVSLGQRATISCRASESVDSYGQSYMHWYQQKAGQPP KLLIYLASNLESGVPARFSGSGSRTDFTLTIDPVQAEDAATY YCQQNAEDSRTFGGGTKLEIKRTVAAPSVFIFPPSDEQLKS GTASVVCLLNNFYPREAKVQWKVDNALQSGNSQESVTEQ DSKDSTYSLSSTLTLSKADYEKHKVYACEVTHQGLSSPVTKSF NRGECGGGGSDKTHTCPPCPAPELLGGPSVFLFPPKPKDTL MISRTPEVTCVVVDVSHEDPEVKFNWYVDGVEVHNAKTKP REEQYNSTYRVVSVLTVLHQDWLNGKEYKCKVSNKALPAPI EKTISKAKGQPREPQVCTLPPSRDELTKNQVSLSCAVKGFYP SDIAVEWESNGQPENNYKTTPPVLDSDGSFFLVSKLTVDKS RWQQGNVFSCSVMHEALHNRFTQKSLSLSPG | **anti-IL4-VL**-huIGKC-*[PCS1]*-<(AP)7A>-*[PCS1]**-***anti-IL13-VL-**huIGKC-<G4S>*-Fc-huIgG1(hole-RF)* |
| | 26 | QVQLQQSGPELVKPGASVKISCKASGYSFTSYWIHWIKQR PGQGLEWIGMIDPSDGETRLNQRFQGRATLTVDESTSTA YMQLRSPTSEDSAVYYCTRLKEYGNYDSFYFDVWGAGTL VTVSSASTKGPSVFPLAPSSKSTSGGTAALGCLVKDYFPEPV TVSWNSGALTSGVHTFPAVLQSSGLYSLSSVVTVPSSSLGTQ TYICNVNHKPSNTKVDKKVEPKSCDKTHTGGGGSGGGGSG GGGSEVQLKESGPGLVAPGGSLSITCTVSGFSLTDSSINWV RQPPGKGLEWLGMIWGDGRIDYADALKSRLSISKDSSKS QVFLEMTSLRTDDTATYYCARDGYFPYAMDFWGQGTSV TVSSASTKGPSVFPLAPSSKSTSGGTAALGCLVKDYFPEPVT VSWNSGALTSGVHTFPAVLQSSGLYSLSSVVTVPSSSLGTQT YICNVNHKPSNTKVDKKVEPKSCDKTHTCPPCPAPELLGGP SVFLFPPKPKDTLMISRTPEVTCVVVDVSHEDPEVKFNWYV DGVEVHNAKTKPREEQYNSTYRVVSVLTVLHQDWLNGKEY KCKVSNKALPAPIEKTISKAKGQPREPQVYTLPPCRDELTKN QVSLWCLVKGFYPSDIAVEWESNGQPENNYKTTPPVLDSD GSFFLYSKLTVDKSRWQQGNVFSCSVMHEALHNHYTQKSL SLSPG | **anti-IL4-VH**-huIGHG11-huIGH-H1-<(G4S)3>-anti-IL13-**VH**-huIGHG1(knob) |
| scTandem-(anti-IL4xanti-IL13)-charL-PCL1-huIgG1-OL-KIH | 36 | DIQMTQSPASLSVSVGDTITLTCHASQNIDVWLSWFQQK PGNIPKLLIYKASNLHTGVPSRFSGSGSGTGFTLTISSLQPED IATYYCQQAHSYPFTFGGGTKLEIKRTVAAPSVFIFPPSDEQ LKSGTASVVCLLNNFYPREAKVQWKVDNALQSGNSQESVT EQDSKDSTYSLSSTLTLSKADYEKHKVYACEVTHQGLSSPVT KSFNRGECRRKRGSKQGKQKQGKQGSRRKRDIVLTQSPAS LAVSLGQRATISCRASESVDSYGQSYMHWYQQKAGQPP KLLIYLASNLESGVPARFSGSGSRTDFTLTIDPVQAEDAATY YCQQNAEDSRTFGGGTKLEIKRTVAAPSVFIFPPSDEQLKS GTASVVCLLNNFYPREAKVQWKVDNALQSGNSQESVTEQ DSKDSTYSLSSTLTLSKADYEKHKVYACEVTHQGLSSPVTKSF NRGECGGGGSDKTHTCPPCPAPELLGGPSVFLFPPKPKDTL MISRTPEVTCVVVDVSHEDPEVKFNWYVDGVEVHNAKTKP REEQYNSTYRVVSVLTVLHQDWLNGKEYKCKVSNKALPAPI EKTISKAKGQPREPQVCTLPPSRDELTKNQVSLSCAVKGFYP SDIAVEWESNGQPENNYKTTPPVLDSDGSFFLVSKLTVDKS RWQQGNVFSCSVMHEALHNRFTQKSLSLSPG | **anti-IL4-VL**-huIGKC-[*PCS1*]*-*<GS>-<(KQGKQ)2>-<GS>-*[PCS1]*-**anti-IL13-VL-**huIGKC-<G4S>-*Fc-huIgG1(hole-RF)* |
| | 26 | QVQLQQSGPELVKPGASVKISCKASGYSFTSYWIHWIKQR PGQGLEWIGMIDPSDGETRLNQRFQGRATLTVDESTSTA YMQLRSPTSEDSAVYYCTRLKEYGNYDSFYFDVWGAGTL VTVSSASTKGPSVFPLAPSSKSTSGGTAALGCLVKDYFPEPV TVSWNSGALTSGVHTFPAVLQSSGLYSLSSVVTVPSSSLGTQ TYICNVNHKPSNTKVDKKVEPKSCDKTHTGGGGSGGGGSG GGGSEVQLKESGPGLVAPGGSLSITCTVSGFSLTDSSINWV RQPPGKGLEWLGMIWGDGRIDYADALKSRLSISKDSSKS QVFLEMTSLRTDDTATYYCARDGYFPYAMDFWGQGTSV TVSSASTKGPSVFPLAPSSKSTSGGTAALGCLVKDYFPEPVT VSWNSGALTSGVHTFPAVLQSSGLYSLSSVVTVPSSSLGTQT YICNVNHKPSNTKVDKKVEPKSCDKTHTCPPCPAPELLGGP SVFLFPPKPKDTLMISRTPEVTCVVVDVSHEDPEVKFNWYV DGVEVHNAKTKPREEQYNSTYRVVSVLTVLHQDWLNGKEY KCKVSNKALPAPIEKTISKAKGQPREPQVYTLPPCRDELTKN QVSLWCLVKGFYPSDIAVEWESNGQPENNYKTTPPVLDSD GSFFLYSKLTVDKSRWQQGNVFSCSVMHEALHNHYTQKSL SLSPG | **anti-IL4-VH**-huIGHG11-huIGH-H1-<(G4S)3>-**anti-IL13-VH**-huIGHG1(knob) |
| hFurin | 37 | QKVFTNTWAVRIPGGPAVANSVARKHGFLNLGQIFGDYYH FWHRGVTKRSLSPHRPRHSRLQREPQVQWLEQQVAKRRT KRDVYQEPTDPKFPQQWYLSGVTQRDLNVKAAWAQGYT GHGIVVSILDDGIEKNHPDLAGNYDPGASFDVNDQDPDPQ PRYTQMNDNRHGTRCAGEVAAVANNGVCGVGVAYNARI GGVRMLDGEVTDAVEARSLGLNPNHIHIYSASWGPEDDGK TVDGPARLAEEAFFRGVSQGRGGLGSIFVWASGNGGREH DSCNCDGYTNSIYTLSISSATQFGNVPWYSEACSSTLATTYSS GNQNEKQIVTTDLRQKCTESHTGTSASAPLAAGIIALTLEAN KNLTWRDMQHLVVQTSKPAHLNANDWATNGVGRKVSHS YGYGLLDAGAMVALAQNWTTVAPQRKCIIDILTEPKDIGKR LEVRKTVTACLGEPNHITRLEHAQARLTLSYNRRGDLAIHLV SPMGTRSTLLAARPHDYSADGFNDWAFMTTHSWDEDPS GEWVLEIENTSEANNYGTLTKFTLVLYGTAPEGLPVPPESSG CKTLTSSQACVVCEEGFSLHQKSCVQHCPPGFAPQVLDTHY STENDVETIRASVCAPCHASCATCQGPALTDCLSCPSHASLD PVEQTCSRQSQSSRESPPQQQPPRLPPEVEAGQRLRAGLLP | Propeptide Furin-mature Furin |
| | | SHLPEVVAGLSCAFIVLVFVTVFLVLQLRSGFSFRGVKVYTM DRGLISYKGLPPEAWQEECPSDSEEDEGRGERTAFIKDQSAL | |
| hFurin-KDEL | 38 | QKVFTNTWAVRIPGGPAVANSVARKHGFLNLGQIFGDYYH FWHRGVTKRSLSPHRPRHSRLQREPQVQWLEQQVAKRRT KRDVYQEPTDPKFPQQWYLSGVTQRDLNVKAAWAQGYT GHGIVVSILDDGIEKNHPDLAGNYDPGASFDVNDQDPDPQ PRYTQMNDNRHGTRCAGEVAAVANNGVCGVGVAYNARI GGVRMLDGEVTDAVEARSLGLNPNHIHIYSASWGPEDDGK TVDGPARLAEEAFFRGVSQGRGGLGSIFVWASGNGGREH DSCNCDGYTNSIYTLSISSATQFGNVPWYSEACSSTLATTYSS GNQNEKQIVTTDLRQKCTESHTGTSASAPLAAGIIALTLEAN KNLTWRDMQHLVVQTSKPAHLNANDWATNGVGRKVSHS YGYGLLDAGAMVALAQNWTTVAPQRKCIIDILTEPKDIGKR LEVRKTVTACLGEPNHITRLEHAQARLTLSYNRRGDLAIHLV SPMGTRSTLLAARPHDYSADGFNDWAFMTTHSWDEDPS GEWVLEIENTSEANNYGTLTKFTLVLYGTAPEGLPVPPESSG CKTLTSSQACVVCEEGFSLHQKSCVQHCPPGFAPQVLDTHY STENDVETIRASVCAPCHASCATCQGPALTDCLSCPSHASLD PVEQTCSRQSQSSRESPPQQQPPRLPPEVEAGQRLRAGLLP SHLPEVVAGLSCAFIVLVFVTVFLVLQLRSGFSFRGVKVYTM DRGLISYKGLPPEAWQEECPSDSEEDEGRGERTAFIKDQSAL GAAKDEL | Propeptide Furin-mature Furin-<GAA>-**KDEL signal peptide** |
| hPC5/6 | 39 | RVYTNHWAVKIAGGFPEANRIASKYGFINIGQIGALKDYYHF YHSRTIKRSVISSRGTHSFISMEPKVEWIQQQVVKKRTKRDY DFSRAQSTYFNDPKWPSMWYMHCSDNTHPCQSDMNIEG AWKRGYTGKNIVVTILDDGIERTHPDLMQNYDALASCDVN GNDLDPMPRYDASNENKHGTRCAGEVAAAANNSHCTVGI AFNAKIGGVRMLDGDVTDMVEAKSVSFNPQHVHIYSASW GPDDDGKTVDGPAPLTRQAFENGVRMGRRGLGSVFVWA SGNGGRSKDHCSCDGYTNSIYTISISSTAESGKKPWYLEECSS TLATTYSSGESYDKKIITTDLRQRCTDNHTGTSASAPMAAGII ALALEANPFLTWRDVQHVIVRTSRAGHLNANDWKTNAAG FKVSHLYGFGLMDAEAMVMEAEKWTTVPRQHVCVESTDR QIKTIRPNSAVRSIYKASGCSDNPNRHVNYLEHVVVRITITHP RRGDLAIYLTSPSGTRSQLLANRLFDHSMEGFKNWEFMTIH CWGERAAGDWVLEVYDTPSQLRNFKTPGKLKEWSLVLYGT SVQPYSPTNEFPKVERFRYSRVEDPTDDYGTEDYAGPCDPE CSEVGCDGPGPDHCNDCLHYYYKLKNNTRICVSSCPPGHYH ADKKRCRKCAPNCESCFGSHGDQCMSCKYGYFLNEETNSC VTHCPDGSYQDTKKNLCRKCSENCKTCTEFHNCTECRDGLS LQGSRCSVSCEDGRYFNGQDCQPCHRFCATCAGAGADGCI NCTEGYFMEDGRCVQSCSISYYFDHSSENGYKSCKKCDISCL TCNGPGFKNCTSCPSGYLLDLGMCQMGAICKDGEYVDEH GHCQTCEASCAKCQGPTQEDCTTCPMTRIFDDGRCVSNCP SWKFEFENQCHPCHHTCQRCQGSGPTHCTSCGADNYGRE HFLYQGECGDSCPEGHYATEGNTCLPCPDNCELCHSVHVC TRCMKGYFIAPTNHTCQKLECGQGEVQDPDYEECVPCEEG CLGCSLDDPGTCTSCAMGYYRFDHHCYKTCPEKTYSEEVEC KACDSNCGSCDQNGCYWCEEGFFLLGGSCVRKCGPGFYG DQEMGECESCHRACETCTGPGHDECSSCQEGLQLLRGMC VHATKTQEEGKFWNDILRKLQPCHSSCKTCNGSATLCTSCP KGAYLLAQACVSSCPQGTWPSVRSGSCENCTEACAICSGAD LCKKCQMQPGHPLFLHEGRCYSKCPEGSYAEDGICERCSSP CRTCEGNATNCHSCEGGHVLHHGVCQENCPERHVAVKGV CKHCPEMCQDCIHEKTCKECTPEFFLHDDMCHQSCPRGFY ADSRHCVPCHKDCLECSGPKADDCELCLESSWVLYDGLCLE ECPAGTYYEKETKECRDCHKSCLTCSSSGTCTTCQKGLIMNP RGSCMANEKCSPSEYWDEDAPGCKPCHVKCFHCMGPAED QCQTCPMNSLLLNTTCVKDCPEGYYADEDSNRCAHCHSSC RTCEGRHSRQCHSCRPGWFQLGKECLLQCREGYYADNSTG RCERCNRSCKGCQGPRPTDCLSCDRFFFLLRSKGECHRSCP DHYYVEQSTQTCERCHPTCDQCKGKGALNCLSCVWSYHL MGGICTSDCLVGEYRVGEGEKFNCEKCHESCMECKGPGAK NCTLCPANLVLHMDDSHCLHCCNTSDPPSAQECCDCQDTT DECILRTSKVRPATEHFKTALFITSSMMLVLLLGAAVVVWKK SRGRVQPAAKAGYEKLADPNKSYSSYKSSYRESTSFEEDQVI EYRDRDYDEDDDDDIVYMGQDGTVYRKFKYGLLDDDDIDE LEYDDESYSYYQ | Propeptide PCSK5-mature PCSK5 |
| hPC7 | 40 | LAGTGGPDGQGTGGPSWAVHLESLEGDGEEETLEQQADA LAQAAGLVNAGRIGELQGHYLFVQPAGHRPALEVEAIRQQ VEAVLAGHEAVRWHSEQRLLRRAKRSVHFNDPKYPQQWH LNNRRSPGRDINVTGVWERNVTGRGVTVVVVDDGVEHTI QDIAPNYSPEGSYDLNSNDPDPMPHPDVENGNHHGTRCA GEIAAVPNNSFCAVGVAYGSRIAGIRVLDGPLTDSMEAVAF NKHYQINDIYSCSWGPDDDGKTVDGPHQLGKAALQHGVI AGRQGFGSIFVVASGNGGQHNDNCNYDGYANSIYTVTIGA VDEEGRMPFYAEECASMLAVTFSGGDKMLRSIVTTDWDL QKGTGCTEGHTGTSAAAPLAAGMIALMLQVRPCLTWRDV QHIIVFTATRYEDRRAEWVTNEAGFSHSHQHGFGLLNAWR LVNAAKIWTSVPYLASYVSPVLKENKAIPQSPRSLEVLWNVS RMDLEMSGLKTLEHVAVTVSITHPRRGSLELKLFCPSGMMS LIGAPRSMDSDPNGFNDWTFSTVRCWGERARGTYRLVIRD VGDESFQVGILRQWQLTLYGSVWSAVDIRDRQRLLESAMS GKYLHDDFALPCPPGLKIPEEDGYTITPNTLKTLVLVGCFTVF WTVYYMLEVYLSQRNVASNQVCRSGPCHWPHRSRKAKEE GTELESVPLCSSKDPDEVETESRGPPTTSDLLAPDLLEQGDW SLSQNKSALDCPHQHLDVPHGKEEQIC | Propeptide PCSK7-mature PCSK7 |
| hPACE4 | 41 | PPPRPVYTNHWAVQVLGGPAEADRVAAAHGYLNLGQIGN LEDYYHFYHSKTFKRSTLSSRGPHTFLRMDPQVKWLQQQE VKRRVKRQVRSDPQALYFNDPIWSNMWYLHCGDKNSRCR SEMNVQAAWKRGYTGKNVVVTILDDGIERNHPDLAPNYD SYASYDVNGNDYDPSPRYDASNENKHGTRCAGEVAASAN NSYCIVGIAYNAKIGGIRMLDGDVTDVVEAKSLGIRPNYIDIY SASWGPDDDGKTVDGPGRLAKQAFEYGIKKGRQGLGSIFV WASGNGGREGDYCSCDGYTNSIYTISVSSATENGYKPWYLE ECASTLATTYSSGAFYERKIVTTDLRQRCTDGHTGTSVSAPM VAGIIALALEANSQLTWRDVQHLLVKTSRPAHLKASDWKV NGAGHKVSHFYGFGLVDAEALVVEAKKWTAVPSQHMCVA ASDKRPRSIPLVQVLRTTALTSACAEHSDQRVVYLEHVVVRT SISHPRRGDLQIYLVSPSGTKSQLLAKRLLDLSNEGFTNWEF MTVHCWGEKAEGQWTLEIQDLPSQVRNPEKQGKLKEWSL ILYGTAEHPYHTFSAHQSRSRMLELSAPELEPPKAALSPSQV EVPEDEEDYTAQSTPGSANILQTSVCHPECGDKGCDGPNA DQCLNCVHFSLGSVKTSRKCVSVCPLGYFGDTAARRCRRCH KGCETCSSRAATQCLSCRRGFYHHQEMNTCVTLCPAGFYA DESQKNCLKCHPSCKKCVDEPEKCTVCKEGFSLARGSCIPDC EPGTYFDSELIRCGECHHTCGTCVGPGREECIHCAKNFHFH DWKCVPACGEGFYPEEMPGLPHKVCRRCDENCLSCAGSSR NCSRCKTGFTQLGTSCITNHTCSNADETFCEMVKSNRLCER KLFIQFCCRTCLLAG | Propeptide PCSK6-mature PCSK6 |
| PCS General Formula | 42 | HRRX₁X₂RSVDE | |
| PCS2 | 43 | HRRRKRSVDE | |
| PCS2a | 44 | HRRQQRSVDE | |
| Linker | 45 | GSHHHHHHHHGGGGS | |
| Linker | 46 | GGGGSEAAAKEAAAKGGGGS | |
| Linker | 47 | APAPAPAPAPAPAPA | |
| Linker | 48 | GSKQGKQKQGKQGS | |
| PCS1 | 49 | RRKR | |
| PCS3 | 50 | PMKKRRAGVP | |
| Linker | 51 | (G₄S)n | n = 1-10 |
| Linker | 52 | (EAAAK)ₙ | n = 1-10 |
| Linker | 53 | (AP)ₙA | n = 1-10 |
| Linker | 54 | (KQGKQ)ₙ | n = 1-10 |
| Linker | 55 | GGGGSGGGGSGGGGS | |
| TRI-(CODV-anti-PD-1 x anti-OX40) x anti-GITR-huIgG1-LALA-KIH-RF | 56 | DIQMTQSPSSLSASVGDRVTITCRASQDISNYLNWYQQKP GKAPKLLIYYTSRLRSGVPSRFSGSGSGTDFTLTISSLQPEDF ATYYCQQGHTLPPTFGQGTKVEIKGGGGSGGGGSEIVLTQ SPATLSLSPGERATLSCRASQSVSSYLAWYQQKPGQAPRLL IYDASNRATGIPARFSGSGSGTDFTLTISSLEPEDFAVYYCQ QSSNWPRTFGQGTKVEIKGGGGSGGGGSRTVAAPSVFIFP PSDEQLKSGTASVVCLLNNFYPREAKVQWKVDNALQSGNS QESVTEQDSKDSTYSLSSTLTLSKADYEKHKVYACEVTHQGL SSPVTKSFNRGEC | **anti-OX40-VL-**<(G4S)2>-***anti-PD1-VL***-<(G4S)2>-huIGKC |
| | 57 | QVQLVESGGGVVQPGRSLRLDCKASGITFSNSGMHWVR QAPGKGLEWVAVIWYDGSKRYYADSVKGRFTISRDNSKN TLFLQMNSLRAEDTAVYYCATNDDYWGQGTLVTVSSEVQ LVQSGAEVKKPGASVKVSCKASGYTFTDSYMSWVRQAP GQGLEWIGDMYPDNGDSSYNQKFRERVTITRDTSTSTAYL ELSSLRSEDTAVYYCVLAPRWYFSVWGQGTLVTVSSASTK GPSVFPLAPSSKSTSGGTAALGCLVKDYFPEPVTVSWNSGA LTSGVHTFPAVLQSSGLYSLSSVVTVPSSSLGTQTYICNVNHK PSNTKVDKKVEPKSCDKTHTCPPCPAPEAAGGPSVFLFPPK PKDTLMISRTPEVTCVVVDVSHEDPEVKFNWYVDGVEVHN AKTKPREEQYNSTYRVVSVLTVLHQDWLNGKEYKCKVSNK ALPAPIEKTISKAKGQPREPQVYTLPPCRDELTKNQVSLWCL VKGFYPSDIAVEWESNGQPENNYKTTPPVLDSDGSFFLYSK LTVDKSRWQQGNVFSCSVMHEALHNHYTQKSLSLSPG | **anti-PD1-VH-*anti-OX40*-VH-**huIGHG1(LALA-knob) |
| | 58 | AIQLTQSPSSLSASVGDRVTITCRASQGISSALAWYQQKPG KAPKLLIYDASSLESGVPSRFSGSGSGTDFTLTISSLQPEDFA TYYCQQFNSYPYTFGQGTKLEIKRTVAAPSVFIFPPSDEQLK SGTASVVCLLNNFYPREAKVQWKVDNALQSGNSQESVTEQ DSKDSTYSLSSTLTLSKADYEKHKVYACEVTHQGLSSPVTKSF NRGEC | **anti-GITR-VL**-huIGKC |
| | 59 | QVQLVESGGGVVQPGRSLRLSCAASGFTFSSYGMHWVR QAPGKGLEWVAVIWYEGSNKYYADSVKGRFTISRDNSKN TLYLQMNSLRAEDTAVYYCARGGSMVRGDYYYGMDVW GQGTTVTVSSASTKGPSVFPLAPSSKSTSGGTAALGCLVKD YFPEPVTVSWNSGALTSGVHTFPAVLQSSGLYSLSSVVTVPS SSLGTQTYICNVNHKPSNTKVDKKVEPKSCDKTHTCPPCPA PEAAGGPSVFLFPPKPKDTLMISRTPEVTCVVVDVSHEDPE VKFNWYVDGVEVHNAKTKPREEQYNSTYRVVSVLTVLHQD WLNGKEYKCKVSNKALPAPIEKTISKAKGQPREPQVCTLPPS RDELTKNQVSLSCAVKGFYPSDIAVEWESNGQPENNYKTTP PVLDSDGSFFLVSKLTVDKSRWQQGNVFSCSVMHEALHNR FTQKSLSLSPG | **anti-GITR-VH-**huIGHG1(LALA-hole-RF) |
| TRI-(CODV-anti-PD-1 x anti-OX40) x sc- PCL2-anti-GITR-huIgG1-LALA-KIH-RF | 56 | DIQMTQSPSSLSASVGDRVTITCRASQDISNYLNWYQQKP GKAPKLLIYYTSRLRSGVPSRFSGSGSGTDFTLTISSLQPEDF ATYYCQQGHTLPPTFGQGTKVEIKGGGGSGGGGSEIVLTQ SPATLSLSPGERATLSCRASQSVSSYLAWYQQKPGQAPRL LIYDASNRATGIPARFSGSGSGTDFTLTISSLEPEDFAVYYC QQSSNWPRTFGQGTKVEIKGGGGSGGGGSRTVAAPSVFI FPPSDEQLKSGTASVVCLLNNFYPREAKVQWKVDNALQSG NSQESVTEQDSKDSTYSLSSTLTLSKADYEKHKVYACEVTHQ GLSSPVTKSFNRGEC | **anti-OX40-VL-**<(G4S)2>-***anti-PD1-VL***-<(G4S)2>-huIGKC |
| | 57 | QVQLVESGGGVVQPGRSLRLDCKASGITFSNSGMHWVR QAPGKGLEWVAVIWYDGSKRYYADSVKGRFTISRDNSKN TLFLQMNSLRAEDTAVYYCATNDDYWGQGTLVTVSSEVQ LVQSGAEVKKPGASVKVSCKASGYTFTDSYMSWVRQAP GQGLEWIGDMYPDNGDSSYNQKFRERVTITRDTSTSTAYL ELSSLRSEDTAVYYCVLAPRWYFSVWGQGTLVTVSSASTK GPSVFPLAPSSKSTSGGTAALGCLVKDYFPEPVTVSWNSGA LTSGVHTFPAVLQSSGLYSLSSVVTVPSSSLGTQTYICNVNHK PSNTKVDKKVEPKSCDKTHTCPPCPAPEAAGGPSVFLFPPK PKDTLMISRTPEVTCVVVDVSHEDPEVKFNWYVDGVEVHN AKTKPREEQYNSTYRVVSVLTVLHQDWLNGKEYKCKVSNK ALPAPIEKTISKAKGQPREPQVYTLPPCRDELTKNQVSLWCL VKGFYPSDIAVEWESNGQPENNYKTTPPVLDSDGSFFLYSK LTVDKSRWQQGNVFSCSVMHEALHNHYTQKSLSLSPG | **anti-PD1-VH-*anti-OX40*-VH-**huIGHG1(LALA-knob) |
| | 60 | AIQLTQSPSSLSASVGDRVTITCRASQGISSALAWYQQKPG KAPKLLIYDASSLESGVPSRFSGSGSGTDFTLTISSLQPEDFA TYYCQQFNSYPYTFGQGTKLEIKRTVAAPSVFIFPPSDEQL KSGTASVVCLLNNFYPREAKVQWKVDNALQSGNSQESVT EQDSKDSTYSLSSTLTLSKADYEKHKVYACEVTHQGLSSPV TKSFNRGECHRRRKRSVDEGGGGSGGGGSGGGGSGGGG SGGGGSGGGGSHRRRKRSVDEQVQLVESGGGVVQPGRSL RLSCAASGFTFSSYGMHWVRQAPGKGLEWVAVIWYEGS NKYYADSVKGRFTISRDNSKNTLYLQMNSLRAEDTAVYYC ARGGSMVRGDYYYGMDVWGQGTTVTVSSASTKGPSVFP LAPSSKSTSGGTAALGCLVKDYFPEPVTVSWNSGALTSGVH TFPAVLQSSGLYSLSSVVTVPSSSLGTQTYICNVNHKPSNTKV DKKVEPKSCDKTHTCPPCPAPEAAGGPSVFLFPPKPKDTLMI SRTPEVTCVVVDVSHEDPEVKFNWYVDGVEVHNAKTKPRE EQYNSTYRVVSVLTVLHQDWLNGKEYKCKVSNKALPAPIEK TISKAKGQPREPQVCTLPPSRDELTKNQVSLSCAVKGFYPSD IAVEWESNGQPENNYKTTPPVLDSDGSFFLVSKLTVDKSRW QQGNVFSCSVMHEALHNRFTQKSLSLSPG | **anti-GITR-VL-huIGKC:** [*PCS2*]-<(G4S)6>-[*PCS2*]-***anti-GITR-VH-***huIGHG1(LALA-hole-RF) |
| TRI-(CODV-anti-PD-1 x anti-OX40) x sc- PCL2a-anti-GITR-huIgG1-LALA-KIH-RF | 56 | DIQMTQSPSSLSASVGDRVTITCRASQDISNYLNWYQQKP GKAPKLLIYYTSRLRSGVPSRFSGSGSGTDFTLTISSLQPEDF ATYYCQQGHTLPPTFGQGTKVEIKGGGGSGGGGSEIVLTQ SPATLSLSPGERATLSCRASQSVSSYLAWYQQKPGQAPRLL IYDASNRATGIPARFSGSGSGTDFTLTISSLEPEDFAVYYCQ QSSNWPRTFGQGTKVEIKGGGGSGGGGSRTVAAPSVFIFP PSDEQLKSGTASVVCLLNNFYPREAKVQWKVDNALQSGNS QESVTEQDSKDSTYSLSSTLTLSKADYEKHKVYACEVTHQGL SSPVTKSFNRGEC | **anti-OX40-VL-**<(G4S)2>-***anti-PD1-VL***-<(G4S)2>-huIGKC |
| | 57 | QVQLVESGGGVVQPGRSLRLDCKASGITFSNSGMHWVR QAPGKGLEWVAVIWYDGSKRYYADSVKGRFTISRDNSKN TLFLQMNSLRAEDTAVYYCATNDDYWGQGTLVTVSSEVQ LVQSGAEVKKPGASVKVSCKASGYTFTDSYMSWVRQAP | **anti-PD1-VH-*anti-OX40*-VH-**huIGHG1(LALA-knob) |
| | | GQGLEWIGDMYPDNGDSSYNQKFRERVTITRDTSTSTAYL ELSSLRSEDTAVYYCVLAPRWYFSVWGQGTLVTVSSASTK GPSVFPLAPSSKSTSGGTAALGCLVKDYFPEPVTVSWNSGA LTSGVHTFPAVLQSSGLYSLSSVVTVPSSSLGTQTYICNVNHK PSNTKVDKKVEPKSCDKTHTCPPCPAPEAAGGPSVFLFPPK PKDTLMISRTPEVTCVVVDVSHEDPEVKFNWYVDGVEVHN AKTKPREEQYNSTYRVVSVLTVLHQDWLNGKEYKCKVSNK ALPAPIEKTISKAKGQPREPQVYTLPPCRDELTKNQVSLWCL VKGFYPSDIAVEWESNGQPENNYKTTPPVLDSDGSFFLYSK LTVDKSRWQQGNVFSCSVMHEALHNHYTQKSLSLSPG | |
| | 61 | AIQLTQSPSSLSASVGDRVTITCRASQGISSALAWYQQKPG KAPKLLIYDASSLESGVPSRFSGSGSGTDFTLTISSLQPEDFA TYYCQQFNSYPYTFGQGTKLEIKRTVAAPSVFIFPPSDEQL KSGTASVVCLLNNFYPREAKVQWKVDNALQSGNSQESVT EQDSKDSTYSLSSTLTLSKADYEKHKVYACEVTHQGLSSPV TKSFNRGECHRRQQRSVDEGGGGSGGGGSGGGGSGGGG SGGGGSGGGGSHRRQQRSVDEQVQLVESGGGVVQPGRS LRLSCAASGFTFSSYGMHWVRQAPGKGLEWVAVIWYEG SNKYYADSVKGRFTISRDNSKNTLYLQMNSLRAEDTAVYY CARGGSMVRGDYYYGMDVWGQGTTVTVSSASTKGPSVF PLAPSSKSTSGGTAALGCLVKDYFPEPVTVSWNSGALTSGV HTFPAVLQSSGLYSLSSVVTVPSSSLGTQTYICNVNHKPSNT KVDKKVEPKSCDKTHTCPPCPAPEAAGGPSVFLFPPKPKDTL MISRTPEVTCVVVDVSHEDPEVKFNWYVDGVEVHNAKTKP REEQYNSTYRVVSVLTVLHQDWLNGKEYKCKVSNKALPAPI EKTISKAKGQPREPQVCTLPPSRDELTKNQVSLSCAVKGFYP SDIAVEWESNGQPENNYKTTPPVLDSDGSFFLVSKLTVDKS RWQQGNVFSCSVMHEALHNRFTQKSLSLSPG | **anti-GITR-VL**-huIGKC-[*PCS2a*]-<(G4S)6>-[*PCS2a*]*-**anti-GITR-VH***-huIGHG1(LALA-hole-RF) |
| TRI-(CODV-anti-GITR x anti-PD-1) x sc-PCL2-anti-OX40-huIgG1-LALA-KIH-RF | 62 | EIVLTQSPATLSLSPGERATLSCRASQSVSSYLAWYQQKPG QAPRLLIYDASNRATGIPARFSGSGSGTDFTLTISSLEPEDF AVYYCQQSSNWPRTFGQGTKVEIKGGGGSGGGGSAIQLT QSPSSLSASVGDRVTITCRASQGISSALAWYQQKPGKAPK LLIYDASSLESGVPSRFSGSGSGTDFTLTISSLQPEDFATYYC QQFNSYPYTFGQGTKLEIKGGGGSGGGGSRTVAAPSVFIFP PSDEQLKSGTASVVCLLNNFYPREAKVQWKVDNALQSGNS QESVTEQDSKDSTYSLSSTLTLSKADYEKHKVYACEVTHQGL SSPVTKSFNRGEC | **anti-PD1-VL-**<(G4S)2>-***anti-GITR-VL***-<(G4S)2>-huIGKC |
| | 63 | QVQLVESGGGVVQPGRSLRLSCAASGFTFSSYGMHWVR QAPGKGLEWVAVIWYEGSNKYYADSVKGRFTISRDNSKN TLYLQMNSLRAEDTAVYYCARGGSMVRGDYYYGMDVW GQGTTVTVSSQVQLVESGGGVVQPGRSLRLDCKASGITFS NSGMHWVRQAPGKGLEWVAVIWYDGSKRYYADSVKGR FTISRDNSKNTLFLQMNSLRAEDTAVYYCATNDDYWGQG TLVTVSSASTKGPSVFPLAPSSKSTSGGTAALGCLVKDYFPEP VTVSWNSGALTSGVHTFPAVLQSSGLYSLSSVVTVPSSSLGT QTYICNVNHKPSNTKVDKKVEPKSCDKTHTCPPCPAPEAAG GPSVFLFPPKPKDTLMISRTPEVTCVVVDVSHEDPEVKFNW YVDGVEVHNAKTKPREEQYNSTYRVVSVLTVLHQDWLNGK EYKCKVSNKALPAPIEKTISKAKGQPREPQVYTLPPCRDELTK NQVSLWCLVKGFYPSDIAVEWESNGQPENNYKTTPPVLDS DGSFFLYSKLTVDKSRWQQGNVFSCSVMHEALHNHYTQKS LSLSPG | **anti-GITR-VH-anti-*PD1-VH-***huIGHG1(LALA-knob) |
| | 64 | DIQMTQSPSSLSASVGDRVTITCRASQDISNYLNWYQQKP GKAPKLLIYYTSRLRSGVPSRFSGSGSGTDFTLTISSLQPEDF | **anti-OX40-VL**-huIGKC-[*PCS2*]-<(G4S)6>- |
| | | ATYYCQQGHTLPPTFGQGTKVEIKRTVAAPSVFIFPPSDEQ LKSGTASVVCLLNNFYPREAKVQWKVDNALQSGNSQESVT EQDSKDSTYSLSSTLTLSKADYEKHKVYACEVTHQGLSSPVT KSFNRGECHRRRKRSVDEGGGGSGGGGSGGGGSGGGGS GGGGSGGGGSHRRRKRSVDEEVQLVQSGAEVKKPGASVK VSCKASGYTFTDSYMSWVRQAPGQGLEWIGDMYPDNG DSSYNQKFRERVTITRDTSTSTAYLELSSLRSEDTAVYYCVL APRWYFSVWGQGTLVTVSSASTKGPSVFPLAPSSKSTSGG TAALGCLVKDYFPEPVTVSWNSGALTSGVHTFPAVLQSSGL YSLSSVVTVPSSSLGTQTYICNVNHKPSNTKVDKKVEPKSCD KTHTCPPCPAPEAAGGPSVFLFPPKPKDTLMISRTPEVTCVV VDVSHEDPEVKFNWYVDGVEVHNAKTKPREEQYNSTYRV VSVLTVLHQDWLNGKEYKCKVSNKALPAPIEKTISKAKGQP REPQVCTLPPSRDELTKNQVSLSCAVKGFYPSDIAVEWESN GQPENNYKTTPPVLDSDGSFFLVSKLTVDKSRWQQGNVFS CSVMHEALHNRFTQKSLSLSPG | [*PCS2*]-anti-OX40-VH-huIGHG1(LALA-hole-RF) |
| TRI-(CODV-anti-GITR x anti-PD-1)x sc-PCL2a-anti-OX40-huIgG1-LALA-KIH-RF | 62 | EIVLTQSPATLSLSPGERATLSCRASQSVSSYLAWYQQKPG QAPRLLIYDASNRATGIPARFSGSGSGTDFTLTISSLEPEDF AVYYCQQSSNWPRTFGQGTKVEIKGGGGSGGGGSAIQLT QSPSSLSASVGDRVTITCRASQGISSALAWYQQKPGKAPK LLIYDASSLESGVPSRFSGSGSGTDFTLTISSLQPEDFATYYC QQFNSYPYTFGQGTKLEIKGGGGSGGGGSRTVAAPSVFIFP PSDEQLKSGTASVVCLLNNFYPREAKVQWKVDNALQSGNS QESVTEQDSKDSTYSLSSTLTLSKADYEKHKVYACEVTHQGL SSPVTKSFNRGEC | **anti-PD1-VL-**<(G4S)2>-***anti-GITR-VL***-<(G4S)2>-huIGKC |
| | 63 | QVQLVESGGGVVQPGRSLRLSCAASGFTFSSYGMHWVR QAPGKGLEWVAVIWYEGSNKYYADSVKGRFTISRDNSKN TLYLQMNSLRAEDTAVYYCARGGSMVRGDYYYGMDVW GQGTTVTVSSQVQLVESGGGVVQPGRSLRLDCKASGITFS NSGMHWVRQAPGKGLEWVAVIWYDGSKRYYADSVKGR FTISRDNSKNTLFLQMNSLRAEDTAVYYCATNDDYWGQG TLVTVSSASTKGPSVFPLAPSSKSTSGGTAALGCLVKDYFPEP VTVSWNSGALTSGVHTFPAVLQSSGLYSLSSVVTVPSSSLGT QTYICNVNHKPSNTKVDKKVEPKSCDKTHTCPPCPAPEAAG GPSVFLFPPKPKDTLMISRTPEVTCVVVDVSHEDPEVKFNW YVDGVEVHNAKTKPREEQYNSTYRVVSVLTVLHQDWLNGK EYKCKVSNKALPAPIEKTISKAKGQPREPQVYTLPPCRDELTK NQVSLWCLVKGFYPSDIAVEWESNGQPENNYKTTPPVLDS DGSFFLYSKLTVDKSRWQQGNVFSCSVMHEALHNHYTQKS LSLSPG | **anti-GITR-VH-anti-*PD1-VH-***huIGHG1(LALA-knob) |
| | 65 | DIQMTQSPSSLSASVGDRVTITCRASQDISNYLNWYQQKP GKAPKLLIYYTSRLRSGVPSRFSGSGSGTDFTLTISSLQPEDF ATYYCQQGHTLPPTFGQGTKVEIKRTVAAPSVFIFPPSDEQ LKSGTASVVCLLNNFYPREAKVQWKVDNALQSGNSQESVT EQDSKDSTYSLSSTLTLSKADYEKHKVYACEVTHQGLSSPVT KSFNRGECHRRQQRSVDEGGGGSGGGGSGGGGSGGGGS GGGGSGGGGSHRRQQRSVDEEVQLVQSGAEVKKPGASV KVSCKASGYTFTDSYMSWVRQAPGQGLEWIGDMYPDN GDSSYNQKFRERVTITRDTSTSTAYLELSSLRSEDTAVYYCV LAPRWYFSVWGQGTLVTVSSASTKGPSVFPLAPSSKSTSG GTAALGCLVKDYFPEPVTVSWNSGALTSGVHTFPAVLQSSG LYSLSSVVTVPSSSLGTQTYICNVNHKPSNTKVDKKVEPKSC DKTHTCPPCPAPEAAGGPSVFLFPPKPKDTLMISRTPEVTCV VVDVSHEDPEVKFNWYVDGVEVHNAKTKPREEQYNSTYR | **anti-OX40-VL**-huIGKC-[*PCS2a*]-<(G4S)6>-[*PCS2a*]*-****anti-OX40-*VH**-huIGHG1(LALA-hole-RF) |
| | | VVSVLTVLHQDWLNGKEYKCKVSNKALPAPIEKTISKAKGQ PREPQVCTLPPSRDELTKNQVSLSCAVKGFYPSDIAVEWES NGQPENNYKTTPPVLDSDGSFFLVSKLTVDKSRWQQGNVF SCSVMHEALHNRFTQKSLSLSPG | |
| TRI-(CODV-anti-PD-1 x anti-GITR) x sc-PCL2-anti-OX40-huIgG1-LALA-KIH-RF | 66 | AIQLTQSPSSLSASVGDRVTITCRASQGISSALAWYQQKPG KAPKLLIYDASSLESGVPSRFSGSGSGTDFTLTISSLQPEDFA TYYCQQFNSYPYTFGQGTKLEIKGGGGSGGGGSEIVLTQSP ATLSLSPGERATLSCRASQSVSSYLAWYQQKPGQAPRLLIY DASNRATGIPARFSGSGSGTDFTLTISSLEPEDFAVYYCQQS SNWPRTFGQGTKVEIKGGGGSGGGGSRTVAAPSVFIFPPS DEQLKSGTASVVCLLNNFYPREAKVQWKVDNALQSGNSQ ESVTEQDSKDSTYSLSSTLTLSKADYEKHKVYACEVTHQGLSS PVTKSFNRGEC | **anti-GITR-VL-**<(G4S)2>*-**anti-PD1-VL***-<(G4S)2>-huIGKC |
| | 67 | QVQLVESGGGVVQPGRSLRLDCKASGITFSNSGMHWVR QAPGKGLEWVAVIWYDGSKRYYADSVKGRFTISRDNSKN TLFLQMNSLRAEDTAVYYCATNDDYWGQGTLVTVSSQV QLVESGGGVVQPGRSLRLSCAASGFTFSSYGMHWVRQAP GKGLEWVAVIWYEGSNKYYADSVKGRFTISRDNSKNTLYL QMNSLRAEDTAVYYCARGGSMVRGDYYYGMDVWGQG TTVTVSSASTKGPSVFPLAPSSKSTSGGTAALGCLVKDYFPE PVTVSWNSGALTSGVHTFPAVLQSSGLYSLSSVVTVPSSSLG TQTYICNVNHKPSNTKVDKKVEPKSCDKTHTCPPCPAPEAA GGPSVFLFPPKPKDTLMISRTPEVTCVVVDVSHEDPEVKFN WYVDGVEVHNAKTKPREEQYNSTYRVVSVLTVLHQDWLN GKEYKCKVSNKALPAPIEKTISKAKGQPREPQVYTLPPCRDE LTKNQVSLWCLVKGFYPSDIAVEWESNGQPENNYKTTPPV LDSDGSFFLYSKLTVDKSRWQQGNVFSCSVMHEALHNHYT QKSLSLSPG | **anti-PD1-VH-*anti-GITR-VH-***huIGHG1(LALA-knob) |
| | 68 | DIQMTQSPSSLSASVGDRVTITCRASQDISNYLNWYQQKP GKAPKLLIYYTSRLRSGVPSRFSGSGSGTDFTLTISSLQPEDF ATYYCQQGHTLPPTFGQGTKVEIKRTVAAPSVFIFPPSDEQ LKSGTASVVCLLNNFYPREAKVQWKVDNALQSGNSQESV TEQDSKDSTYSLSSTLTLSKADYEKHKVYACEVTHQGLSSP VTKSFNRGECHRRRKRSVDEGGGGSGGGGSGGGGSGGG GSGGGGSGGGGSHRRRKRSVDEEVQLVQSGAEVKKPGAS VKVSCKASGYTFTDSYMSWVRQAPGQGLEWIGDMYPD NGDSSYNQKFRERVTITRDTSTSTAYLELSSLRSEDTAVYYC VLAPRWYFSVWGQGTLVTVSSASTKGPSVFPLAPSSKSTS GGTAALGCLVKDYFPEPVTVSWNSGALTSGVHTFPAVLQSS GLYSLSSVVTVPSSSLGTQTYICNVNHKPSNTKVDKKVEPKS CDKTHTCPPCPAPEAAGGPSVFLFPPKPKDTLMISRTPEVTC VVVDVSHEDPEVKFNWYVDGVEVHNAKTKPREEQYNSTY RVVSVLTVLHQDWLNGKEYKCKVSNKALPAPIEKTISKAKG QPREPQVCTLPPSRDELTKNQVSLSCAVKGFYPSDIAVEWE SNGQPENNYKTTPPVLDSDGSFFLVSKLTVDKSRWQQGNV FSCSVMHEALHNRFTQKSLSLSPG | **anti-OX40-VL-**huIGKC-[*PCS2*]-<(G4S)6>-[*PCS2*]***-anti-OX40-VH-***huIGHG1(LALA-hole-RF) |
| TRI-(CODV-anti-PD-1 x anti-GITR) x sc-PCL2a-anti-OX40-huIgG1- | 66 | AIQLTQSPSSLSASVGDRVTITCRASQGISSALAWYQQKPG KAPKLLIYDASSLESGVPSRFSGSGSGTDFTLTISSLQPEDFA TYYCQQFNSYPYTFGQGTKLEIKGGGGSGGGGSEIVLTQSP ATLSLSPGERATLSCRASQSVSSYLAWYQQKPGQAPRLLIY DASNRATGIPARFSGSGSGTDFTLTISSLEPEDFAVYYCQQS SNWPRTFGQGTKVEIKGGGGSGGGGSRTVAAPSVFIFPPS DEQLKSGTASVVCLLNNFYPREAKVQWKVDNALQSGNSQ | **anti-GITR-VL-**<(G4S)2>-***anti-PD1-VL***-<(G4S)2>-huIGKC |
| LALA-KIH-RF | | ESVTEQDSKDSTYSLSSTLTLSKADYEKHKVYACEVTHQGLSS PVTKSFNRGEC | |
| | 67 | QVQLVESGGGVVQPGRSLRLDCKASGITFSNSGMHWVR QAPGKGLEWVAVIWYDGSKRYYADSVKGRFTISRDNSKN TLFLQMNSLRAEDTAVYYCATNDDYWGQGTLVTVSSQV QLVESGGGVVQPGRSLRLSCAASGFTFSSYGMHWVRQAP GKGLEWVAVIWYEGSNKYYADSVKGRFTISRDNSKNTLYL QMNSLRAEDTAVYYCARGGSMVRGDYYYGMDVWGQG TTVTVSSASTKGPSVFPLAPSSKSTSGGTAALGCLVKDYFPE PVTVSWNSGALTSGVHTFPAVLQSSGLYSLSSVVTVPSSSLG TQTYICNVNHKPSNTKVDKKVEPKSCDKTHTCPPCPAPEAA GGPSVFLFPPKPKDTLMISRTPEVTCVVVDVSHEDPEVKFN WYVDGVEVHNAKTKPREEQYNSTYRVVSVLTVLHQDWLN GKEYKCKVSNKALPAPIEKTISKAKGQPREPQVYTLPPCRDE LTKNQVSLWCLVKGFYPSDIAVEWESNGQPENNYKTTPPV LDSDGSFFLYSKLTVDKSRWQQGNVFSCSVMHEALHNHYT QKSLSLSPG | **anti-PD1-VH-*anti-GITR-VH-***huIGHG1(LALA-knob) |
| | 69 | DIQMTQSPSSLSASVGDRVTITCRASQDISNYLNWYQQKP GKAPKLLIYYTSRLRSGVPSRFSGSGSGTDFTLTISSLQPEDF ATYYCQQGHTLPPTFGQGTKVEIKRTVAAPSVFIFPPSDEQ LKSGTASVVCLLNNFYPREAKVQWKVDNALQSGNSQESV TEQDSKDSTYSLSSTLTLSKADYEKHKVYACEVTHQGLSSP VTKSFNRGECHRRQQRSVDEGGGGSGGGGSGGGGSGGG GSGGGGSGGGGSHRRQQRSVDEEVQLVQSGAEVKKPGA SVKVSCKASGYTFTDSYMSWVRQAPGQGLEWIGDMYPD NGDSSYNQKFRERVTITRDTSTSTAYLELSSLRSEDTAVYYC VLAPRWYFSVWGQGTLVTVSSASTKGPSVFPLAPSSKSTS GGTAALGCLVKDYFPEPVTVSWNSGALTSGVHTFPAVLQSS GLYSLSSVVTVPSSSLGTQTYICNVNHKPSNTKVDKKVEPKS CDKTHTCPPCPAPEAAGGPSVFLFPPKPKDTLMISRTPEVTC VVVDVSHEDPEVKFNWYVDGVEVHNAKTKPREEQYNSTY RVVSVLTVLHQDWLNGKEYKCKVSNKALPAPIEKTISKAKG QPREPQVCTLPPSRDELTKNQVSLSCAVKGFYPSDIAVEWE SNGQPENNYKTTPPVLDSDGSFFLVSKLTVDKSRWQQGNV FSCSVMHEALHNRFTQKSLSLSPG | **anti-OX40-VL**-huIGKC-[*PCS2a*]-<(G4S)6>-[*PCS2a*]*-**anti-OX40-VH***-huIGHG1(LALA-hole-RF) |
| sLC-Tandem-( anti-PD-1)x(anti-4.1BB)-PCL2 huIgG1-LALA_AB0-2 | 70 | EIVLTQSPATLSLSPGERATLSCRASQSVSSYLAWYQQKPG QAPRLLIYDASNRATGIPARFSGSGSGTDFTLTISSLEPEDF AVYYCQQSSNWPRTFGQGTKVEIKRTVAAPSVFIFPPSDE QLKSGTASVVCLLNNFYPREAKVQWKVDNALQSGNSQESV TEQDSKDSTYSLSSTLTLSKADYEKHKVYACEVTHQGLSSPV TKSFNRGECHRRRKRSVDEGGGGSGGGGSGGGGSHRRRK RSVDEEIVLTQSPATLSLSPGERATLSCRASQSVSSYLAWYQ QKPGQAPRLLIYDASNRATGIPARFSGSGSGTDFTLTISSLE PEDFAVYYCQQRSNWPPALTFGGGTKVEIKRTVAAPSVFIF PPSDEQLKSGTASVVCLLNNFYPREAKVQWKVDNALQSGN SQESVTEQDSKDSTYSLSSTLTLSKADYEKHKVYACEVTHQG LSSPVTKSFNRGEC | **anti-PD1-VL**-huIGKC-*[PCS2]-<(G4S)3>-[PCS2]-**anti-4.1BB-VL-***huIGKC |
| | 71 | QVQLVESGGGVVQPGRSLRLDCKASGITFSNSGMHWVR QAPGKGLEWVAVIWYDGSKRYYADSVKGRFTISRDNSKN TLFLQMNSLRAEDTAVYYCATNDDYWGQGTLVTVSSAST KGPSVFPLAPSSKSTSGGTAALGCLVKDYFPEPVTVSWNSG ALTSGVHTFPAVLQSSGLYSLSSVVTVPSSSLGTQTYICNVN HKPSNTKVDKKVEPKSCDKTHTGGGGSGGGGSGGGGSQV QLQQWGAGLLKPSETLSLTCAVYGGSFSGYYWSWIRQSP | **anti-PD1-VH**-huIGHG11-huIGH-H1-<(*G4S*)3>-**anti-4.1BB-VH**-huIGHG1-(LALA) |
| | | EKGLEWIGEINHGGYVTYNPSLESRVTISVDTSKNQFSLKLS SVTAADTAVYYCARDYGPGNYDWYFDLWGRGTLVTVSS ASTKGPSVFPLAPSSKSTSGGTAALGCLVKDYFPEPVTVSW NSGALTSGVHTFPAVLQSSGLYSLSSVVTVPSSSLGTQTYICN VNHKPSNTKVDKKVEPKSCDKTHTCPPCPAPEAAGGPSVFL FPPKPKDTLMISRTPEVTCVVVDVSHEDPEVKFNWYVDGV EVHNAKTKPREEQYNSTYRVVSVLTVLHQDWLNGKEYKCK VSNKALPAPIEKTISKAKGQPREPQVYTLPPSRDELTKNQVS LTCLVKGFYPSDIAVEWESNGQPENNYKTTPPVLDSDGSFFL YSKLTVDKSRWQQGNVFSCSVMHEALHNHYTQKSLSLSPG | |
| sLC-Tandem-( anti-PD-1)x(anti-4.1BB)-PCL2a huIgG1-LALA_AB0-2 | 72 | EIVLTQSPATLSLSPGERATLSCRASQSVSSYLAWYQQKPG QAPRLLIYDASNRATGIPARFSGSGSGTDFTLTISSLEPEDF AVYYCQQSSNWPRTFGQGTKVEIKRTVAAPSVFIFPPSDE QLKSGTASVVCLLNNFYPREAKVQWKVDNALQSGNSQESV TEQDSKDSTYSLSSTLTLSKADYEKHKVYACEVTHQGLSSPV TKSFNRGECHRRQQRSVDEGGGGSGGGGSGGGGSHRRQ QRSVDEEIVLTQSPATLSLSPGERATLSCRASQSVSSYLAWY QQKPGQAPRLLIYDASNRATGIPARFSGSGSGTDFTLTISSL EPEDFAVYYCQQRSNWPPALTFGGGTKVEIKRTVAAPSVFI FPPSDEQLKSGTASVVCLLNNFYPREAKVQWKVDNALQSG NSQESVTEQDSKDSTYSLSSTLTLSKADYEKHKVYACEVTHQ GLSSPVTKSFNRGEC | **anti-PD1-VL-huIGKC-***[PCS2a]-<(G4S)3>-[PCS2a]-**anti-4.1BB-VL***-huIGKC |
| | 71 | QVQLVESGGGVVQPGRSLRLDCKASGITFSNSGMHWVR QAPGKGLEWVAVIWYDGSKRYYADSVKGRFTISRDNSKN TLFLQMNSLRAEDTAVYYCATNDDYWGQGTLVTVSSAST KGPSVFPLAPSSKSTSGGTAALGCLVKDYFPEPVTVSWNSG ALTSGVHTFPAVLQSSGLYSLSSVVTVPSSSLGTQTYICNVN HKPSNTKVDKKVEPKSCDKTHTGGGGSGGGGSGGGGSQV QLQQWGAGLLKPSETLSLTCAVYGGSFSGYYWSWIRQSP EKGLEWIGEINHGGYVTYNPSLESRVTISVDTSKNQFSLKLS SVTAADTAVYYCARDYGPGNYDWYFDLWGRGTLVTVSS ASTKGPSVFPLAPSSKSTSGGTAALGCLVKDYFPEPVTVSW NSGALTSGVHTFPAVLQSSGLYSLSSVVTVPSSSLGTQTYICN VNHKPSNTKVDKKVEPKSCDKTHTCPPCPAPEAAGGPSVFL FPPKPKDTLMISRTPEVTCVVVDVSHEDPEVKFNWYVDGV EVHNAKTKPREEQYNSTYRVVSVLTVLHQDWLNGKEYKCK VSNKALPAPIEKTISKAKGQPREPQVYTLPPSRDELTKNQVS LTCLVKGFYPSDIAVEWESNGQPENNYKTTPPVLDSDGSFFL YSKLTVDKSRWQQGNVFSCSVMHEALHNHYTQKSLSLSPG | **anti-PD1-VH**-huIGHG11-huIGH-H1-<(G4S)3>-**anti-4.1BB-VH**-huIGHG1-(LALA) |
| sLC-Tandem-( anti-PD-1)x(anti-OX40)-PCL2 huIgG1-LALA_AC0-2 | 73 | EIVLTQSPATLSLSPGERATLSCRASQSVSSYLAWYQQKPG QAPRLLIYDASNRATGIPARFSGSGSGTDFTLTISSLEPEDF AVYYCQQSSNWPRTFGQGTKVEIKRTVAAPSVFIFPPSDE QLKSGTASVVCLLNNFYPREAKVQWKVDNALQSGNSQESV TEQDSKDSTYSLSSTLTLSKADYEKHKVYACEVTHQGLSSPV TKSFNRGECHRRRKRSVDEGGGGSGGGGSGGGGSHRRRK RSVDEDIQMTQSPSSLSASVGDRVTITCRASQDISNYLNW YQQKPGKAPKLLIYYTSRLRSGVPSRFSGSGSGTDFTLTISSL QPEDFATYYCQQGHTLPPTFGQGTKVEIKRTVAAPSVFIFP PSDEQLKSGTASVVCLLNNFYPREAKVQWKVDNALQSGNS QESVTEQDSKDSTYSLSSTLTLSKADYEKHKVYACEVTHQGL SSPVTKSFNRGEC | **anti-PD1-VL**-huIGKC-*[PCS2]-<(G4S)3>-[PCS2]-**anti-OX40-VL-***huIGKC |
| | 74 | QVQLVESGGGVVQPGRSLRLDCKASGITFSNSGMHWVR QAPGKGLEWVAVIWYDGSKRYYADSVKGRFTISRDNSKN TLFLQMNSLRAEDTAVYYCATNDDYWGQGTLVTVSSAST | **anti-PD1-VH**-huIGHG11-huIGH-H1- |
| | | KGPSVFPLAPSSKSTSGGTAALGCLVKDYFPEPVTVSWNSG ALTSGVHTFPAVLQSSGLYSLSSVVTVPSSSLGTQTYICNVN HKPSNTKVDKKVEPKSCDKTHTGGGGSGGGGSGGGGSEV QLVQSGAEVKKPGASVKVSCKASGYTFTDSYMSWVRQA PGQGLEWIGDMYPDNGDSSYNQKFRERVTITRDTSTSTA YLELSSLRSEDTAVYYCVLAPRWYFSVWGQGTLVTVSSAST KGPSVFPLAPSSKSTSGGTAALGCLVKDYFPEPVTVSWNSG ALTSGVHTFPAVLQSSGLYSLSSVVTVPSSSLGTQTYICNVN HKPSNTKVDKKVEPKSCDKTHTCPPCPAPEAAGGPSVFLFP PKPKDTLMISRTPEVTCVVVDVSHEDPEVKFNWYVDGVEV HNAKTKPREEQYNSTYRVVSVLTVLHQDWLNGKEYKCKVS NKALPAPIEKTISKAKGQPREPQVYTLPPSRDELTKNQVSLTC LVKGFYPSDIAVEWESNGQPENNYKTTPPVLDSDGSFFLYS KLTVDKSRWQQGNVFSCSVMHEALHNHYTQKSLSLSPG | *<(G4S)3>-***anti-OX40-VH**-huIGHG1-(LALA) |
| sLC-Tandem-( anti-PD-1)x(anti-OX40)-PCL2a huIgG1-LALA_AC0-2 | 75 | EIVLTQSPATLSLSPGERATLSCRASQSVSSYLAWYQQKPG QAPRLLIYDASNRATGIPARFSGSGSGTDFTLTISSLEPEDF AVYYCQQSSNWPRTFGQGTKVEIKRTVAAPSVFIFPPSDE QLKSGTASVVCLLNNFYPREAKVQWKVDNALQSGNSQESV TEQDSKDSTYSLSSTLTLSKADYEKHKVYACEVTHQGLSSPV TKSFNRGECHRRQQRSVDEGGGGSGGGGSGGGGSHRRQ QRSVDEDIQMTQSPSSLSASVGDRVTITCRASQDISNYLN WYQQKPGKAPKLLIYYTSRLRSGVPSRFSGSGSGTDFTLTIS SLQPEDFATYYCQQGHTLPPTFGQGTKVEIKRTVAAPSVFI FPPSDEQLKSGTASVVCLLNNFYPREAKVQWKVDNALQSG NSQESVTEQDSKDSTYSLSSTLTLSKADYEKHKVYACEVTHQ GLSSPVTKSFNRGEC | **anti-PD1-VL**-huIGKC-*[PCS2a]-<(G4S)3>-[PCS2a]**-anti-OX40-VL***-huIGKC |
| | 74 | QVQLVESGGGVVQPGRSLRLDCKASGITFSNSGMHWVR QAPGKGLEWVAVIWYDGSKRYYADSVKGRFTISRDNSKN TLFLQMNSLRAEDTAVYYCATNDDYWGQGTLVTVSSAST KGPSVFPLAPSSKSTSGGTAALGCLVKDYFPEPVTVSWNSG ALTSGVHTFPAVLQSSGLYSLSSVVTVPSSSLGTQTYICNVN HKPSNTKVDKKVEPKSCDKTHTGGGGSGGGGSGGGGSEV QLVQSGAEVKKPGASVKVSCKASGYTFTDSYMSWVRQA PGQGLEWIGDMYPDNGDSSYNQKFRERVTITRDTSTSTA YLELSSLRSEDTAVYYCVLAPRWYFSVWGQGTLVTVSSAST KGPSVFPLAPSSKSTSGGTAALGCLVKDYFPEPVTVSWNSG ALTSGVHTFPAVLQSSGLYSLSSVVTVPSSSLGTQTYICNVN HKPSNTKVDKKVEPKSCDKTHTCPPCPAPEAAGGPSVFLFP PKPKDTLMISRTPEVTCVVVDVSHEDPEVKFNWYVDGVEV HNAKTKPREEQYNSTYRVVSVLTVLHQDWLNGKEYKCKVS NKALPAPIEKTISKAKGQPREPQVYTLPPSRDELTKNQVSLTC LVKGFYPSDIAVEWESNGQPENNYKTTPPVLDSDGSFFLYS KLTVDKSRWQQGNVFSCSVMHEALHNHYTQKSLSLSPG | **anti-PD1-VH**-huIGHG11-huIGH-H1-*<(G4S)3>*-**anti-OX40-VH**-huIGHG1-(LALA) |

## Claims

1. A protein comprising at least one polypeptide chain having the formula
PP₁-PCL-PP₂,
wherein
PP₁ is a first polypeptide,
PP₂ is a second polypeptide, and
PCL is a protease-cleavable linker comprising at least one protease-cleavage site comprising the amino acid sequence HRRX₁X₂RSVDE (SEQ ID NO: 42), wherein X₁ and X₂ are independently selected from the group consisting of KK, KQ, KR, QK, QQ, QR, RK, RQ and RR..

2. The protein of claim 1, wherein the at least one protease-cleavage site comprises the amino acid sequence HRRRKRSVDE (SEQ ID NO: 43) or the amino acid sequence HRRQQRSVDE (SEQ ID NO: 44).

3. The protein of claim 1 or 2, wherein protease-cleavage of the linker results in a change in activity of the protein, wherein, preferably, the change in activity is binding or increased binding to at least one antigen.

4. The protein of any one of claims 1 to 3, wherein the protease-cleavable linker comprises two protease-cleavage sites, wherein the two protease-cleavage sites are located at the N-terminus and at the C-terminus of the protease-cleavable linker, respectively, wherein the two protease-cleavage sites may be the same or different.

5. The protein of any one of claims 1 to 4, wherein PP₁ comprises at least one immunoglobulin constant region and/or at least one immunoglobulin variable region, and/or wherein PP₂ comprises at least one immunoglobulin constant region and/or at least one immunoglobulin variable region, wherein, preferably,
PP₁ comprises an immunoglobulin constant region and PP₂ comprises an immunoglobulin variable region,
PP₁ comprises an immunoglobulin variable region and PP₂ comprises an immunoglobulin constant region,
PP₁ comprises an immunoglobulin constant region and PP₂ comprises an immunoglobulin constant region, or
PP₁ comprises an immunoglobulin variable region and PP₂ comprises an immunoglobulin variable region.

6. The protein of any one of claims 1 to 5, wherein the protein is an antibody or antibody derivative, wherein, preferably, the protein is a single chain antibody, preferably a multispecific single chain antibody.

7. The protein of any one of claims 1 to 6, comprising a polypeptide chain having a formula selected from the group consisting of:
(a)
VL-CL-PCL-VH-CH1-hinge-CH2-CH3,
wherein
VL is an immunoglobulin light chain variable region;
CL is an immunoglobulin light chain constant region;
VH is an immunoglobulin heavy chain variable region;
CH1 is an immunoglobulin CH1 heavy chain constant region;
hinge is an immunoglobulin hinge region;
CH2 is an immunoglobulin CH2 heavy chain constant region;
CH3 is an immunoglobulin CH3 heavy chain constant region; and
PCL is the protease-cleavable linker;
(b)
VLₐ-CLₐ-PCL- VL_{b}-CL_{b},
wherein
VLₐ is a first immunoglobulin light chain variable region;
VL_{b} is a second immunoglobulin light chain variable region;
CLₐ is a first immunoglobulin light chain constant region;
CL_{b} is a second immunoglobulin light chain constant region; and
PCL is the protease-cleavable linker; and
(c)
VLₐ-CLₐ-PCL-VL_{b}-CL_{b}-L-hinge-CH2-CH3
wherein
VLₐ is a first immunoglobulin light chain variable region;
VL_{b} is a second immunoglobulin light chain variable region;
CLₐ is a first immunoglobulin light chain constant region;
CL_{b} is a second immunoglobulin light chain constant region;
L is an optional linker;
hinge is an immunoglobulin hinge region;
CH2 is an immunoglobulin CH2 heavy chain constant region;
CH3 is an immunoglobulin CH3 heavy chain constant region; and
PCL is the protease-cleavable linker.

8. A nucleic acid or set of nucleic acids encoding the protein of any one of claims 1 to 7.

9. A vector or set of vectors comprising the nucleic acid or set of nucleic acids of claim 8.

10. A host cell comprising a protein of any one of claims 1 to 7, a nucleic acid or set of nucleic acids of claim 8, or a vector or set of vectors of claim 9, wherein, preferably, the host cell is a mammalian cell.

11. Method of producing a protein comprising the steps:
i) culturing a host cell according to claim 10; and
ii) isolating the protein from the host cell.

12. A protein obtainable by the method of claim 11.

13. The protein of any one of claims 1 to 7, or 12, wherein the protein is a therapeutically active protein.

14. The protein of claim 13 for use in therapy.

## Patentansprüche

1. Protein, umfassend mindestens eine Polypeptidkette mit der Formel
PP₁-PCL-PP₂,
wobei
PP₁ für ein erstes Polypeptid steht,
PP₂ für ein zweites Polypeptid steht und
PCL für einen durch Protease spaltbaren Linker steht, der mindestens eine Protease-Spaltstelle umfasst, die die Aminosäuresequenz HRRX₁X₂RSVDE (SEQ ID NO: 42) umfasst, wobei X₁ und X₂ unabhängig aus der Gruppe bestehend aus KK, KQ, KR, QK, QQ, QR, RK, RQ und RR ausgewählt sind.

2. Protein nach Anspruch 1, wobei die mindestens eine Protease-Spaltstelle die Aminosäuresequenz HRRRKRSVDE (SEQ ID NO: 43) oder die Aminosäuresequenz HRRQQRSVDE (SEQ ID NO: 44) umfasst.

3. Protein nach Anspruch 1 oder 2, wobei die Protease-Spaltung des Linkers zu einer Änderung der Aktivität des Proteins führt, wobei es sich bei der Änderung der Aktivität vorzugsweise um Binden oder erhöhtes Binden an mindestens ein Antigen handelt.

4. Protein nach einem der Ansprüche 1 bis 3, wobei der durch Protease spaltbare Linker zwei Protease-Spaltstellen umfasst, wobei sich die beiden Protease-Spaltstellen am N-Terminus bzw. am C-Terminus des durch Protease spaltbaren Linkers befinden, wobei die beiden Protease-Spaltstellen gleich oder verschieden sein können.

5. Protein nach einem der Ansprüche 1 bis 4, wobei PP₁ mindestens eine konstante Immunglobulinregion und/oder mindestens eine variable Immunglobulinregion umfasst und/oder wobei PP₂ mindestens eine konstante Immunglobulinregion und/oder mindestens eine variable Immunglobulinregion umfasst, wobei vorzugsweise
PP₁ eine konstante Immunglobulinregion und PP₂ eine variable Immunglobulinregion umfasst,
PP₁ eine variable Immunglobulinregion und PP₂ eine konstante Immunglobulinregion umfasst,
PP₁ eine konstante Immunglobulinregion und PP₂ eine konstante Immunglobulinregion umfasst oder
PP₁ eine variable Immunglobulinregion und PP₂ eine variable Immunglobulinregion umfasst.

6. Protein nach einem der Ansprüche 1 bis 5, wobei es sich bei dem Protein um einen Antikörper oder ein Antikörperderivat handelt, wobei es sich vorzugsweise bei dem Protein um einen einkettigen Antikörper, vorzugsweise einen multispezifischen einkettigen Antikörper handelt.

7. Protein nach einem der Ansprüche 1 bis 6, umfassend eine Polypeptidkette mit einer Formel, die aus der Gruppe bestehend aus Folgendem ausgewählt ist:
(a)
VL-CL-PCL-VH-CH1-hinge-CH2-CH3,
wobei
VL für eine variable Region einer Immunglobulin-Leichtkette steht;
CL für eine konstante Region einer Immunglobulin-Leichtkette steht;
VH für eine variable Region einer Immunglobulin-Schwerkette steht;
CH1 für eine konstante Immunglobulin-Schwerkettenregion CH1 steht;
hinge für eine Immunglobulin-Hinge-Region steht;
CH2 für eine konstante Immunglobulin-Schwerkettenregion CH2 steht;
CH3 für eine konstante Immunglobulin-Schwerkettenregion CH3 steht; und
PCL für den durch Protease spaltbaren Linker steht;
(b)
VLₐ-CLₐ-PCL-VL_{b}-CL_{b},
wobei
VLₐ für eine variable Region einer ersten Immunglobulin-Leichtkette steht;
VL_{b} für eine variable Region einer zweiten Immunglobulin-Leichtkette steht;
CLₐ für eine konstante Region einer ersten Immunglobulin-Leichtkette steht;
CL_{b} für eine konstante Region einer zweiten Immunglobulin-Leichtkette steht; und
PCL für den durch Protease spaltbaren Linker steht; und
(c)
VLₐ-CLₐ-PCL-VL_{b}-CL_{b}-L-hinge-CH2-CH3
wobei
VLₐ für eine variable Region einer ersten Immunglobulin-Leichtkette steht;
VL_{b} für eine variable Region einer zweiten Immunglobulin-Leichtkette steht;
CLₐ für eine konstante Region einer ersten Immunglobulin-Leichtkette steht;
CL_{b} für eine konstante Region einer zweiten Immunglobulin-Leichtkette steht;
L für einen optionalen Linker steht;
hinge für eine Immunglobulin-Hinge-Region steht;
CH2 für eine konstante Immunglobulin-Schwerkettenregion CH2 steht;
CH3 für eine konstante Immunglobulin-Schwerkettenregion CH3 steht; und
PCL für den durch Protease spaltbaren Linker steht.

8. Nukleinsäure oder Satz von Nukleinsäuren, codierend das Protein nach einem der Ansprüche 1 bis 7.

9. Vektor oder Satz von Vektoren, umfassend die Nukleinsäure bzw. den Satz von Nukleinsäuren nach Anspruch 8.

10. Wirtszelle, umfassend ein Protein nach einem der Ansprüche 1 bis 7, eine Nukleinsäure oder einen Satz von Nukleinsäuren nach Anspruch 8 oder einen Vektor oder Satz von Vektoren nach Anspruch 9, wobei es sich bei der Wirtszelle vorzugsweise um eine Säugerzelle handelt.

11. Verfahren zur Herstellung eines Proteins, umfassend die Schritte:
i) Kultivieren einer Wirtszelle gemäß Anspruch 10; und
ii) Isolieren des Proteins aus der Wirtszelle.

12. Protein, erhältlich mit dem Verfahren nach Anspruch 11.

13. Protein nach einem der Ansprüche 1 bis 7 oder 12, wobei es sich bei dem Protein um ein therapeutisch wirkendes Protein handelt.

14. Protein nach Anspruch 13 zur therapeutischen Verwendung.

## Revendications

1. Protéine comprenant au moins une chaîne polypeptidique ayant la formule
PP₁-PCL-PP₂,
dans laquelle
PP₁ est un premier polypeptide,
PP₂ est un second polypeptide, et
PCL est un lieur clivable par une protéase comprenant au moins un site de clivage par une protéase comprenant la séquence d'acides aminés HRRX₁X₂RSVDE (SEQ ID NO: 42), dans laquelle X₁ et X₂ sont indépendamment choisis dans le groupe constitué par KK, KQ, KR, QK, QQ, QR, RK, RQ et RR.

2. Protéine selon la revendication 1, dans laquelle l'au moins un site de clivage par une protéase comprend la séquence d'acides aminés HRRRKRSVDE (SEQ ID NO: 43) ou la séquence d'acides aminés HRRQQRSVDE (SEQ ID NO: 44).

3. Protéine selon la revendication 1 ou 2, dans laquelle le clivage par une protéase du lieur entraîne un changement d'activité de la protéine, dans laquelle, de préférence, le changement d'activité est une liaison ou une liaison accrue à au moins un antigène.

4. Protéine selon l'une quelconque des revendications 1 à 3, dans laquelle le lieur clivable par une protéase comprend deux sites de clivage par une protéase, dans laquelle les deux sites de clivage par une protéase sont situés au niveau de l'extrémité N-terminale et au niveau de l'extrémité C-terminale du lieur clivable par une protéase, respectivement, dans laquelle les deux sites de clivage par une protéase peuvent être identiques ou différents.

5. Protéine selon l'une quelconque des revendications 1 à 4, dans laquelle PP₁ comprend au moins une région constante d'immunoglobuline et/ou au moins une région variable d'immunoglobuline, et/ou dans laquelle PP₂ comprend au moins une région constante d'immunoglobuline et/ou au moins une région variable d'immunoglobuline, dans laquelle, de préférence,
PP₁ comprend une région constante d'immunoglobuline et PP₂ comprend une région variable d'immunoglobuline,
PP₁ comprend une région variable d'immunoglobuline et PP₂ comprend une région constante d'immunoglobuline,
PP₁ comprend une région constante d'immunoglobuline et
PP₂ comprend une région constante d'immunoglobuline, ou
PP₁ comprend une région variable d'immunoglobuline et PP₂ comprend une région variable d'immunoglobuline.

6. Protéine selon l'une quelconque des revendications 1 à 5, dans laquelle la protéine est un anticorps ou un dérivé d'anticorps, dans laquelle, de préférence, la protéine est un anticorps monocaténaire, de préférence un anticorps monocaténaire multispécifique.

7. Protéine selon l'une quelconque des revendications 1 à 6, comprenant une chaîne polypeptidique ayant une formule choisie dans le groupe constitué par :
(a)
VL-CL-PCL-VH-CHI-charnière-CH2-CH3,
dans laquelle
VL est une région variable de chaîne légère d'immunoglobuline ;
CL est une région constante de chaîne légère d'immunoglobuline ;
VH est une région variable de chaîne lourde d'immunoglobuline ;
CH1 est une région constante de chaîne lourde CH1 d'immunoglobuline ;
la charnière est une région charnière d'immunoglobuline ; CH2 est une région constante de chaîne lourde CH2 d'immunoglobuline ;
CH3 est une région constante de chaîne lourde CH3 d'immunoglobuline ;
PCL est le lieur clivable par une protéase ;
(b)
VLₐ-CLₐ-PCL-VL_{b}-CL_{b},
dans laquelle
VLₐ est une première région variable de chaîne légère d'immunoglobuline ;
VL_{b} est une deuxième région variable de chaîne légère d'immunoglobuline ;
CLₐ est une première région constante de chaîne légère d'immunoglobuline ;
CL_{b} est une deuxième région constante de chaîne légère d'immunoglobuline ; et
PCL est le lieur clivable par une protéase ; et
(c)
VLₐ-CLₐ-PCL-VL_{b}-CL_{b}-L-charnière-CH2-CH3
dans laquelle
VLₐ est une première région variable de chaîne légère d'immunoglobuline ;
VL_{b} est une deuxième région variable de chaîne légère d'immunoglobuline ;
CLₐ est une première région constante de chaîne légère d'immunoglobuline ;
CL_{b} est une deuxième région constante de chaîne légère d'immunoglobuline ;
L est un lieur éventuel ;
la charnière est une région charnière d'immunoglobuline ; CH2 est une région constante de chaîne lourde CH2 d'immunoglobuline ;
CH3 est une région constante de chaîne lourde CH3 d'immunoglobuline ;
PCL est le lieur clivable par une protéase.

8. Acide nucléique ou ensemble d'acides nucléiques codant pour la protéine selon l'une quelconque des revendications 1 à 7.

9. Vecteur ou ensemble de vecteurs comprenant l'acide nucléique ou l'ensemble d'acides nucléiques selon la revendication 8.

10. Cellule hôte comprenant une protéine selon l'une quelconque des revendications 1 à 7, un acide nucléique ou un ensemble d'acides nucléiques selon la revendication 8, ou un vecteur ou un ensemble de vecteurs selon la revendication 9, dans laquelle, de préférence, la cellule hôte est une cellule de mammifère.

11. Procédé de production d'une protéine comprenant les étapes :
i) culture d'une cellule hôte selon la revendication 10 ; et
ii) isolement de la protéine à partir de la cellule hôte.

12. Protéine pouvant être obtenue par le procédé de la revendication 11.

13. Protéine selon l'une quelconque des revendications 1 à 7 ou 12, dans laquelle la protéine est une protéine thérapeutiquement active.

14. Protéine selon la revendication 13, destinée à être utilisée en thérapie.
